(19) Europäisches Patentamt
European Patent Office
Office européen des brevets

(11) EP 4 342 491 A1

(12) EUROPEAN PATENT APPLICATION
published in accordance with Art. 153(4) EPC

(43) Date of publication:
27.03.2024 Bulletin 2024/13

(21) Application number: 22804712.2

(22) Date of filing: 18.05.2022

(51) International Patent Classification (IPC):
A61K 39/21 (2006.01) A61K 9/127 (2006.01)
A61K 31/7115 (2006.01) A61K 38/16 (2006.01)
A61K 47/10 (2017.01) A61K 47/18 (2017.01)
A61K 47/24 (2006.01) A61K 47/28 (2006.01)
A61P 31/14 (2006.01) C12N 15/88 (2006.01)

(52) Cooperative Patent Classification (CPC):
A61K 9/127; A61K 31/7115; A61K 38/16;
A61K 39/21; A61K 47/10; A61K 47/18;
A61K 47/24; A61K 47/28; A61P 31/14; C12N 15/88

(86) International application number:
PCT/JP2022/020646

(87) International publication number:
WO 2022/244801 (24.11.2022 Gazette 2022/47)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 19.05.2021 JP 2021084942

(71) Applicants:
- National Institutes of Biomedical Innovation, Health and Nutrition
Ibaraki-shi, Osaka 567-0085 (JP)
- University of The Ryukyus
Nakagami-gun, Okinawa 903-0213 (JP)
- Daiichi Sankyo Company, Limited
Tokyo 103-8426 (JP)

(72) Inventors:
- ISHII Ken
Ibaraki-shi, Osaka 567-0085 (JP)
- FUKUSHIMA Takuya
Nakagami-gun, Okinawa 903-0213 (JP)
- TANAKA Yuetsu
Nakagami-gun, Okinawa 903-0213 (JP)
- TAKESHITA Fumihiko
Tokyo 103-8426 (JP)
- KOIZUMI Makoto
Tokyo 103-8426 (JP)
- NIWA Takako
Tokyo 103-8426 (JP)
- NOGUSA Shoko
Tokyo 103-8426 (JP)
- JONAI Nao
Tokyo 103-8426 (JP)
- ONODERA Yoshikuni
Tokyo 103-8426 (JP)

(74) Representative: Vossius & Partner
Patentanwälte Rechtsanwälte mbB
Siebertstrasse 3
81675 München (DE)

Remarks:
The complete document including Reference Table(s) and the Sequence Listing(s) can be downloaded from the EPO website

# (54) HTLV-1 NUCLEIC ACID LIPID PARTICLE VACCINE

(57) Provided is a vaccine for preventing and/or treating infection with human T-cell leukemia virus type 1 (HTLV-1).

A lipid particle encapsulating a nucleic acid expressing a gp46 antigen or a Tax antigen of human T-cell leukemia virus type 1 (HTLV-1), wherein the lipid comprises a cationic lipid represented by general formula (Ia):

EP 4 342 491 A1

[Formula 1]

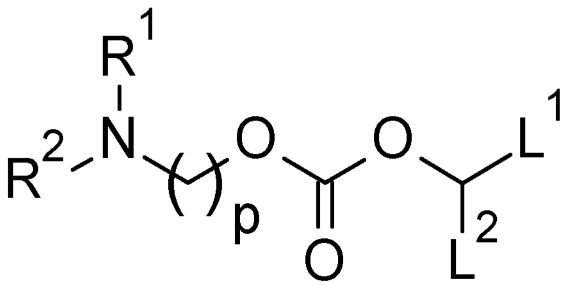

(Ia)

or a pharmaceutically acceptable salt thereof,
wherein
$R^1$ and $R^2$ each independently represent a $C_1$-$C_3$ alkyl group;
$L^1$ represents a $C_{17}$-$C_{19}$ alkenyl group optionally having one or more $C_2$-$C_4$ alkanoyloxy groups;
$L^2$ represents a $C_{10}$-$C_{19}$ alkyl group optionally having one or more $C_2$-$C_4$ alkanoyloxy groups, or a $C_{10}$-$C_{19}$ alkenyl group optionally having one or more $C_2$-$C_4$ alkanoyloxy groups; and
p is 3 or 4.

Fig. 5

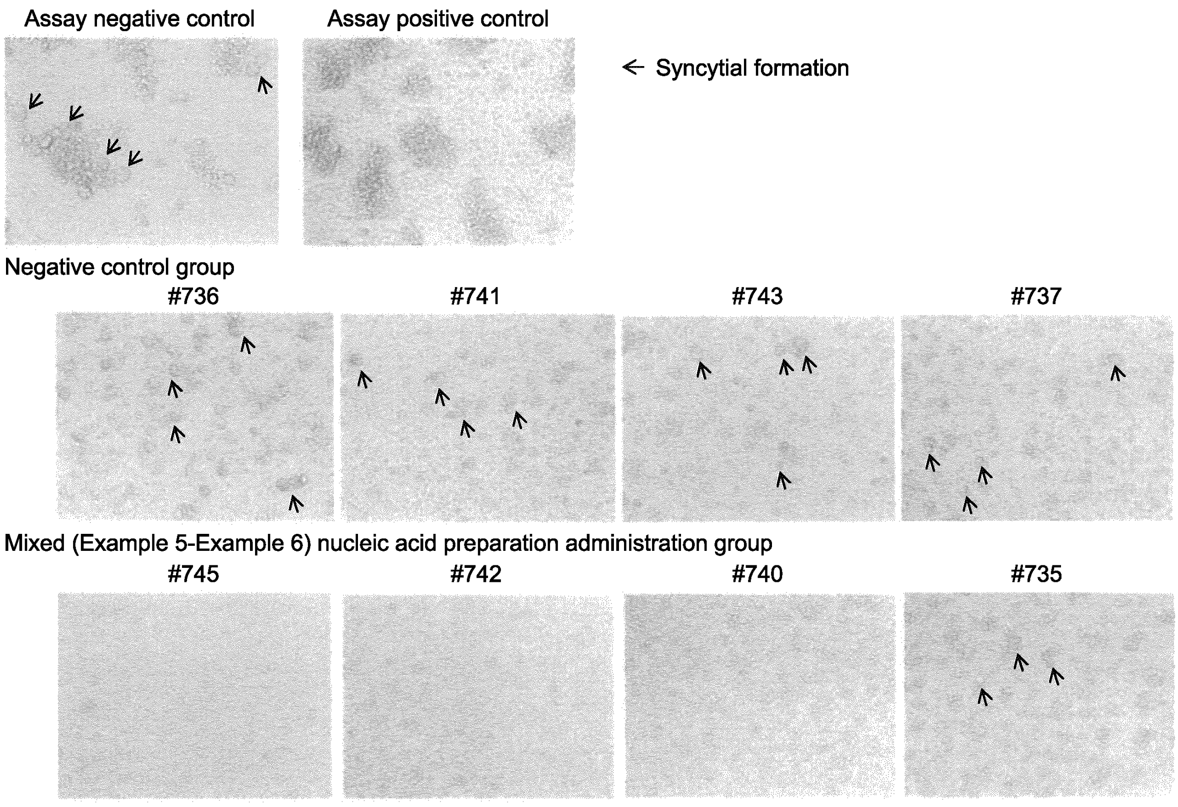

## Description

### Technical Field

The present invention relates to a nucleic-acid lipid particle vaccine encapsulating HTLV-1 mRNA.

### Background Art

Human T-cell leukemia virus type 1 (HTLV-1) is a retrovirus having high carcinogenicity. There are 10 million to 20 million infected persons with HTLV-1 in the world and one million infected persons in Japan (Non Patent Literature 1). Of the HTLV-1 infected persons, 3 to 5% persons develop adult T cell leukemia (ATL) with a poor prognosis. At present, there are neither prophylactic vaccines for HTLV-1 infection nor drugs for preventing the onset of ATL. In addition, there is no established standard treatment contributing to a satisfactory/effective therapy for ATL. For example, if a multi-drug chemotherapy as a standard therapy, is applied, the percentage of the long-term survivors is less than 10%.

Recently, allogeneic hematopoietic stem-cell transplantation has come to be applied to ATL patients and some of the patients highly benefit from the transplantation therapy. To the antitumor effect of the allogeneic hematopoietic stem-cell transplantation, not only the effect of pre-transplantation treatments, such as high-dose anticancer drug administration and radiation of the whole body, but also an antitumor effect (graft versus leukemia effect; GVL effect) produced by donor's immunocompetent cells after the transplantation may conceivably contribute (Non Patent Literature 2). Particularly, an increase of cytotoxic T lymphocytes (CTLs) specific to a transcription activator Tax essential for HTLV-1 replication has been reported (Non Patent Literature 3). However, in the allogeneic hematopoietic stem cell transplantation, the mortality of patients due to a side effect, graft versus host disease (GVHD), is high.

Tax is a protein having an ability to activate NF-kB and being essential for the replication of HTLV-1 (Non Patent Literature 4). It has been confirmed that excessive NF-kB activation through Tax occurs in HTLV-1 infected cells and ATL cells. The NF-kB activation by Tax is clearly correlated with the canceration of infected cells (Non Patent Literature 5, Non Patent Literature 6). Non Patent Literature 7 discloses that amino acids important for NF-kB activation by Tax were identified, and that the mechanism how Tax activates NF-kB has been analyzed by use of a Tax mutant prepared by inserting a point mutation to the amino acids (Non Patent Literature 8 and Non Patent Literature 9). However, effective vaccines using Tax or a Tax mutant for preventing or treating HTLV-1 have not yet been obtained.

From the results of experiments on HTLV-1 infection using immunodeficient mice having human peripheral blood mononuclear cells (hPBMCs) transplanted therein, it was clarified that HTLV-1 infection is neutralized by an anti-gp46 antibody targeting an envelope protein gp46 of HTLV-1 (Non Patent Literature 10). Also, in transovarial transmission rat models, it is suggested that an anti-gp46 antibody having a neutralization activity can prevent transovarial transmission. However, an effective vaccine using gp46 for preventing or treating HTLV-1 has not been reported.

As an LNP preparation (LNP-mRNA) encapsulating mRNA encoding an antigen in lipid nanoparticles (LNP), a SARS-CoV-2 vaccine (Non Patent Literature 11) or an influenza vaccine (patent literature 1) is known.

### Citation List

#### Patent Literature

Patent literature 1: International Publication No. WO2015/164674

#### Non Patent Literatures

Non Patent Literature 1: Front Microbiol. (2012) 3: 388.
Non Patent Literature 2: Blood (2012) 119 (10): 2409-2416.
Non Patent Literature 3: Cancer Research (2010) 70 (15): 6181-6192.
Non Patent Literature 4: Lancet Infect Dis (2007) 7: 266-281.
Non Patent Literature 5: Front Microbiol. (2012) 3: 406.
Non Patent Literature 6: Viruses (2011) 3 (6): 714-749.
Non Patent Literature 7: Gene Dev. (1990) 4: 1875.
Non Patent Literature 8: J. Biochem. (2011) 150 (6): 679-686.
Non Patent Literature 9: J. Biol Chem. (2006) 281 (19): 13075-13082.
Non Patent Literature 10: Viruses (2016) 8 (2): 41.
Non Patent Literature 11: Science (2021) 371: 1152.

Summary of Invention

Technical Problem

An object of the present invention is to provide a vaccine for preventing and/or treating infection with human T cell leukemia virus type 1 (HTLV-1).

Solution to Problem

The present inventors have found that a nucleic-acid lipid particle encapsulating HTLV-1 mRNA (LNP-mRNA) can be applied to the treatment and prevention of the onset of adult T cell leukemia, and have arrived at the achievement of the present invention. More specifically, they have prepared LNP-mRNA-HTLV-1 encapsulating mRNA expressing HTLV-1 Tax and gp46; and administered LNP-mRNA-HTLV-1 to mice and monkeys. As a result, they have found that the production of antibodies to HTLV-1 Tax and gp46 and CTLs and the neutralization activity to inhibit the proliferation of HTLV-1 infected cells are induced in the mice and monkeys.

The gist of the present invention is as follows.

(1) A lipid particle encapsulating a nucleic acid capable of expressing a gp46 antigen or a Tax antigen of human T-cell leukemia virus type 1 (HTLV-1), wherein the lipid comprises a cationic lipid represented by general formula (Ia):

[Formula 1]

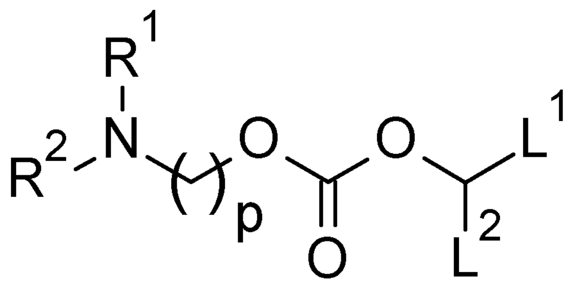

(Ia)

or a pharmaceutically acceptable salt thereof,
wherein

$R^1$ and $R^2$ each independently represent a $C_1$-$C_3$ alkyl group;
$L^1$ represents a $C_{17}$-$C_{19}$ alkenyl group optionally having one or more $C_2$-$C_4$ alkanoyloxy group
$L^2$ represents a $C_{10}$-$C_{19}$ alkyl group optionally having one or more $C_2$-$C_4$ alkanoyloxy groups, or a $C_{10}$-$C_{19}$ alkenyl group optionally having one or more $C_2$-$C_4$ alkanoyloxy groups; and p is 3 or 4.

(2) The particle according to (1), wherein, in general formula (Ia), $R^1$ and $R^2$ both are methyl groups.
(3) The particle according to (1) or (2), wherein, in general formula (Ia), "p" is 3.
(4) The particle according to any one of (1) to (3), wherein, in general formula (Ia), $L^1$ is a $C_{17}$-$C_{19}$ alkenyl group optionally having one or more acetoxy group
(5) The particle according to any one of (1) to (4), wherein in general formula (Ia), $L^2$ is a $C_{10}$-$C_{12}$ alkyl group optionally having one or more acetoxy groups, or a $C_{10}$-$C_{19}$ alkenyl group optionally having one or more acetoxy groups.
(6) The particle according to any one of (1) to (4), wherein in general formula (Ia), $L^2$ is a $C_{10}$-$C_{12}$ alkyl group optionally having one or more acetoxy groups or a $C_{17}$-$C_{19}$ alkenyl group optionally having one or more acetoxy groups.
(7) The particle according to any one of (1) to (6), wherein in general formula (Ia), $L^1$ is an (R)-11-acetyloxy-cis-8-heptadecenyl group, a cis-8-heptadecenyl group, or an (8Z,11Z)-heptadecadienyl group.
(8) The particle according to any one of (1) to (7), wherein in general formula (Ia), $L^2$ is a decyl group, a cis-7-decenyl group, a dodecyl group, or an (R)-11-acetyloxy-cis-8-heptadecenyl group.
(9) The particle according to (1), wherein the cationic lipid is represented by the following structural formula:

[Formula 2]

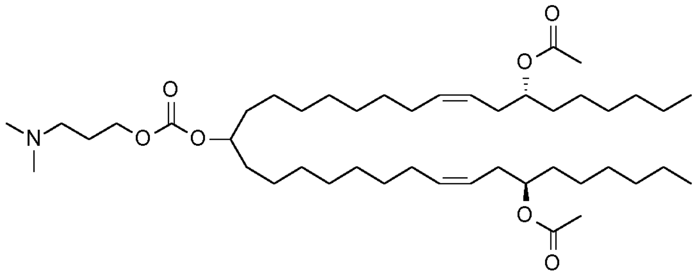

(10) The particle according to (1), wherein the cationic lipid is represented by the following structural formula:

[Formula 3]

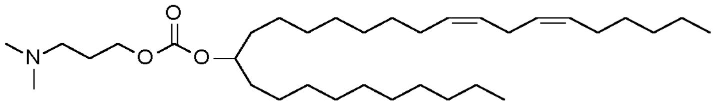

(11) The particle according to (1), wherein the cationic lipid is represented by the following structural formula:

[Formula 4]

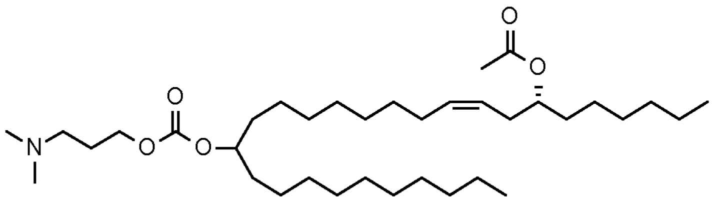

(12) The particle according to (9) or (10), wherein the lipid further comprises an amphipathic lipid, a sterol and a PEG lipid.
(13) The particle according to (11), wherein the lipid further comprises an amphipathic lipid, a sterol and a PEG lipid.
(14) The particle according to (12), wherein the amphipathic lipid is at least one selected from the group consisting of distearoylphosphatidylcholine, dioleoylphosphatidylcholine and dioleoyl phosphatidylethanolamine.
(15) The particle according to (13), wherein the amphipathic lipid is at least one selected from the group consisting of distearoylphosphatidylcholine, dioleoylphosphatidylcholine and dioleoyl phosphatidylethanolamine.
(16) The particle according to (12) or (14), wherein the sterol is cholesterol.
(17) The particle according to (13) or (15), wherein the sterol is cholesterol.
(18) The particle according to any one of (12), (14) and (16), wherein the PEG lipid is 1,2-dimyristoyl-sn-glycerol methoxypolyethylene glycol and/or N-[methoxypoly(ethylene glycol)2000]carbamoyl]-1,2-dimyristyloxypropyl-3-amine.
(19) The particle according to any one of (13), (15) and (17), wherein the PEG lipid is 1,2-dimyristoyl-sn-glycerol methoxypolyethylene glycol and/or N-[methoxypoly(ethylene glycol)2000]carbamoyl]-1,2-dimyristyloxypropyl-3-amine.
(20) The particle according to any one of (12) to (19), wherein the lipid composition of the amphipathic lipid, the sterol, the cationic lipid, and the PEG lipid is as follows: 5 to 25% of the amphipathic lipid, 10 to 55% of the sterol, 40 to 65% of the cationic lipid, and 1 to 5% of the PEG lipid on a molar basis.
(21) The particle according to (20), wherein the amphipathic lipid is contained in a ratio of 10 to 25%.
(22) The particle according to any one of (12), (14), (16) and (18), wherein the lipid composition of the amphipathic lipid, the sterol, the cationic lipid, and the PEG lipid is as follows: 5 to 15% of the amphipathic lipid, 35 to 50% of the sterol, 40 to 55% of the cationic lipid, and 1 to 3% of the PEG lipid on a molar basis.
(23) The particle according to (22), wherein the amphipathic lipid, sterol, cationic lipid and PEG lipid are contained in a ratio of 10 to 15%, 35 to 45%, 40 to 50%, and 1 to 2.5%, respectively.

(24) The particle according to (23), wherein the PEG lipid is contained in a ratio of 1 to 2%.
(25) The particle according to any one of (13), (15), (17) and (19), wherein the lipid composition of the amphipathic lipid, the sterol, the cationic lipid, and the PEG lipid is as follows: 10 to 25% of the amphipathic lipid, 10 to 50% of the sterol, 40 to 65% of the cationic lipid, and 1 to 3% of the PEG lipid on a molar basis.
(26) The particle according to (25), wherein the sterol, cationic lipid and PEG lipid are contained in a ratio of 10 to 45%, 42.5 to 65%, and 1 to 2.5%, respectively.
(27) The particle according to (26), wherein the PEG lipid is contained in a ratio of 1 to 2%.
(28) The particle according to any one of (20) to (27), wherein a weight ratio of the total lipid to the nucleic acid is from 15 to 30.
(29) The particle according to (28), wherein the weight ratio of the total lipid to the nucleic acid is from 15 to 25.
(30) The particle according to (29), wherein the weight ratio of the total lipid to the nucleic acid is from 17.5 to 22.5.
(31) The particle according to any one of (1) to (30), wherein the gp46 antigen of human T-cell leukemia virus type 1 (HTLV-1) is a fusion protein with an oligomerization domain.
(32) The particle according to (31), wherein the oligomerization domain is fibritin.
(33) The particle according to (31) or (32), wherein the gp46 antigen of human T-cell leukemia virus type 1 (HTLV-1) consists of an amino acid sequence having an identity of at least 95% with the amino acid sequence of SEQ ID NO: 14.
(34) The particle according to any one of (1) to (33), wherein the Tax antigen of human T-cell leukemia virus type 1 (HTLV-1) has at least one mutation selected from the group consisting of T130A, L131S, L319R and L320S relative to the amino acid sequence of SEQ ID NO: 11, and the amino acid sequence consisting of amino acids except the mutant amino acids has an identity of at least 95% with the amino acid sequence of SEQ ID NO: 11.
(35) The particle according to (34), wherein the Tax antigen of human T-cell leukemia virus type 1 (HTLV-1) has mutations of T130A, L131S, L319R and L320S relative to the amino acid sequence of SEQ ID NO: 11, and the amino acid sequence consisting of amino acids except the mutant amino acids has an identity of at least 95% with the amino acid sequence of SEQ ID NO: 11.
(36) The particle according to any one of (1) to (35), wherein the Tax antigen of human T-cell leukemia virus type 1 (HTLV-1) is a fusion protein with a signal peptide.
(37) The particle according to (36), wherein the signal peptide is a peptide consisting of the sequence from positions 1 to 18 in the amino acid sequence of SEQ ID NO: 13.
(38) The particle according to any one of (31) to (37), wherein nucleic acid capable of expressing the gp46 antigen or tax antigen of human T-cell leukemia virus type 1 (HTLV-1) is mRNA comprising a cap structure (Cap), a 5'-noncoding region (5'-UTR), a coding region of the gp46 antigen or Tax antigen, a 3'-noncoding region (3'-UTR) and poly A tail (polyA).
(39) The particle according to (38), wherein the sequence of the nucleic acid capable of expressing the gp46 antigen of human T-cell leukemia virus type 1 (HTLV-1) consists of a nucleotide sequence having an identity of at least 90% with the sequence of SEQ ID NO: 17 or 18.
(40) The particle according to (38), wherein the sequence of the nucleic acid capable of expressing the Tax antigen of human T-cell leukemia virus type 1 (HTLV-1) consists of a nucleotide sequence having an identity of at least 90% with the sequence of SEQ ID NO: 20.
(41) The particle according to any one of (1) to (40), wherein the nucleic acid comprises at least one modified nucleotide.
(42) The particle according to (41), wherein the modified nucleotide comprises at least one of a pyrimidine nucleotide substituted at position 5 and/or pseudouridine optionally substituted at position 1.
(43) The particle according to (41), wherein the modified nucleotide comprises at least one selected from the group consisting of 5-methylcytidine, 5-methoxyuridine, 5-methyluridine, pseudouridine, and a 1-alkylpseudouridine.
(44) The particle according to (41), wherein the modified nucleotide comprises at least one selected from the group consisting of 5-methylcytidine, 5-methyluridine, and 1-methylpseudouridine.
(45) The particle according to any one of (1) to (44), wherein the particle has an average particle size of 30 to 300 nm.
(46) Use of the particle according to any one of (1) to (45), for preparing a composition for preventing and/or treating infection with human T-cell leukemia virus type 1 (HTLV-1).
(47) A composition comprising the particle according to any one of (1) to (45).
(48) The composition according to (47) for expressing the gp46 antigen or Tax antigen of human T-cell leukemia virus type 1 (HTLV-1) *in vivo* or *in vitro.*
(49) The composition according to (47) or (48) for use as a medicament.
(50) The composition according to (49), for inducing an immune response to human T-cell leukemia virus type 1 (HTLV-1).
(51) The composition according to (49) or (50), for preventing and/or treating infection with human T-cell leukemia virus type 1 (HTLV-1).

(52) The composition according to (49) or (50), for preventing the onset of and/or treating a disease caused by HTLV-1 selected from the group consisting of adult T cell leukemia/lymphoma (ATLL), HTLV-1 associated myelopathy (HAM) and HTLV-1 uveitis (HU), in an HTLV-1 infected person.

(53) A method for expressing the gp46 antigen or Tax antigen of human T-cell leukemia virus type 1 (HTLV-1) *in vitro,* comprising introducing the composition according to any one of (47) to (50) into a cell.

(54) A method for expressing the gp46 antigen or Tax antigen of human T-cell leukemia virus type 1 (HTLV-1) *in vivo,* comprising administering the composition according to any one of (47) to (51) to a mammal.

(55) A method for inducing an immune response to human T-cell leukemia virus type 1 (HTLV-1), comprising administering the composition according to (49) or (50) to a mammal.

(56) A method for preventing and/or treating infection with human T-cell leukemia virus type 1 (HTLV-1), comprising administering the composition according to any one of (49) to (52) to a mammal.

(57) A method for preventing the onset of and/or treating a disease caused by HTLV-1 selected from the group consisting of adult T cell leukemia/lymphoma (ATLL), HTLV-1 associated myelopathy (HAM) and HTLV-1 uveitis (HU), comprising administering the composition according to any one of (49) to (52) to a mammal.

(58) A peptide prepared by fusing the Tax antigen of human T-cell leukemia virus type 1 (HTLV-1) having a mutation, by which carcinogenicity deteriorates or disappears, with a signal peptide.

(59) The peptide according to (58), wherein the Tax antigen has at least one mutation selected from the group consisting of T130A, L131S, L319R and L320S relative to the amino acid sequence of SEQ ID NO: 11, and the amino acid sequence consisting of amino acids except the mutant amino acids has an identity of at least 95% with the amino acid sequence of SEQ ID NO: 11.

(60) The peptide according to (58) or (59), wherein the signal peptide is a peptide consisting of an amino acid sequence from positions 1 to 18 in the amino acid sequence of SEQ ID NO: 13.

(61) The particle according to any one of (31) to (37), wherein a nucleic acid capable of expressing the gp46 antigen or Tax antigen of human T-cell leukemia virus type 1 (HTLV-1) is mRNA comprising a structure consisting of a cap structure (Cap), a 5'-noncoding region (5'-UTR), a coding region of the gp46 antigen or Tax antigen and a 3'-noncoding region (3'-UTR).

(62) The particle according to (61), wherein the structure consisting of a cap structure (Cap), a 5'-noncoding region (5'-UTR), a coding region of the gp46 antigen or Tax antigen, and a 3'-noncoding region (3'-UTR), is a nucleotide sequence having an identity of at least 90% with a sequence from positions 1 to 1141 in SEQ ID NO: 17 or a sequence from positions 1 to 1222 in SEQ ID NO: 18.

(63) The particle according to (61), wherein the structure consisting of a cap structure (Cap), a 5'-noncoding region (5'-UTR), a coding region of the gp46 antigen or Tax antigen, and a 3'-noncoding region (3'-UTR) is a nucleotide sequence having an identity of at least 90% with a sequence from positions 1 to 1315 in SEQ ID NO: 20.

The specification incorporates the contents disclosed by JP Patent Application No. 2021-084942, based on which the priority of the present application claims.

Advantageous Effects of Invention

Infection with human T-cell leukemia virus type 1 (HTLV-1) can be prevented and/or treated by the present invention. Also, a disease caused by infection with HTLV-1 can be prevented and/or treated by the present invention. The particle of the present invention has excellent properties in view of, e.g., metabolic stability, *in-vitro* activity, *in-vivo* activity, rapid exertion of a medicinal effect, persistency of a medicinal effect, physical stability, drug interaction and safety, and is useful as a medical drug for treating or preventing the diseases mentioned above.

Brief Description of Drawings

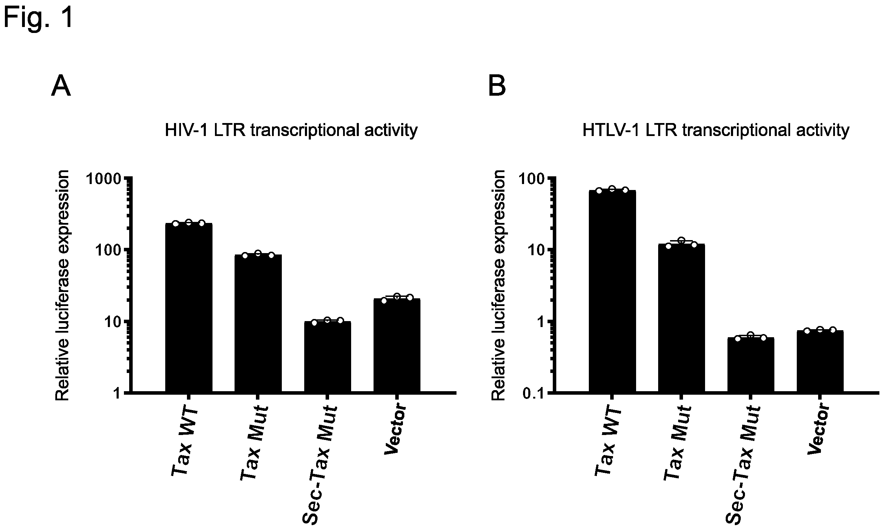

[Figure 1] Figure 1 shows HIV-1 LTR transcriptional activity (A) and HTLV-1 LTR transcriptional activity (B) of a Tax wild type, a Tax mutant, and a secretory Tax mutant. "pHIV-1 LTR" represents an HIV-1 LTR reporter plasmid administration group and, "pHTLV-1 LTR" represents an HTLV-1 LTR reporter plasmid administration group. "Empty" represents a negative control group, pcDNA3.1 + vector. The measurement is performed in triplicate. The vertical bars show average values of the measurement data and the error bars show SDs. The open circles on the bar graph show individual data obtained by triplicate measurement. "Tax WT": Tax wild type, "Tax Mut": a Tax mutant and "Sec-Tax Mut": a secretory Tax mutant.

[Figure 2] Figure 2 shows the Tax-specific CTL induction level and anti-Tax antibody titer in the blood in C3H mice administered with the lipid particle of Example 12. Figure 2A shows the Tax-specific CTL induction level and Figure

2B shows the anti-Tax antibody titer in the blood.

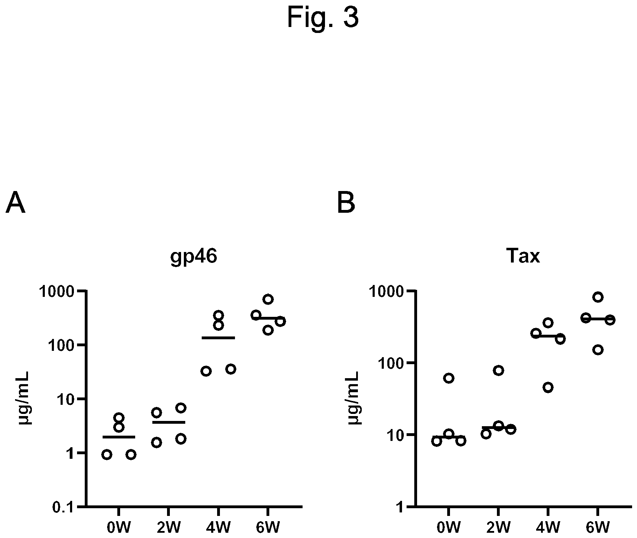

[Figure 3] Figure 3 shows the anti-gp46 antibody titer in the blood and the anti-Tax antibody titer in the blood in monkeys. Figure 3A shows the anti-gp46-specific antibody titer in the blood and Figure 3B shows the anti-Tax antibody titer in the blood. A group consists of 4 monkeys. Data are shown at time points: 0W, 2W, 4W and 6W, which represent before administration, 2 weeks after the first administration, 4 weeks after the first administration (2 weeks after the second administration), 6 weeks after the first administration (2 weeks after the third administration), respectively. The bars show average values and open circles show individual data.

[Figure 4] Figure 4 shows gp46-specific and Tax-specific cell-mediated immune responses. A group consists of 4 monkeys. The vertical bars show average values of the measurement data and the error bars show SDs. The open circles on the bar graph show individual data.

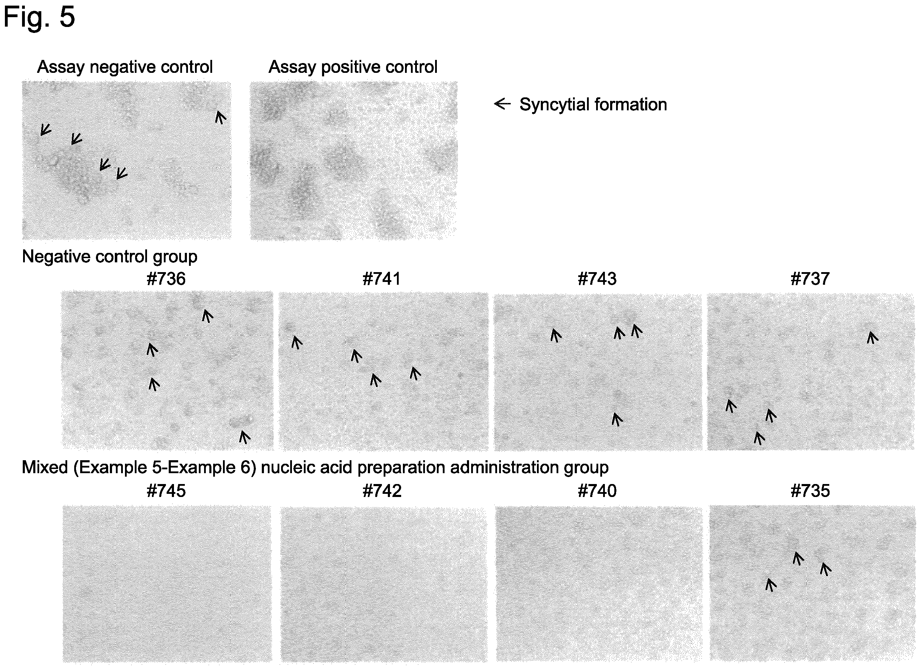

[Figure 5] Figure 5 shows anti-HTLV-1 neutralization activity in monkey blood. "Assay negative control" shows the result of a sample not treated with an antibody and "Assay positive control" shows the result of a sample treated with an HTLV-1 neutralizing antibody commonly known in the technical field. A group consists of 4 monkeys. The numbers, #736, #741, #743 and #737 show individual monkey IDs in a negative control group. The numbers, #745, #742, #740 and #735 show individual monkey IDs in a group of monkeys administered with a preparation containing mRNA molecules of Example 5 and Example 6. The arrows indicate syncytia formation.

[Figure 6] Figure 6 shows the anti-gp46 antibody titer in the blood of C57BL/6 mice.

[Figure 7] Figure 7 shows gp46-specific and Tax-specific cell-mediated immune responses of C57BL/6 mice. Figure 7A shows IFN-$\gamma$ production levels and Figure 7B shows IL-2 production levels.

[Figure 8] Figure 8 shows gp46 and Tax protein expression levels in CHO-S cells treated with the lipid particles of Example 9 to Example 12. Figure 8A shows the expression levels in cell lysates and Figure 8B shows expression levels in culture supernatants.

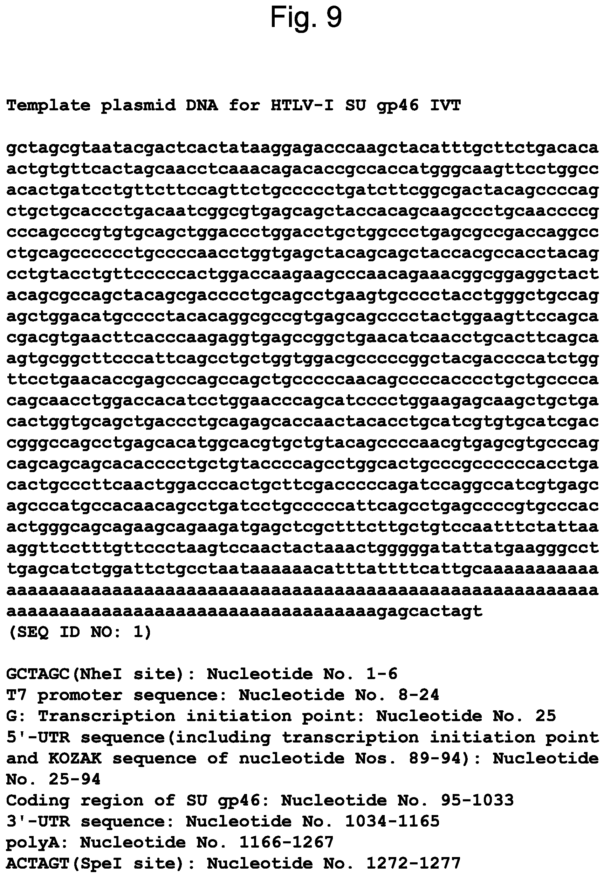

[Figure 9] Figure 9 shows the nucleotide sequence (SEQ ID NO: 1) of template plasmid DNA for IVT of HTLV-I SU gp46.

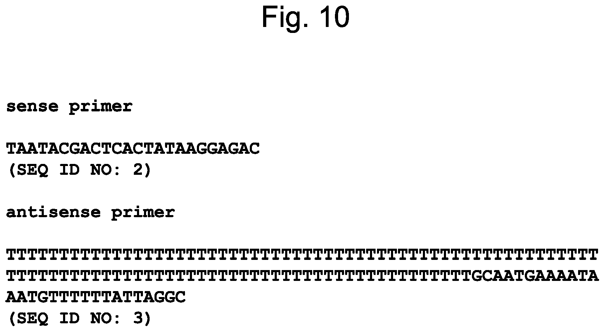

[Figure 10] Figure 10 shows the nucleotide sequence of a sense primer (SEQ ID NO: 2) and the nucleotide sequence of an antisense primer (SEQ ID NO: 3).

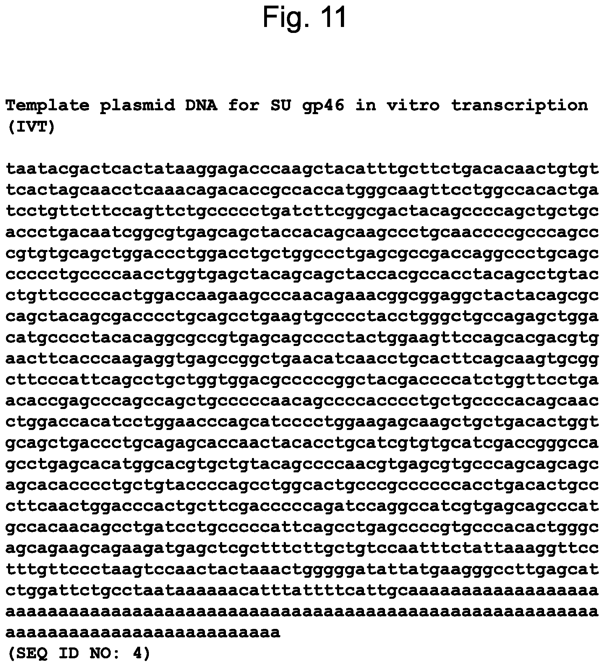

[Figure 11] Figure 11 shows the nucleotide sequence (SEQ ID NO: 4) of template DNA for *in-vitro* transcription (IVT) of SU gp46.

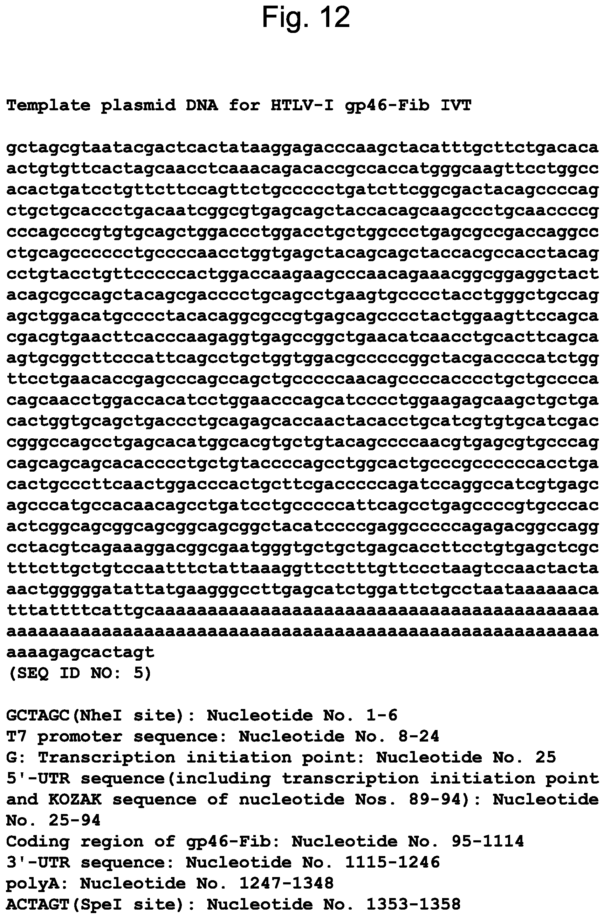

[Figure 12] Figure 12 shows the nucleotide sequence (SEQ ID NO: 5) of template plasmid DNA for IVT of HTLV-I gp46-Fib.

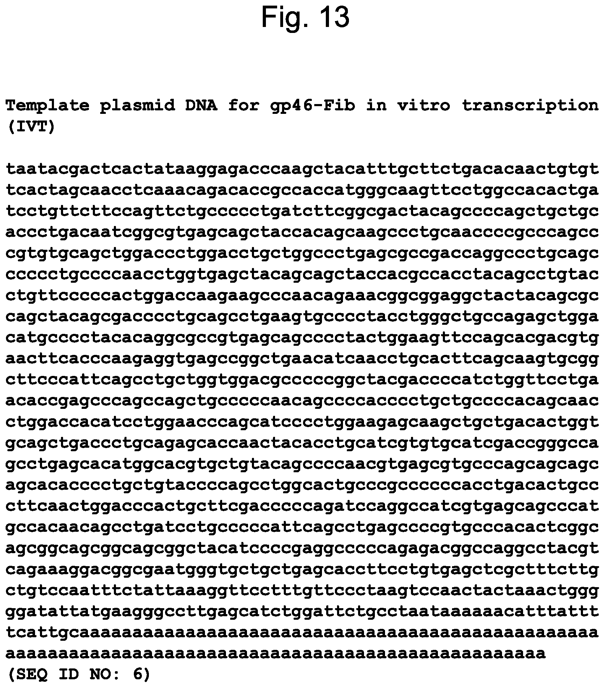

[Figure 13] Figure 13 shows the nucleotide sequence (SEQ ID NO: 6) of template DNA for *in-vitro* transcription (IVT) of gp46-Fib.

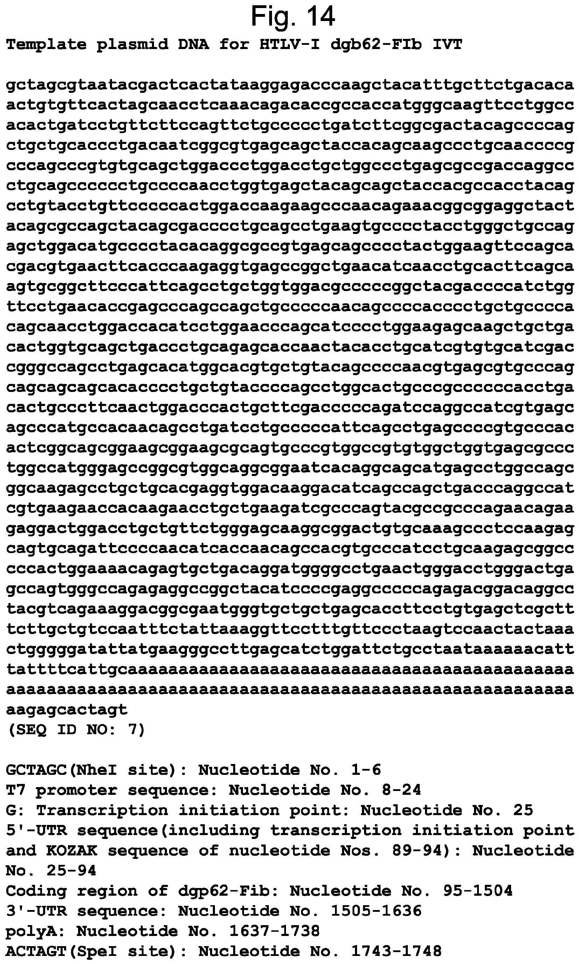

[Figure 14] Figure 14 shows the nucleotide sequence (SEQ ID NO: 7) of template plasmid DNA for IVT of HTLV-I dgp62-Fib.

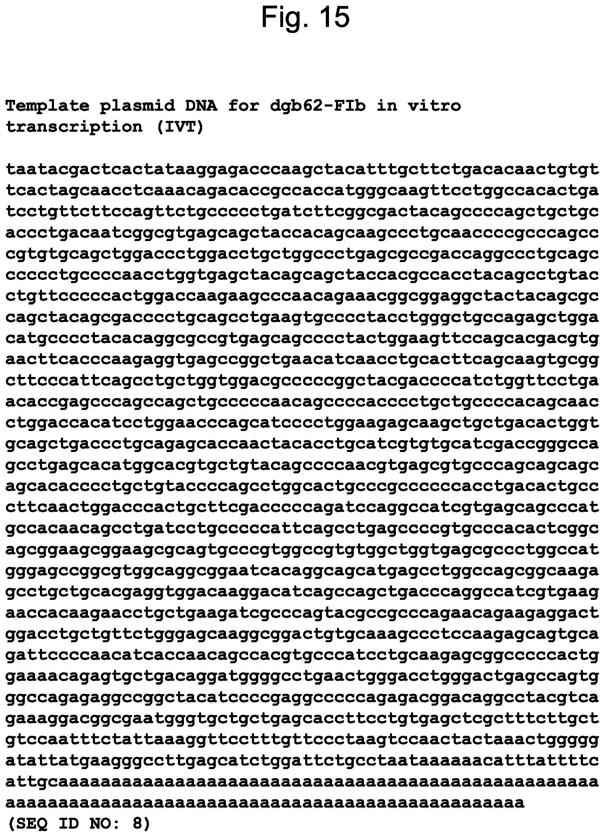

[Figure 15] Figure 15 shows the nucleotide sequence (SEQ ID NO: 8) of template DNA for *in-vitro* transcription (IVT) of dgp62-Fib.

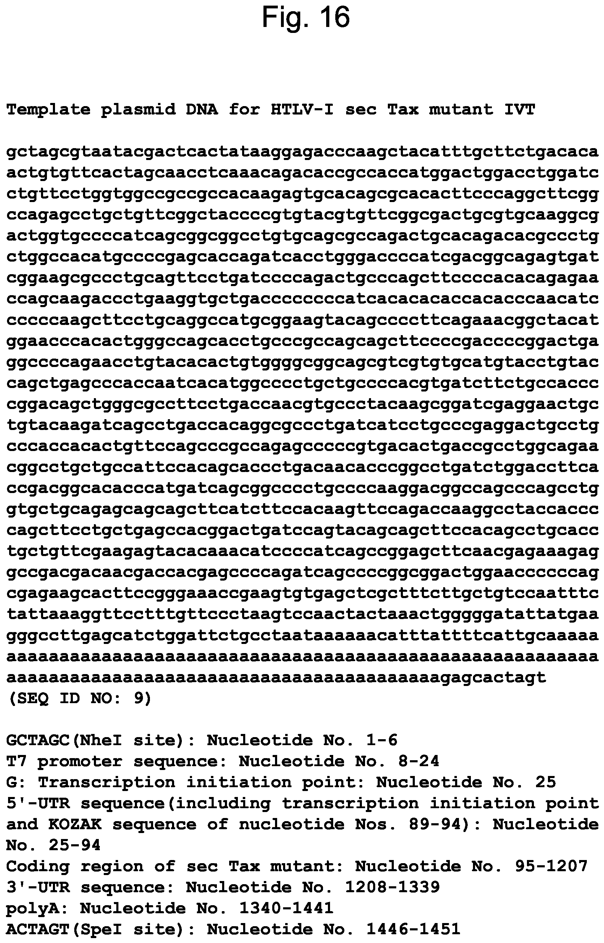

[Figure 16] Figure 16 shows the nucleotide sequence (SEQ ID NO: 9) of template plasmid DNA for IVT of an HTLV-I sec Tax mutant.

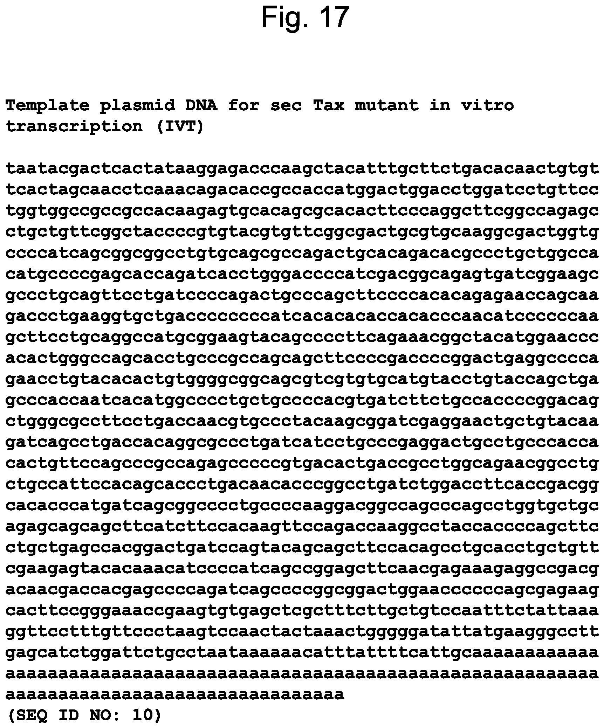

[Figure 17] Figure 17 shows the nucleotide sequence (SEQ ID NO: 10) of template DNA for *in-vitro* transcription (IVT) of a sec Tax mutant.

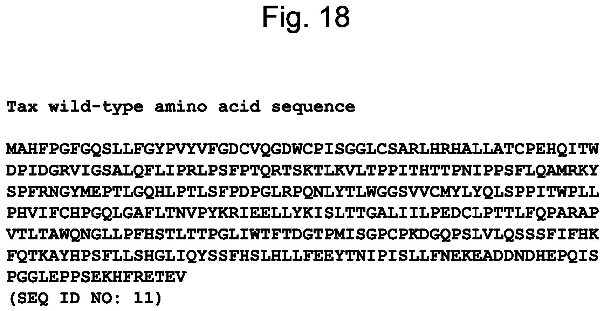

[Figure 18] Figure 18 shows the amino acid sequence (SEQ ID NO: 11) of a Tax wild type.

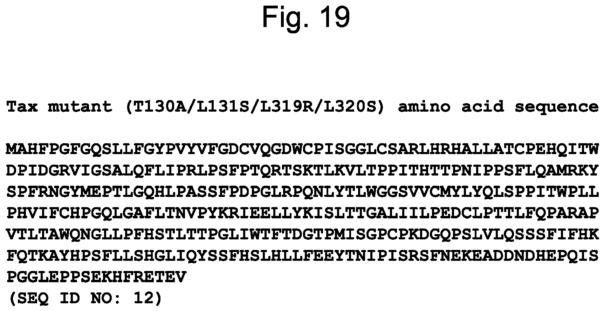

[Figure 19] Figure 19 shows the amino acid sequence (SEQ ID NO: 12) of a Tax mutant (T130A/L131S/L319R/L320S).

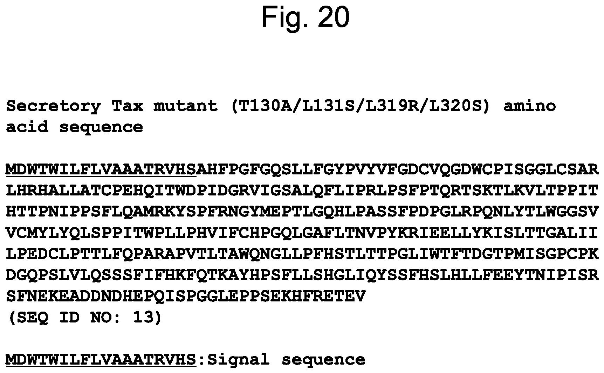

[Figure 20] Figure 20 shows the amino acid sequence (SEQ ID NO: 13) of a secretory Tax mutant (T130A/L131S/L319R/L320S).

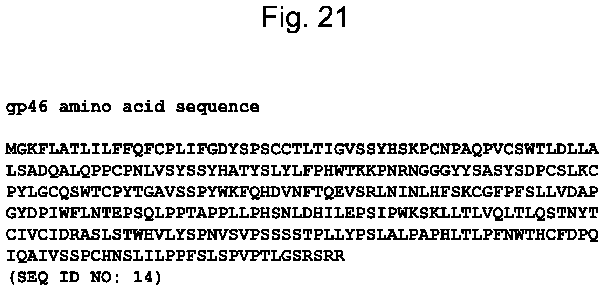

[Figure 21] Figure 21 shows the amino acid sequence (SEQ ID NO: 14) of gp46.

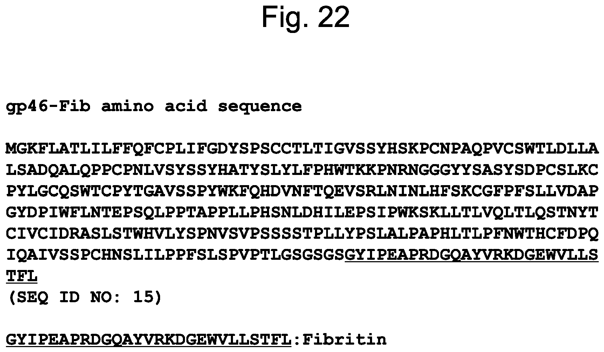

[Figure 22] Figure 22 shows the amino acid sequence (SEQ ID NO: 15) of gp46-Fib.

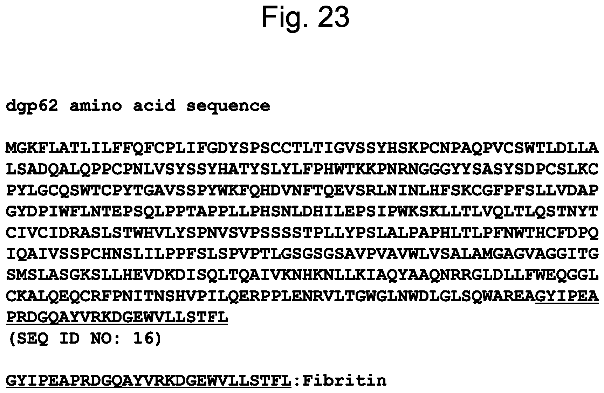

[Figure 23] Figure 23 shows the amino acid sequence (SEQ ID NO: 16) of dgp62-Fib.

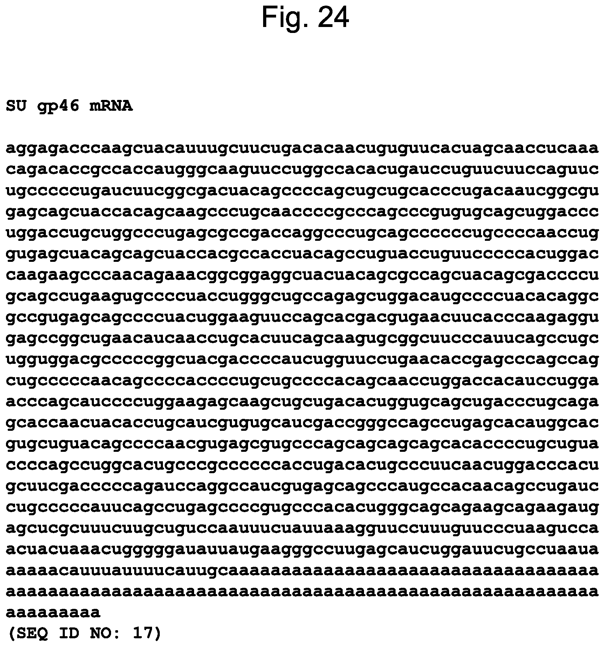

[Figure 24] Figure 24 shows the nucleotide sequence (SEQ ID NO: 17) of SU gp46 mRNA.

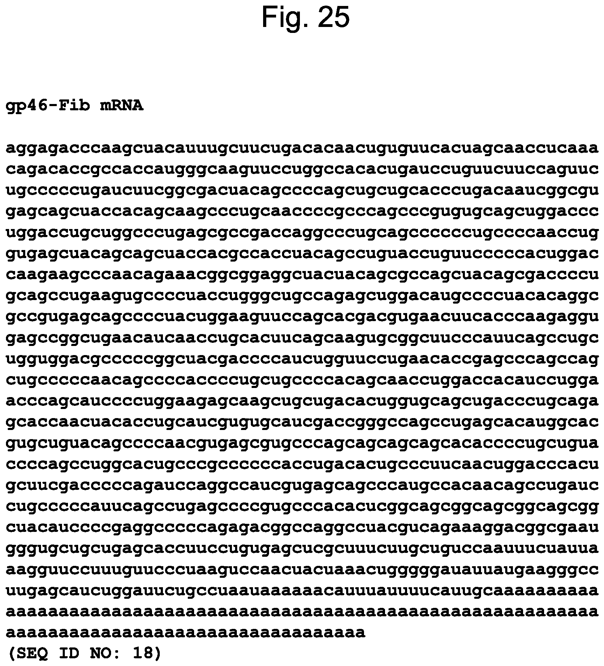

[Figure 25] Figure 25 shows the nucleotide sequence (SEQ ID NO: 18) of gp46-Fib mRNA.

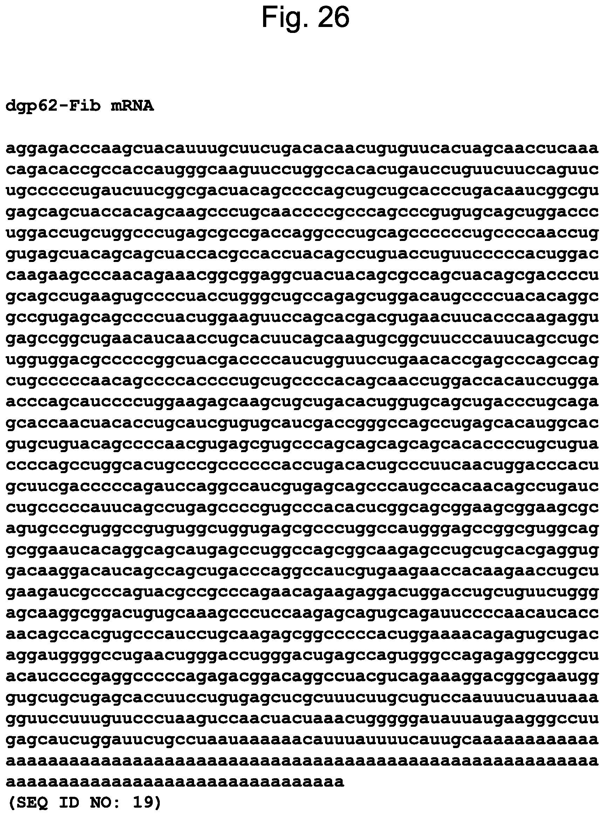

[Figure 26] Figure 26 shows the nucleotide sequence (SEQ ID NO: 19) of dgp62-Fib mRNA.

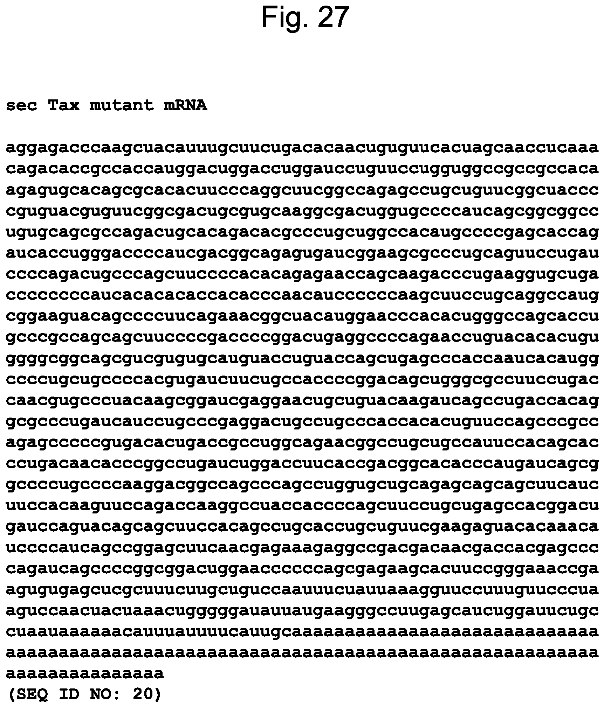

[Figure 27] Figure 27 shows the nucleotide sequence (SEQ ID NO: 20) of sec Tax mutant mRNA.

[Figure 28] Figure 28 shows the gp46 protein expression levels in CHO-S cells treated with Examples 30 to 34. The dotted line shows the OD value of Buffer as a negative control.

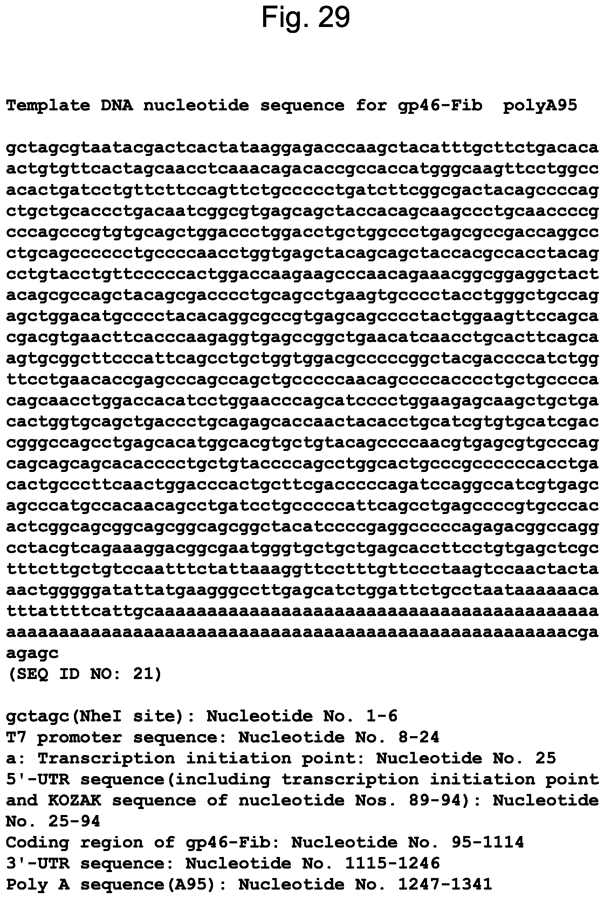

[Figure 29] Figure 29 shows the nucleotide sequence of the template DNA for gp46-Fib + polyA95 (SEQ ID NO: 21).

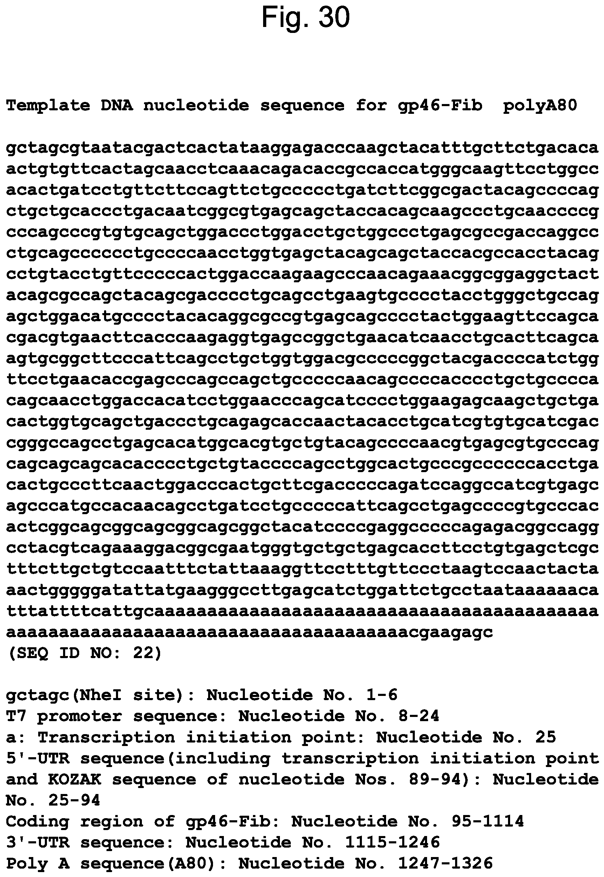

[Figure 30] Figure 30 shows the nucleotide sequence of the template DNA for gp46-Fib + polyA80 (SEQ ID NO: 22).

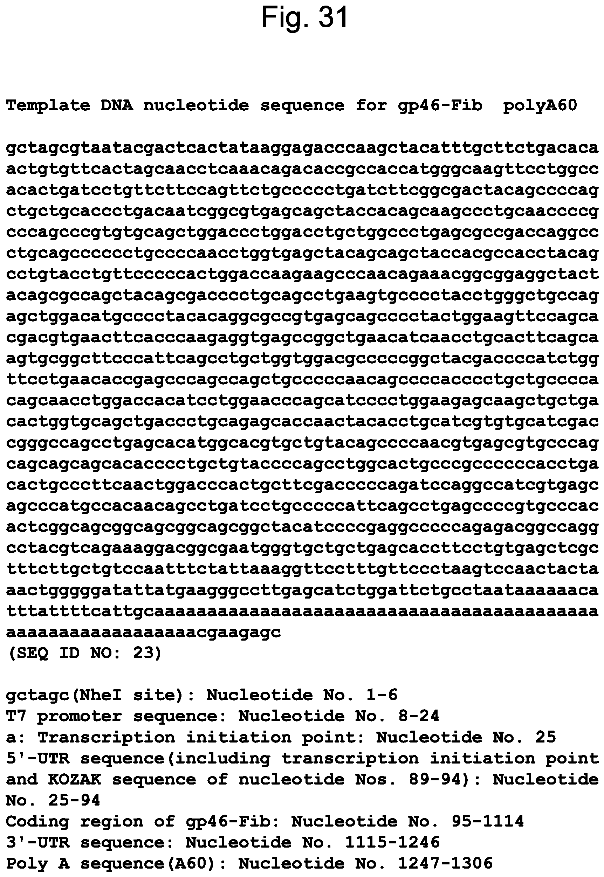

[Figure 31] Figure 31 shows the nucleotide sequence of the template DNA for gp46-Fib + polyA60 (SEQ ID NO: 23).

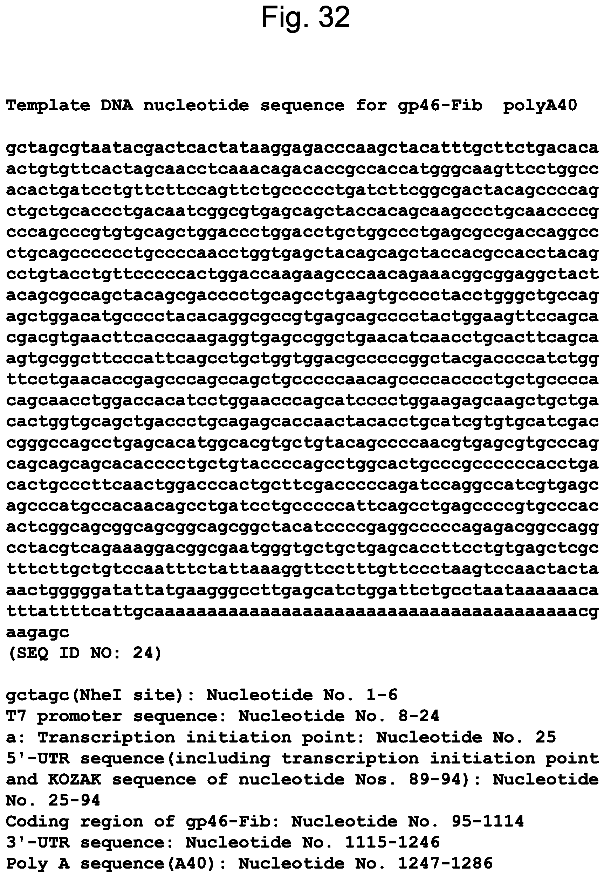

[Figure 32] Figure 32 shows the nucleotide sequence of the template DNA for gp46-Fib + polyA40 (SEQ ID NO: 24).

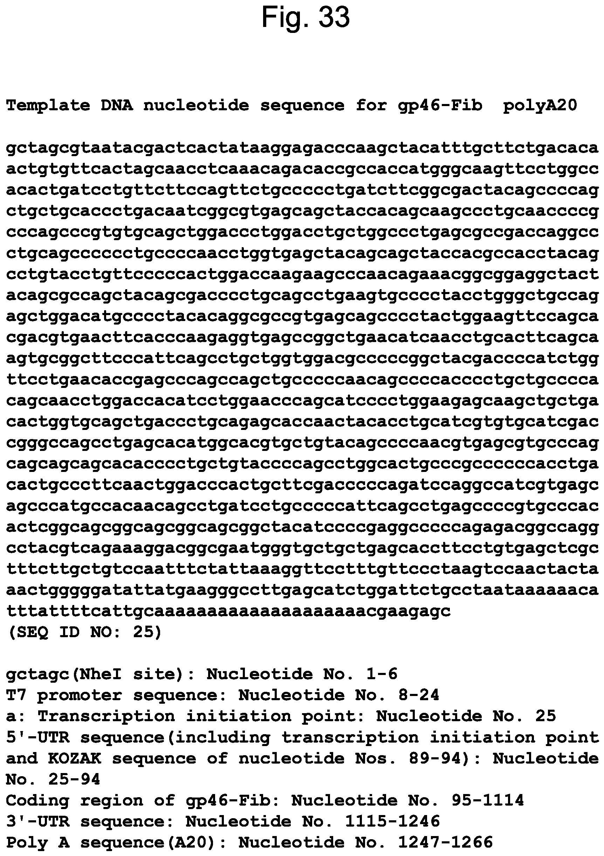

[Figure 33] Figure 33 shows the nucleotide sequence of the template DNA for gp46-Fib + polyA20 (SEQ ID NO: 25).

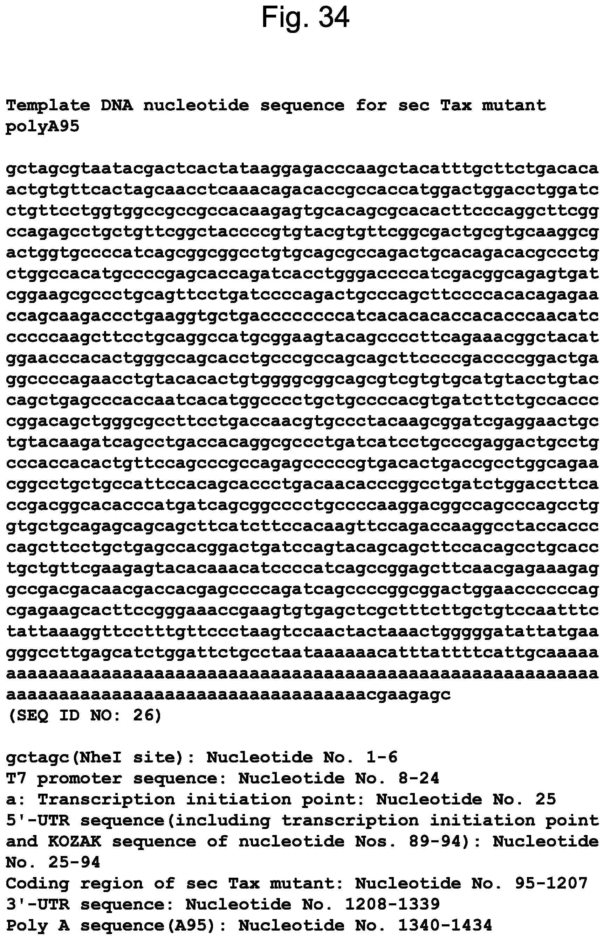

[Figure 34] Figure 34 shows the nucleotide sequence of the template DNA for sec Tax mutant + polyA95 (SEQ ID NO: 26).

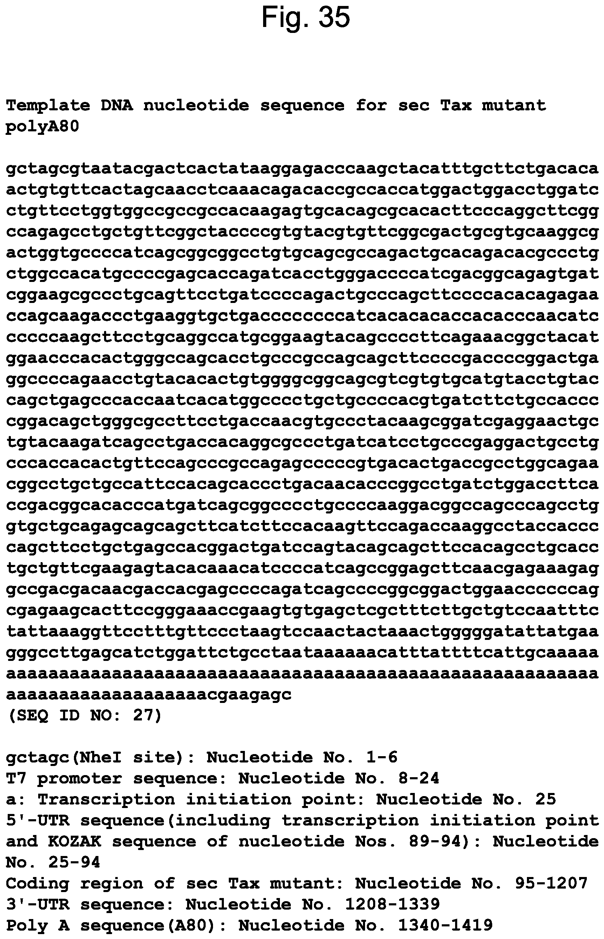

[Figure 35] Figure 35 shows the nucleotide sequence of the template DNA for sec Tax mutant + polyA80 (SEQ ID NO: 27).

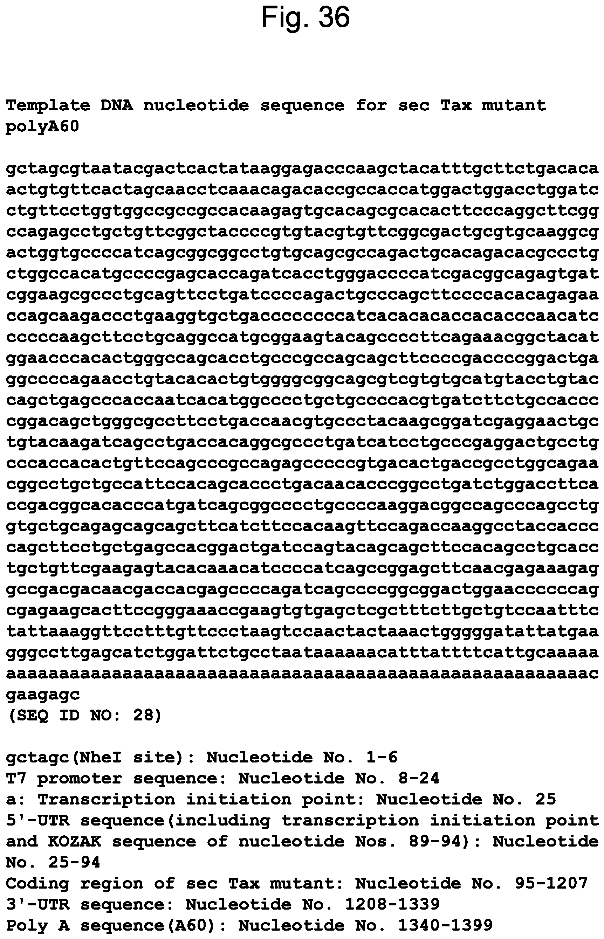

[Figure 36] Figure 36 shows the nucleotide sequence of the template DNA for sec Tax mutant + polyA60 (SEQ ID NO: 28).

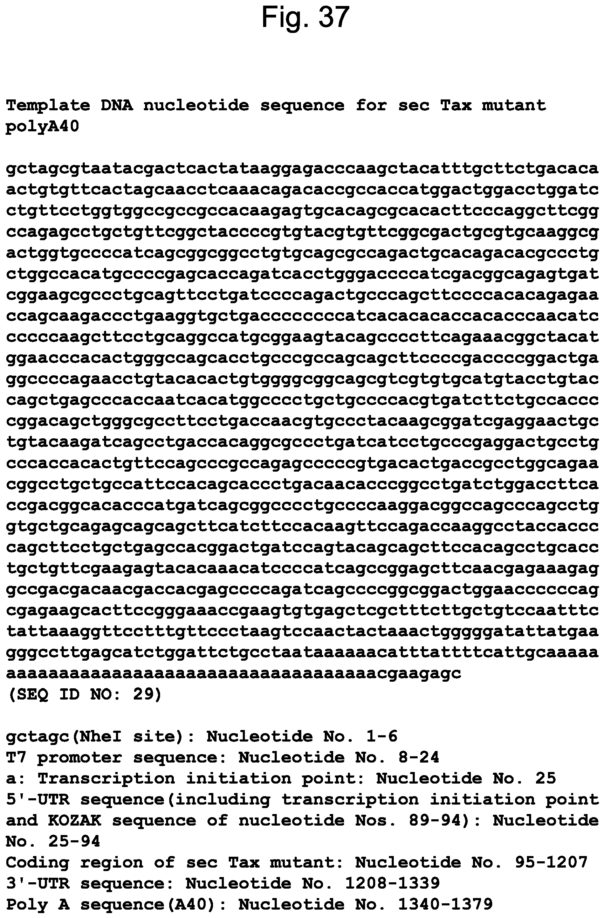

[Figure 37] Figure 37 shows the nucleotide sequence of the template DNA for sec Tax mutant + polyA40 (SEQ ID NO: 29).

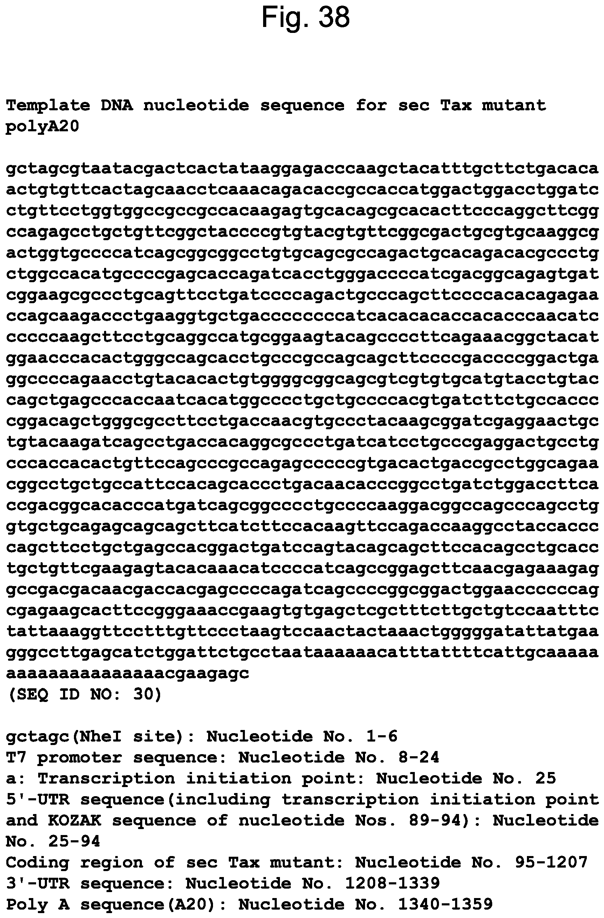

[Figure 38] Figure 38 shows the nucleotide sequence of the template DNA for sec Tax mutant + polyA20 (SEQ ID NO: 30).

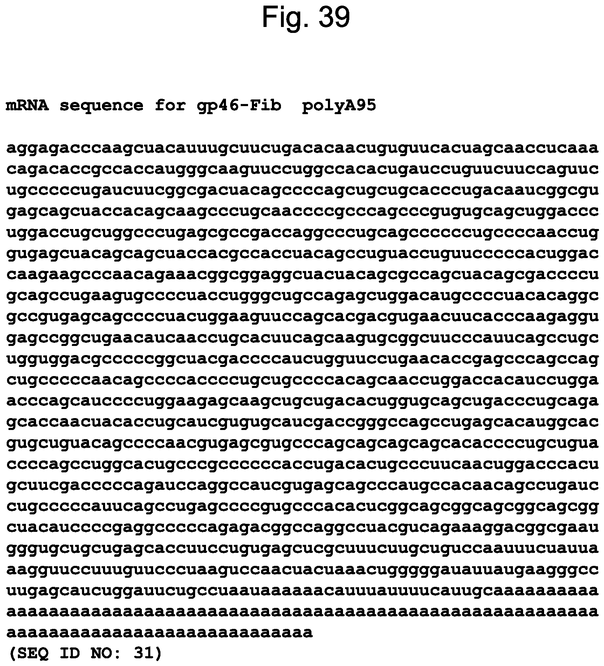

[Figure 39] Figure 39 shows the mRNA sequence of gp46-Fib + polyA95 (SEQ ID NO: 31).

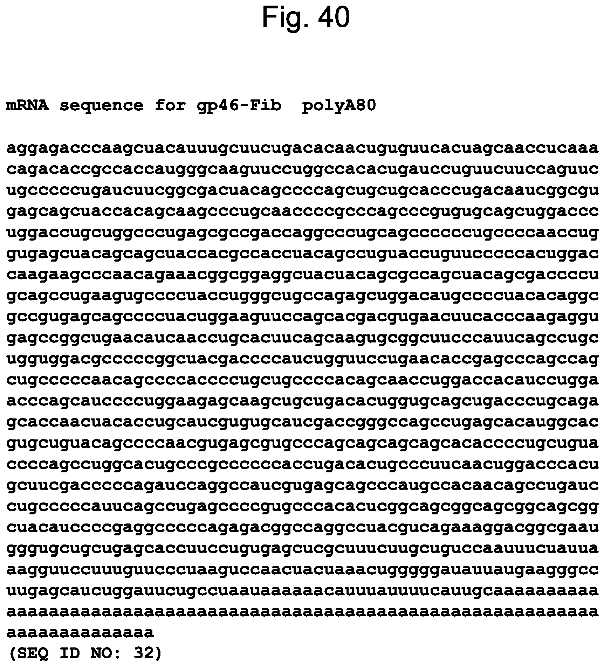

[Figure 40] Figure 40 shows the mRNA sequence of gp46-Fib + polyA80 (SEQ ID NO: 32).

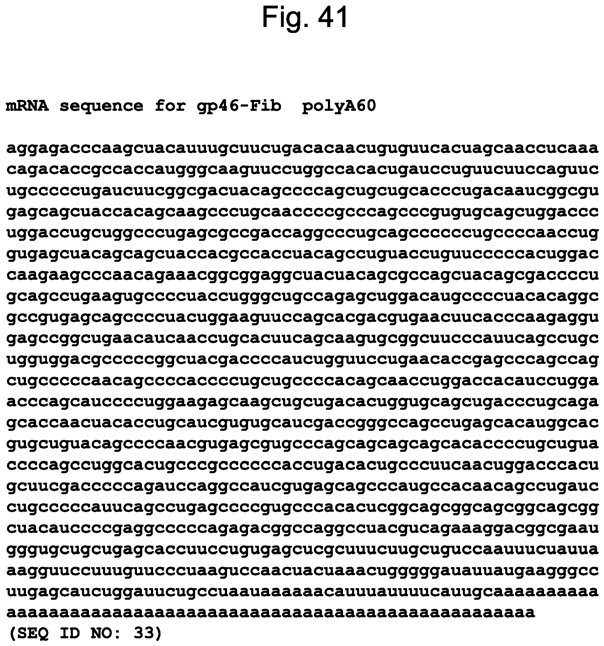

[Figure 41] Figure 41 shows the mRNA sequence of gp46-Fib + polyA60 (SEQ ID NO: 33).

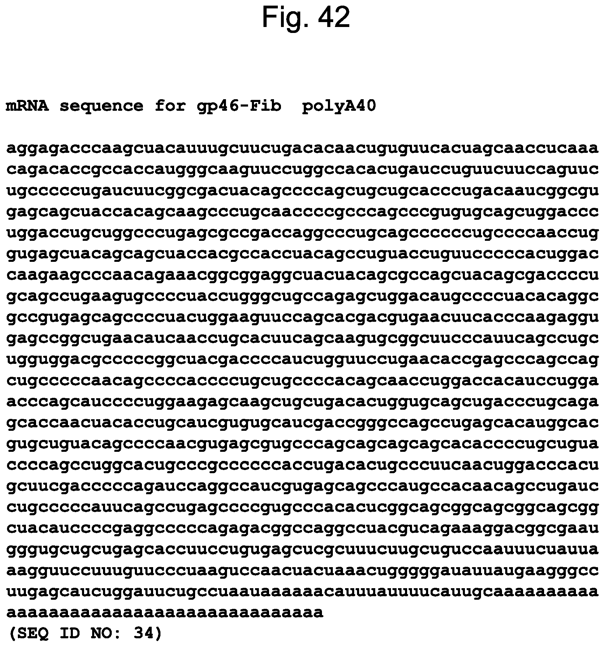

[Figure 42] Figure 42 shows the mRNA sequence of gp46-Fib + polyA40 (SEQ ID NO: 34).

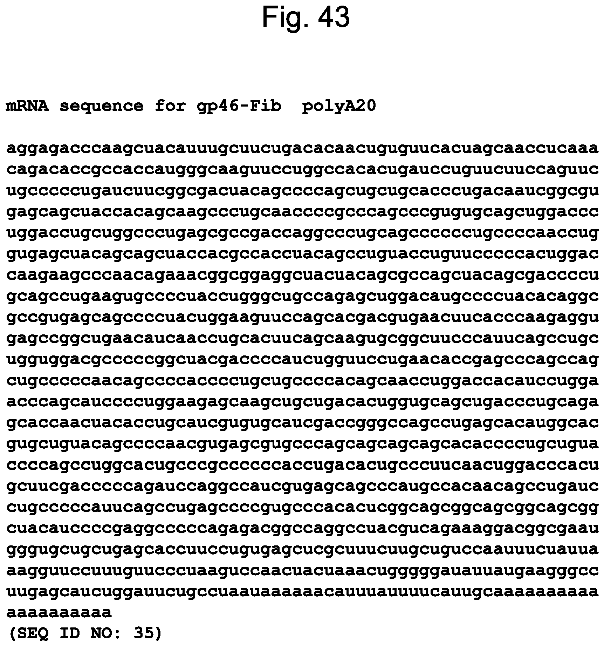

[Figure 43] Figure 43 shows the mRNA sequence of gp46-Fib + polyA20 (SEQ ID NO: 35).

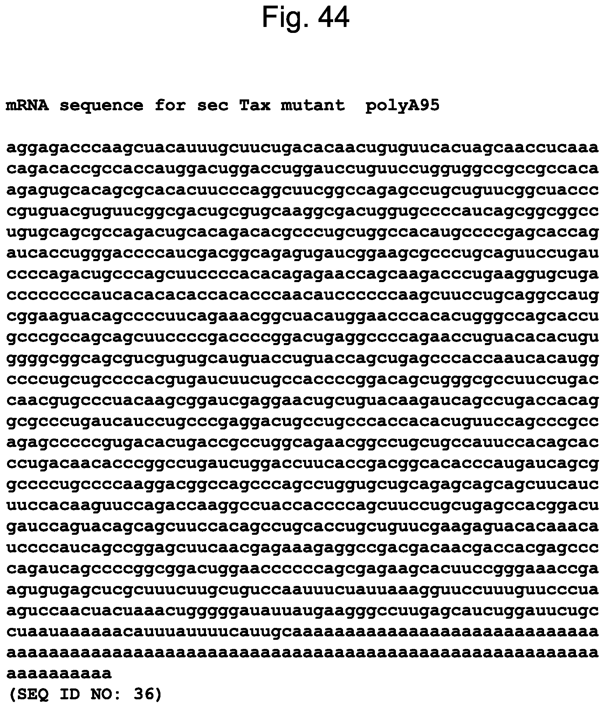

[Figure 44] Figure 44 shows the mRNA sequence of sec Tax mutant + polyA95 (SEQ ID NO: 36).

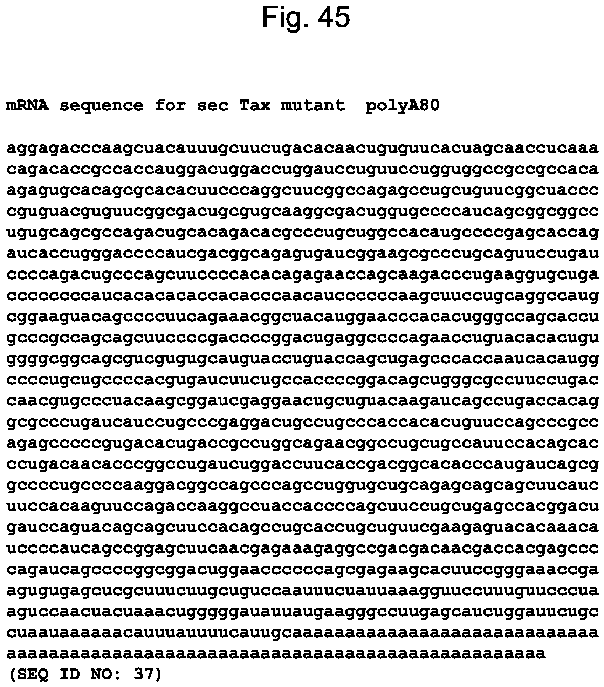

[Figure 45] Figure 45 shows the mRNA sequence of sec Tax mutant + polyA80 (SEQ ID NO: 37).

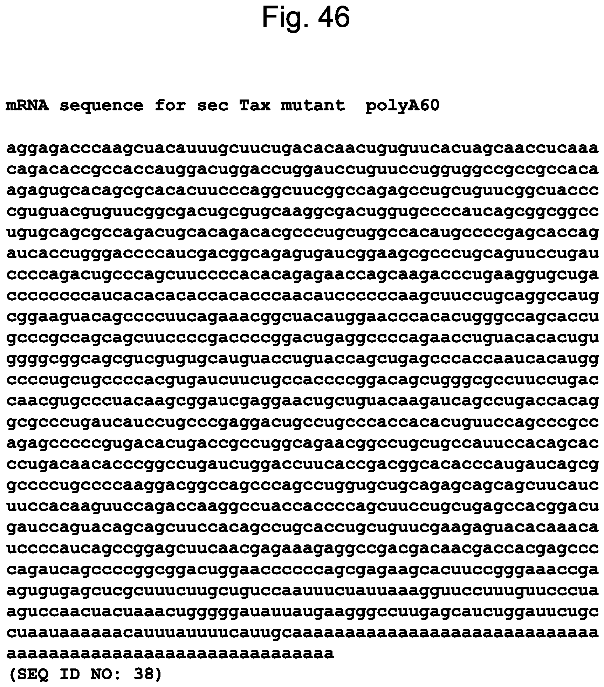

[Figure 46] Figure 46 shows the mRNA sequence of sec Tax mutant + polyA60 (SEQ ID NO: 38).

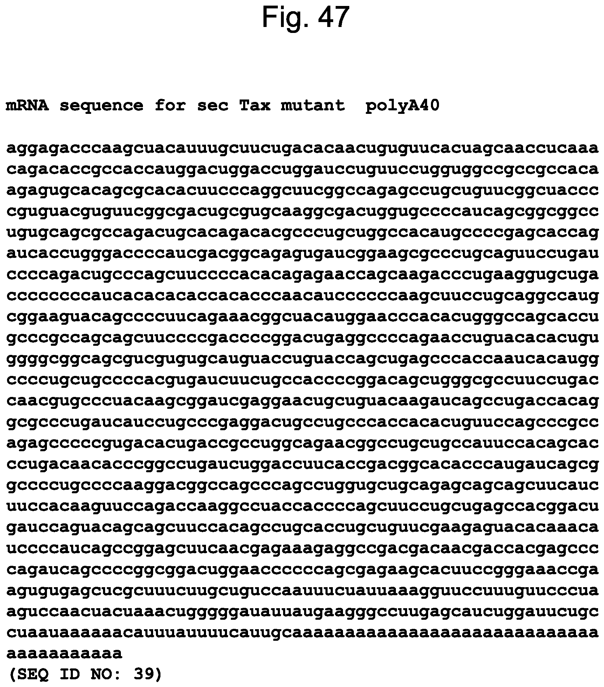

[Figure 47] Figure 47 shows the mRNA sequence of sec Tax mutant + polyA40 (SEQ ID NO: 39).

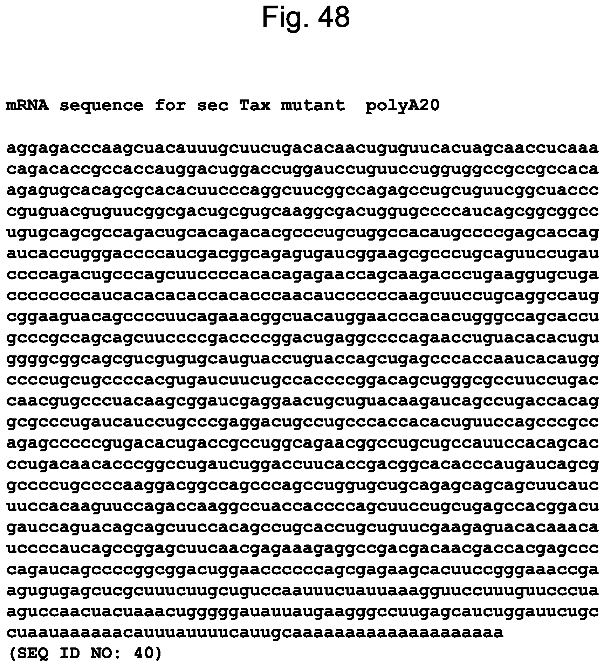

[Figure 48] Figure 48 shows the mRNA sequence of sec Tax mutant + polyA20 (SEQ ID NO: 40).

Description of Embodiments

Now, embodiments of the present invention will be more specifically described.

The present invention provides a lipid particle encapsulating a nucleic acid capable of expressing a gp46 antigen or a Tax antigen of HTLV-1, wherein the lipid comprises a cationic lipid represented by general formula (Ia):

[Formula 5]

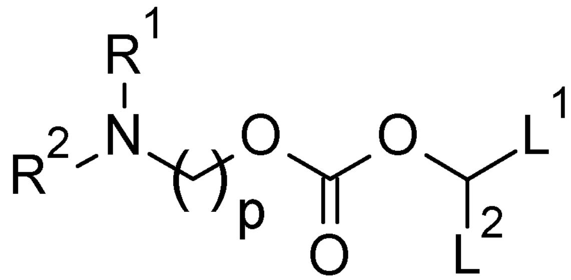

(Ia)

or a pharmaceutically acceptable salt thereof,
wherein

$R^1$ and $R^2$ each independently represent a $C_1$-$C_3$ alkyl group;
$L^1$ represents a $C_{17}$-$C_{19}$ alkenyl group optionally having one or more $C_2$-$C_4$ alkanoyloxy groups;
$L^2$ represents a $C_{10}$-$C_{19}$ alkyl group optionally having one or more $C_2$-$C_4$ alkanoyloxy groups, or a $C_{10}$-$C_{19}$ alkenyl group optionally having one or more $C_2$-$C_4$ alkanoyloxy groups; and p is 3 or 4.

In general formula (Ia), $R^1$ and $R^2$ each independently represent a $C_1$-$C_3$ alkyl group, preferably both are methyl groups.

In general formula (Ia), p is 3 or 4, preferably 3.

In general formula (Ia), $L^1$ represents a $C_{17}$-$C_{19}$ alkenyl group optionally having one or more $C_2$-$C_4$ alkanoyloxy groups, and preferably, a $C_{17}$-$C_{19}$ alkenyl group optionally having one or more acetoxy groups. Examples of the group represented by $L^1$ include an (R)-11-acetyloxy-*cis*-8-heptadecenyl group, a *cis*-8-heptadecenyl group and an (8Z,11Z)-heptadecadienyl group.

In general formula (Ia), $L^2$ represents a $C_{10}$-$C_{19}$ alkyl group optionally having one or more $C_2$-$C_4$ alkanoyloxy groups, or a $C_{10}$-$C_{19}$ alkenyl group optionally having one or more $C_2$-$C_4$ alkanoyloxy groups, and preferably, a $C_{10}$-$C_{12}$

alkyl group optionally having one or more acetoxy groups, or a $C_{10}$-$C_{19}$ alkenyl group optionally having one or more acetoxy groups. Alternatively, in general formula (Ia), $L^2$ is preferably a $C_{10}$-$C_{12}$ alkyl group optionally having one or more acetoxy groups or a $C_{17}$-$C_{19}$ alkenyl group optionally having one or more acetoxy groups. Examples of the group represented by $L^2$ include a decyl group, a *cis*-7-decenyl group, a dodecyl group, and an (R)-11-acetyloxy-cis-8-heptadecenyl group.

Examples of the cationic lipid constituting the particle of the present invention include (7R,9Z,26Z,29R)-18-({[3-(dimethylamino)propoxy]carbonyl}oxy)pentatriaconta-9,26-diene-7,29-diyl diacetate, 3-dimethylaminopropyl(9Z,12Z)-octacosa-19,22-dien-11-yl carbonate, and (7R,9Z)-18-({[3-(dimethylamino)propyloxy]carbonyl}oxy)octacosa-9-en-7-yl acetate, which are respectively shown by the following structural formulas:

[Formula 6]

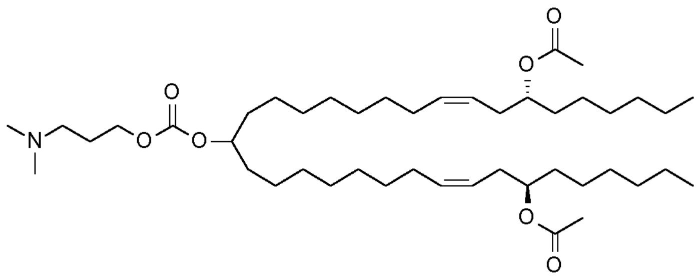

[Formula 7]

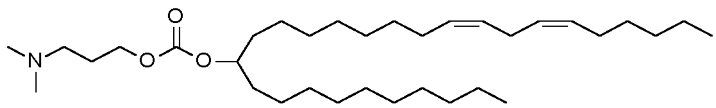

[Formula 8]

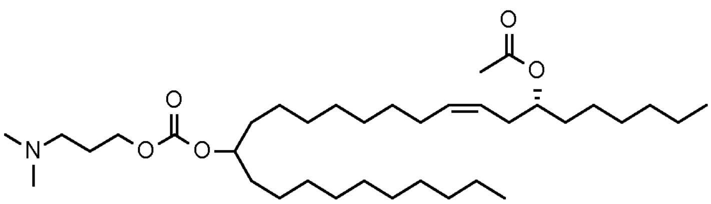

The cationic lipid represented by general formula (Ia) may be a single compound or a combination of two or more compounds.

A method for producing a cationic lipid represented by general formula (Ia) is disclosed in International Patent No. WO2015/005253.

The lipid of the present invention may further contain an amphipathic lipid, a sterol and a PEG lipid.

The amphipathic lipid is a lipid having an affinity for both polar and non-polar solvents. Examples of the amphipathic lipid include distearoylphosphatidylcholine, dioleoylphosphatidylcholine, dioleoylphosphatidylethanolamine and a combination of these. The amphipathic lipid to be used in the particle of the present invention is preferably distearoylphosphatidylcholine and/or dioleoylphosphatidylethanolamine, and more preferably, distearoylphosphatidylcholine.

The sterol refers to a sterol having a hydroxy group, such as cholesterol.

The PEG lipid is a lipid modified with PEG. Examples of the PEG lipid include 1,2-dimyristoyl-*sn*-glycerol methoxypolyethylene glycol and/or N-[methoxypoly(ethylene glycol)2000]carbamoyl]-1,2-dimyristyloxypropyl-3-amine and a combination of these. The PEG lipid is preferably 1,2-dimyristoyl-*sn*-glycerol methoxypolyethylene glycol. The average molecular weight of the PEG lipid, although it is not particularly limited, is, for example, 1000 to 5000, preferably 1500 to 3000, and more preferably, 1800 to 2200.

The lipid composition of an amphipathic lipid, a sterol, a cationic lipid and a PEG lipid, although it is not particularly limited, is as follows: for example, 5 to 25% of the amphipathic lipid; 10 to 55% of the sterol; 40 to 65% of the cationic lipid; and 1 to 5% of the PEG lipid, on a molar basis; preferably 10 to 25% of the amphipathic lipid; 10 to 55% of the

sterol; 40 to 65% of the cationic lipid; and 1 to 5% of the PEG lipid on a molar basis; and more preferably 10 to 22.5% of the amphipathic lipid, 15 to 55% of the sterol; 40 to 65% of the cationic lipid; and 1 to 5% of the PEG lipid on a molar basis. In the above lipid composition, the ratio of the PEG lipid is more preferably 1 to 3%, further more preferably 1 to 2%, further more preferably 1.2 to 2%, further more preferably 1.25 to 2%, further more preferably 1.3 to 2%, and particularly preferably 1.5 to 2% on a molar basis. In the above lipid composition, the weight ratio of the total lipid to the nucleic acid, although it is not particularly limited, may be satisfactorily 15 to 30, preferably 15 to 25, more preferably 15 to 22.5, and further more preferably 17.5 to 22.5.

When 3-dimethylaminopropyl(9Z,12Z)-octacosa-19,22-dien-11-yl carbonate or (7R,9Z,26Z,29R)-18-({[3-(dimethylamino)propoxy]carbonyl}oxy)pentatriaconta-9,26-diene-7,29-diyl diacetate is used as the cationic lipid, the lipid composition of an amphipathic lipid, a sterol, a cationic lipid and a PEG lipid, although it is not particularly limited, contains, for example, 5 to 25% of the amphipathic lipid, 10 to 55% of the sterol, 40 to 65% of the cationic lipid, and 1 to 5% of the PEG lipid on a molar basis; preferably 15% or less of the amphipathic lipid, 20 to 55% of the sterol, 40 to 65% of the cationic lipid, and 1 to 5% of the PEG lipid; more preferably 5 to 15% of the amphipathic lipid, 35 to 50% of the sterol, 40 to 55% of the cationic lipid, and 1 to 3% of the PEG lipid, on a molar basis; further more preferably 10 to 15% of the amphipathic lipid, 35 to 45% of the sterol, 40 to 50% of the cationic lipid, and 1 to 2.5% of the PEG lipid, on a molar basis; and further more preferably 10 to 15% of the amphipathic lipid, 35 to 45% of the sterol, 40 to 50% of the cationic lipid, and 1 to 2% of the PEG lipid, on a molar basis. In the above lipid composition, the content of PEG lipid is further more preferably 1.2 to 2%, further more preferably 1.25 to 2%, further more preferably 1.3 to 2%, and further more preferably 1.5 to 2%. In the above lipid composition, the weight ratio of the total lipid to the nucleic acid, although it is not particularly limited, is satisfactorily 15 to 30, preferably 15 to 25, more preferably 15 to 22.5, and further more preferably 17.5 to 22.5.

When (7R,9Z)-18-({ [3-(dimethylamino)propyloxy]carbonyl}oxy)octacosa-9-en-7-yl acetate is used as the cationic lipid, the lipid composition of an amphipathic lipid, a sterol, a cationic lipid and a PEG lipid, although it is not particularly limited, is, for example, 5 to 25% of the amphipathic lipid, 10 to 55% of the sterol, 40 to 65% of the cationic lipid, and 1 to 5% of the PEG lipid, on a molar basis; preferably 10 to 25% of the amphipathic lipid, 10 to 50% of the sterol, 40 to 65% of the cationic lipid, and 1 to 5% of the PEG lipid; more preferably 10 to 25% of the amphipathic lipid, 10 to 50% of the sterol, 40 to 65% of the cationic lipid, and 1 to 3% of the PEG lipid, on a molar basis; further more preferably 10 to 25% of the amphipathic lipid, 10 to 45% of the sterol, 42.5 to 65% of the cationic lipid, and 1 to 2.5% of the PEG lipid, on a molar basis; further more preferably 15 to 22.5% of the amphipathic lipid, 15 to 40% of the sterol, 45 to 65% of the cationic lipid, and 1 to 2% of the PEG lipid on a molar basis; and further more preferably 17.5 to 22.5% of the amphipathic lipid, 15 to 40% of the sterol, 45 to 65% of the cationic lipid, and 1 to 2% of the PEG lipid on a molar basis. In the above lipid composition, the content of PEG lipid is further more preferably 1.2 to 2%, further more preferably 1.25 to 2%, further more preferably 1.3 to 2%, and further more preferably 1.5 to 2%. In the above lipid composition, the weight ratio of the total lipid to the nucleic acid, although it is not particularly limited, is preferably 15 to 30, more preferably 15 to 25, further more preferably 15 to 22.5, and further more preferably 17.5 to 22.5.

The combination of the lipids in the present invention to be used may consist of an amphipathic lipid such as distearoylphosphatidylcholine, dioleoylphosphatidylcholine, or dioleoylphosphatidylethanolamine, a sterol such as cholesterol, a cationic lipid such as (7R,9Z,26Z,29R)-18-({[3-(dimethylamino)propoxy]carbonyl}oxy)pentatriaconta-9,26-diene-7,29-diyl diacetate, 3-dimethylaminopropyl(9Z,12Z)-octacosa-19,22-dien-11-yl carbonate, or (7R,9Z)-18-({[3-(dimethylamino)propyloxy]carbonyl}oxy)octacosa-9-en-7-yl acetate, and a PEG lipid such as 1,2-dimyristoyl-*sn*-glycerol methoxypolyethylene glycol or N-[methoxypoly(ethylene glycol)2000]carbamoyl]-1,2-dimyristyloxypropyl-3-amine. A preferable combination of the lipids to be used consists of an amphipathic lipid such as distearoylphosphatidylcholine or dioleoylphosphatidylethanolamine, a sterol such as cholesterol, a cationic lipid such as (7R,9Z,26Z,29R)-18-({[3-(dimethylamino)propoxy]carbonyl}oxy)pentatriaconta-9,26-diene-7,29-diyl diacetate, or (7R,9Z)-18-({[3-(dimethylamino)propyloxy]carbonyl}oxy)octacosa-9-en-7-yl acetate, and a PEG lipid such as 1,2-dimyristoyl-sn-glycerol methoxypolyethylene glycol. A more preferable combination of the lipids to be used in the present invention consists of an amphipathic lipid such as distearoylphosphatidylcholine, a sterol such as cholesterol, a cationic lipid such as (7R,9Z,26Z,29R)-18-({[3-(dimethylamino)propoxy]carbonyl}oxy)pentatriaconta-9,26-diene-7,29-diyl diacetate or (7R,9Z)-18-({[3-(dimethylamino)propyloxy]carbonyl}oxy)octacosa-9-en-7-yl acetate, and a PEG lipid such as 1,2-dimyristoyl-*sn*-glycerol methoxypolyethylene glycol.

In the present invention, the nucleic acid to be encapsulated in a lipid particle is a nucleic acid capable of expressing a gp46 antigen or Tax antigen of HTLV-1.

The amino acid sequence of the gp46 antigen of HTLV-1 is represented by SEQ ID NO: 14. The nucleic acid to be encapsulated in a lipid particle is preferably a nucleic acid encoding the gp46 antigen of HTLV-1, which consists of an amino acid sequence having an identity of at least 95%, preferably 96%, more preferably 97%, and more preferably 98% with the amino acid sequence of SEQ ID NO: 14.

The gp46 antigen may be a fusion protein with an oligomerization domain. The oligomerization domain is a protein responsible for multimerization of a protein fused thereto. Examples of the oligomerization domain include fibritin

derived from T4 bacteriophage. Also, a trimeric domain of fibritin, a foldon domain, can be used. The amino acid sequence of a fusion protein of gp46 of HTLV-1 and fibritin is represented by SEQ ID NO: 15. The amino acid sequence from positions 313 to 339 in the amino acid sequence of SEQ ID NO: 15 is the amino acid sequence of fibritin. The oligomerization domain may be positioned at the C-terminal or N-terminal of the gp46 antigen.

The amino acid sequence of the wild-type Tax antigen of HTLV-1 is represented by SEQ ID NO: 11. The nucleic acid to be encapsulated in a lipid particle is preferably a nucleic acid encoding the Tax antigen of HTLV-1, which consists of an amino acid sequence having an identity of at least 95%, preferably 96%, and more preferably 97% with the amino acid sequence of SEQ ID NO: 11. Since the wild-type Tax antigen has HTLV-1 LTR transcriptional activity and carcinogenicity, toxicity, i.e., carcinogenicity, to a living body must be reduced or eliminated. For example, a Tax antigen having a mutation, by which carcinogenicity deteriorates or disappears, may be used. Since when Tax is secreted outside a cell, the carcinogenicity to the cells deteriorates, Tax may be a fusion protein with a signal peptide. Examples of the mutation, by which carcinogenicity deteriorates or disappears, include T130A, L131S, L319R and L320S. T130A and L131S are mutations, by which HIV-1 LTR and HTLV-1 transcriptional activities are damaged, whereas, L319R and L320S are mutations, by which HTLV-1 transcriptional activity is eliminated. The Tax antigen is satisfactory if it has at least one of these mutations, preferably 2, more preferably 3, and particularly preferably 4 mutations. The amino acid sequence of the mutant-type Tax antigen having the 4 mutations mentioned above is represented by SEQ ID NO: 12. As a signal peptide for secreting the Tax antigen outside a cell, an IgE secretory signal peptide is mentioned. The signal peptide is preferably fused to the N-terminal side of the Tax antigen. The amino acid sequence of a fusion protein of the signal peptide and the mutant-type Tax antigen having the 4 mutations is represented by SEQ ID NO: 13. The amino acid sequence from positions 1 to 18 in the amino acid sequence of SEQ ID NO: 13 is the amino acid sequence of an IgE secretory signal peptide.

The present invention includes a peptide of a secretory Tax mutant prepared by adding an IgE secretory signal to a Tax mutant, which is prepared by fusing a Tax antigen having at least one, preferably 2, more preferably 3, and particularly preferably 4 of the 4 amino acid mutations, to a signal peptide. The peptide is a peptide prepared by fusing the Tax antigen of human T-cell leukemia virus type 1 (HTLV-1) having a mutation, by which carcinogenicity deteriorates or disappears, and a signal peptide. Examples of the mutation, by which carcinogenicity deteriorates or disappears, include T130A, L131S, L319R and L320S. More specifically, the above peptide of the Tax antigen, is, for example, a peptide having at least one mutation selected from the group consisting of T130A, L131S, L319R and L320S relative to the amino acid sequence of SEQ ID NO: 11 and consisting of an amino acid sequence having an identity of at least 95%, preferably 96%, and more preferably 97% with the amino acid sequence of SEQ ID NO: 11 when the sequence consisting of amino acids except the mutant amino acids. The signal peptide for secreting the Tax antigen outside a cell is, e.g., an IgE secretory signal peptide. The signal peptide is fused preferably to the N-terminal side of the Tax antigen. The amino acid sequence from positions 1 to 18 in the amino acid sequence of SEQ ID NO: 13 is the amino acid sequence of an IgE secretory signal peptide. More specifically, the above peptide is, e.g., a peptide consisting of an amino acid sequence from positions 1 to 18 in the amino acid sequence of SEQ ID NO: 13.

In the present invention, the nucleic acid to be encapsulated in a lipid particle may be a nucleic acid capable of expressing a protein prepared by binding the gp46 antigen and the Tax antigen of HTLV-1 via a linker. The linker may be a linker consisting of an amino acid sequence containing a sequence cleavable with a protease, more specifically, containing an amino acid sequence that is recognized/cleaved by a protease, Furin. The sequence cleavable with a protease is not limited as long as it can be cleaved by a Furin protein. Examples of the sequence cleavable with a protease include a sequence represented by R-X-K/R-R (R represents arginine, K lysine, X arbitrary amino acid)(J. Biol. Chem. 1992, 267, 16396; J. Biol. Chem. 1991, 266, 12127).

The identity of an amino acid sequence refers to a numerical value representing a matching rate of amino acids to the full-length sequence, provided that sequences in which the corresponding amino acids completely match are defined as the same amino acid sequences. The sequence identity in the present invention is calculated using sequence analysis software, GENETYX-SV/RC (made by Genetics Co., Ltd.). The algorithm employed in the software is commonly used in the technical field. The amino acid sequence encoded by the nucleic acid to be encapsulated in a lipid particle of the present invention may have a mutation (substitution), deletion, insertion and/or addition of an amino acid, as long as the amino acid sequence has an identity above a certain percentage with those of SEQ ID NOs: 11 to 16.

The amino acid sequence encoded by the nucleic acid to be encapsulated in a lipid particle of the present invention and having the aforementioned sequence identity, may have a substitution, deletion, insertion and/or addition at several sites (preferably 5 sites or less, more preferably 3, 2 or 1 site) at a ratio of several amino acids per site (preferably 10 or less, more preferably 7 or less, further preferably 5, 4, 3, 2 or 1 amino acid), in each of the amino acid sequences of SEQ ID NOs: 11 to 16.

The nucleic acid capable of expressing the gp46 antigen or Tax antigen of HTLV-1 is preferably mRNA containing a cap structure (Cap), a 5'-noncoding region (5'-UTR), a gp46 or Tax coding region, a 3'-noncoding region (3'-UTR) and a poly A tail (polyA). The KOZAK sequence may be present 5' to the gp46 or Tax coding region. The cap structure (Cap) is a site present on the 5'-end of mRNA molecules in many eukaryotes and having a 7-methyl guanosine structure.

Examples of the cap structure include Cap-0, Cap-1, Cap-2 and a cap structure using ARCA (Anti-Reverse Cap Analog). The cap structures are shown in the following formulas.

[Formula 9]

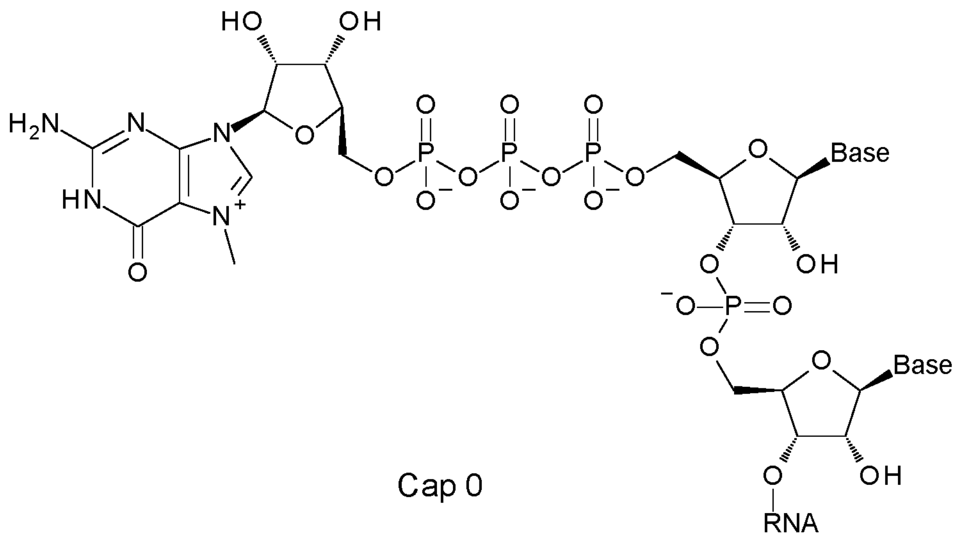

Cap 0

wherein "Base" represents an unmodified or modified nucleobase, and RNA represents a polynucleotide.

[Formula 10]

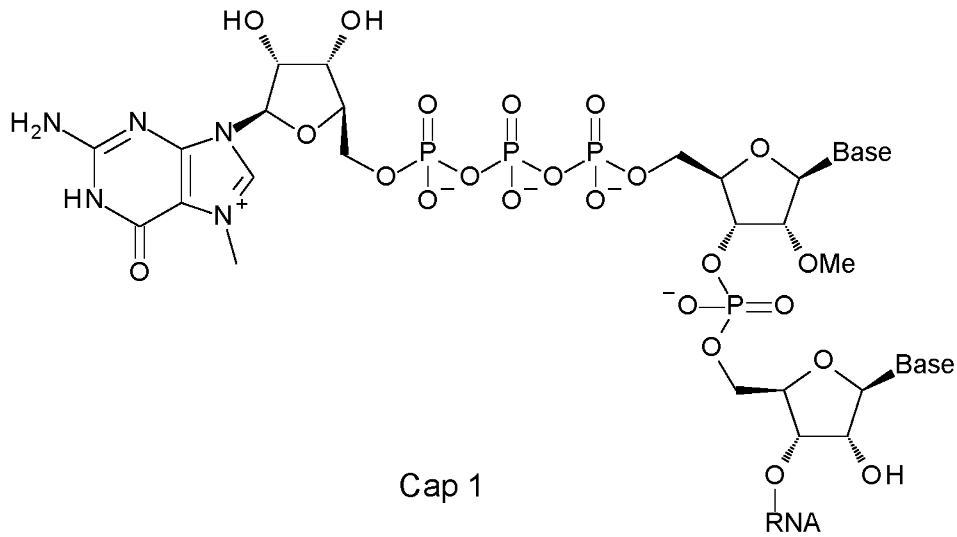

Cap 1

[Formula 11]

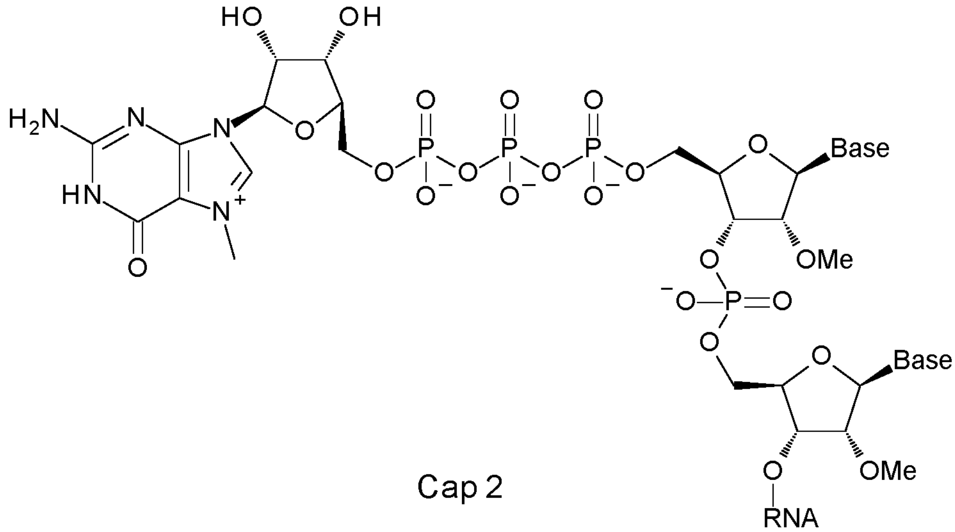

Cap 2

[Formula 12]

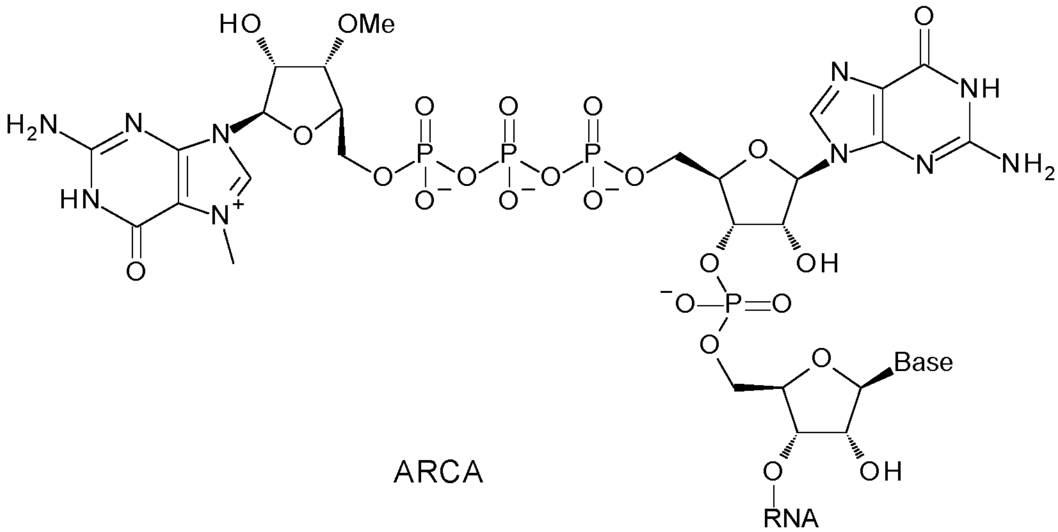

ARCA

The cap structure of mRNA of the present invention is preferably Cap-0 or Cap-1, and more preferably, Cap-1. As the sequence of the 5'-noncoding region (5'-UTR), for example, a sequence containing the 5'-noncoding region of the human β globin gene can be used. For example, the sequence of the 5'-noncoding region of the human β globin gene is a sequence of nucleotide Nos. 15 to 64 in the sequence of SEQ ID NO: 17. The sequence of the coding region for gp46 is a sequence capable of expressing all or part of the amino acid sequence of the gp46 antigen; may contain an initiation codon and/or a termination codon; and is, for example, the sequence of nucleotide Nos. 71 to 1009 in the sequence of SEQ ID NO: 17. The sequence of the coding region for gp46 may be a sequence to which the sequence of fibritin is ligated, for example, the sequence of nucleotide Nos. 71 to 1090 in SEQ ID NO: 18. The sequence of the coding region for dgp62 is a sequence capable of expressing all or part of the amino acid sequence of the dgp62 antigen; may contain an initiation codon and/or a termination codon; and is, for example, the sequence of nucleotide Nos. 71 to 1396 in the sequence of SEQ ID NO: 19. The sequence of the coding region for dgp62 may be a sequence to which the sequence of fibritin is ligated, for example, represented by the sequence of nucleotide Nos. 71 to 1480 in the sequence of SEQ ID NO: 19. The sequence of the coding region for Tax is a sequence capable of expressing all or part of the amino acid sequence of the Tax antigen; and may contain an initiation codon and/or a termination codon. The Tax antigen contains 4 mutations such as T130A, L131S, L319R and L320S4. A sequence obtained by ligating the sequence of the coding region of mutant-type Tax antigen having the 4 mutations and a sequence encoding an IgE secretory signal

peptide for secreting the Tax antigen outside a cell is mentioned, that is, the sequence of nucleotide Nos. 71 to 1183 in the sequence of SEQ ID NO: 20. As the sequence of the 3'-noncoding region (3'-UTR), for example, the 3'-noncoding region of the human β globin gene can be used. The sequence is, for example, the sequence of nucleotide Nos. 1010 to 1141 in the sequence of SEQ ID NO: 17. The sequence of poly A tail (polyA) is, for example, a sequence of nucleotide Nos. 1142 to 1241 in the sequence of SEQ ID NO: 17. The sequences of the cap structure (Cap), 5'-noncoding region (5'-UTR), gp46 or Tax coding region, 3'-noncoding region (3'-UTR) and poly A tail (polyA) may be modified. The sequence of the nucleic acid capable of expressing the gp46 antigen satisfactorily consists of a nucleotide sequence having an identity of at least 90%, preferably 95% and more preferably 97% with the sequence of SEQ ID NO: 17. The sequence of the nucleic acid capable of expressing the Tax antigen satisfactorily consists of a nucleotide sequence having an identity of at least 90%, preferably 95% and more preferably 97% with the sequence of SEQ ID NO: 20.

The length of the poly A tail, although it is not limited, is for example, a length of 10 to 250 nucleotides, preferably 15 to 120 nucleotides, further preferably 15 to 115 nucleotides, and particularly preferably 20 to 110 nucleotides.

The mRNA of the present invention has a sequence containing the cap structure (Cap), 5'-noncoding region (5'-UTR), gp46 or Tax coding region and 3'-noncoding region (3'-UTR). The portion consisting of the cap structure (Cap), 5'-noncoding region (5'-UTR), gp46 antigen or Tax antigen coding region and 3'-noncoding region (3'-UTR) may be mRNA consisting of a nucleotide sequence having an identity of at least 90%, preferably 95%, and more preferably 97% with the sequence from positions 1 to 1141 in SEQ ID NO: 17, the sequence from positions 1 to 1222 in SEQ ID NO: 18 or the sequence from positions 1 to 1315 in SEQ ID NO: 20.

The nucleic acid to be encapsulated in a lipid particle may be present in any form as long as it is a nucleic acid expressing the gp46 antigen or Tax antigen of HTLV-1. Examples of the form of the nucleic acid include a single-stranded DNA, single-stranded RNA (for example, mRNA), a single-stranded polynucleotide consisting of a combination of DNA and RNA, double-stranded DNA, double-stranded RNA, a hybrid polynucleotide consisting of DNA-RNA, and a double-stranded polynucleotide consisting of two types of polynucleotides, i.e., a combination of DNA and RNA. The nucleic acid is preferably mRNA.

The nucleotides constituting the nucleic acid to be encapsulated in a lipid particle may be natural-type nucleotides or modified nucleotides. At least one of the modified nucleotides is preferably contained.

The modified nucleotide is satisfactory as long as any one of a nucleotide, a sugar and a phosphodiester bond is modified. The number of modification sites may be 1 or 2 or more.

Examples of the nucleotide modification include 5-methylation of cytosine, 5-fluorination, N4-methylation, 5-methylation (thymine) of uracil, 5-fluorination, N6-methylation of adenine and N2-methylation of guanine.

Examples of the sugar modification include 2'-O-methylation of D-ribofuranose.

Examples of the modified phosphodiester-bond include a phosphorothioate bond.

The modified nucleotide is preferably a nucleotide having a modified nucleobase. For example, a pyrimidine nucleotide substituted at position 5, and pseudouridine optionally substituted at position 1 are satisfactory. Examples of the modified nucleotide may be 5-methylcytidine, 5-methoxyuridine, 5-methyluridine, pseudouridine, and a 1-alkylpseudouridine. The 1-alkylpseudouridine may be 1-(C1-C6 alkyl)pseudouridine, and preferably, 1-methylpseudouridine or 1-ethyl pseudouridine. As more preferable modified nucleotide, 5-methylcytidine, 5-methyluridine, and 1-methylpseudouridine are mentioned. As particularly preferable modified nucleotide, a combination of 5-methylcytidine and 5-methyluridine, or a combination of 5-methylcytidine and 1-methylpseudouridine is mentioned.

The nucleic acid capable of expressing the gp46 antigen or Tax antigen of HTLV-1 of the present invention can be produced from DNA having a desired nucleotide sequence by an *in-vitro* transcription reaction. The reagents required for the *in-vitro* transcription such as enzyme, buffer, and, a nucleoside-5'-triphosphate mixture (adenosine-5'-triphosphate (ATP), guanosine-5'-triphosphate (GTP), cytidine-5'-triphosphate (CTP) and uridine-5'-triphosphate (UTP)) are commercially available (e.g., AmpliScribeT7 High Yield Transcription Kit (Epicentre), mMESSAGE mMACHINE T7 Ultra Kit (Life technologies). The DNA for use in producing a single-stranded RNA may be cloned DNA such as plasmid DNA or a DNA fragment. The plasmid DNA or DNA fragment may be a commercially available one or can be produced by a method commonly known in the technical field (for example, method disclosed in Sambrook, J. et al., Molecular Cloning a Laboratory Manual second edition (1989), Rashtchian, A., Current Opinion in Biotechnology, 1995, 6 (1), 30-36, Gibson D. G. et al., Science, 2008, 319 (5867), 1215-1220).

In order to obtain mRNA improved in stability and/or safety, part or whole unmodified nucleotides in mRNA may be replaced with modified nucleotides, by substituting part or whole unmodified nucleoside-5'-triphosphate with modified nucleoside-5'-triphosphate in *in-vitro* transcription reaction (Kormann, M., Nature Biotechnology, 2011, 29, 154-157).

In order to obtain mRNA improved in stability and/or safety, a cap structure (Cap-0 structure mentioned above) can be introduced into the 5'-end of mRNA by a method using a capping enzyme after the *in-vitro* transcription reaction. Cap-0 may be converted into Cap-1 by a method of allowing 2'-O-methyltransferase to act on mRNA having Cap-0. As the capping enzyme and 2'-O-methyltransferase, commercially available ones (for example, Vaccinia Capping System, M2080; mRNA Cap 2'-O-Methyltransferase, M0366, both manufactured by New England Biolab) may be used. When

a commercially available product is used, mRNA having a cap structure can be produced in accordance with the protocol attached to the product.

The cap structure at the 5'-end of mRNA can be introduced also by a method different from the enzymatic method. For example, a cap analogous structure that ARCA has or a Cap-1 structure derived from CleanCap can be introduced into mRNA by adding ARCA or CleanCap (registered trademark) in an *in-vitro* transcription reaction. As the ARCA and CleanCap, commercially available products can be used (ARCA, N-7003; CleanCap Reagent AG, N-7113, both are manufactured by TriLink BioTechnologies). When a commercially available product is used, mRNA having a cap structure can be manufactured in accordance with the protocol attached to the product.

In the present invention, the nucleic acid to be encapsulated in a lipid particle may be purified with a method such as desalting, HPLC (reverse phase, gel filtration, ion exchange, affinity), PAGE and ultrafiltration. Since impurities are removed by a purification treatment, production of inflammatory cytokines can be decreased in a living body administered with the nucleic acid.

The nucleic acid-encapsulated lipid particle of the present invention can be produced by a method such as a thin-film method, a reverse phase evaporation method, an ethanol injection method, an ether injection method, a dehydration-rehydration method, a surfactant dialysis method, a hydration method and a freeze-thaw method. For example, the nucleic acid-encapsulated lipid particle can be produced in accordance with a method disclosed in International Publication No. WO2015/005253. The nucleic acid-encapsulated lipid particle of the present invention can be produced by mixing a nucleic acid solution and a lipid solution in a micro-channel such as NanoAssemblr (registered trademark) of Precision Nanosystems, in accordance with the method set forth in the protocol attached thereto.

The average particle size of the particles of the present invention is satisfactory 30 nm to 300 nm, preferably 30 to 200 nm, and more preferably 30 to 100 nm. The average particle size can be obtained by determining a volume average particle size by a device such as Zeta Potential/Particle Sizer NICOMP (registered trademark) 380ZLS (PARTICLE SIZING SYSTEMS) and based on the principle of dynamic light scattering.

The particle of the present invention can be used for producing a composition for preventing and/or treating a disease caused by HTLV-1 infection. Examples of the disease caused by HTLV-1 infection include adult T cell leukemia (ATL), HTLV-1-associated myelopathy (HAM) and HTLV-1 associated uveitis (HU). A nucleic acid capable of expressing the gp46 antigen of HTLV-1 and a nucleic acid capable of expressing the Tax antigen of HTLV-1 may be encapsulated in the same lipid particle or different lipid particles, and preferably encapsulated separately in different lipid particles.

Using the particle of the present invention, the gp46 antigen and Tax antigen of HTLV-1 can be expressed *in vivo* or *in vitro.* More specifically, a lipid particle encapsulating a nucleic acid capable of expressing the gp46 antigen of HTLV-1 and a lipid particle encapsulating a nucleic acid capable of expressing the Tax antigen of HTLV-1 may be administered to a subject. Accordingly, the present invention provides a method for expressing the gp46 antigen and Tax antigen of HTLV-1 *in vitro,* comprising introducing a composition containing the particle into a cell. The present invention also provides a method for expressing the gp46 antigen and Tax antigen of HTLV-1 *in vivo,* comprising administering a composition containing the particle to a mammal. An immune response to HTLV-1 can be induced by expressing the gp46 antigen and Tax antigen of HTLV-1 *in vivo.* As a result, HTLV-1 infection can be prevented and/or treated. Accordingly, the present invention provides a method for inducing an immune response to HTLV-1, comprising administering a composition containing the particle to a mammal. The present invention also provides a method for preventing and/or treating HTLV-1 infection, comprising administering a composition containing the particle to a mammal. The present invention comprises a kit comprising both of the lipid particle encapsulating a nucleic acid capable of expressing the gp46 antigen of HTLV-1 and the lipid particle encapsulating a nucleic acid capable of expressing the Tax antigen of HTLV-1.

The particle of the present invention can be used as a medical drug or a laboratory reagent. The particle of the present invention is usually added to a carrier such as water, buffer and saline, and then, the mixture (composition) can be introduced into a cell (*in vitro*) or administered to a mammal (*in vivo*). When the particle is administered to a mammal, a pharmaceutically acceptable carrier (for example, saline) is preferably used as a carrier. The particle of the present invention may be mixed with a base such as fat, fatty oil, lanolin, Vaseline, paraffin, wax, resin, plastic, glycol, higher alcohol, glycerin, water, emulsifier and suspending agent to produce a dosage form such as a cream, a paste, an ointment, a gel and a lotion.

The particle of the present invention may be orally or parenterally (such as intramuscular administration, intravenous administration, intrarectal administration, transdermal administration, transmucosal administration, subcutaneous administration or intradermal administration) administered to a mammal such as a human, a mouse, a rat, a hamster, a guinea pig, a rabbit, a pig, a monkey, a cat, a dog, a horse, a goat, a sheep, and a cow.

The particle of the present invention is administered to a human at a dose of, for example, about 0.001 to 1 mg, preferably 0.01 to 0.2 mg per time per adult on a weight basis of mRNA, once or several times, preferably by intramuscular injection, subcutaneous injection, intradermal injection, intravenous drip or intravenous injection. The dose and frequency of administration herein can be appropriately varied depending on the type of disease, symptom, age, and administration method.

When the particle of the present invention is used as an experimental reagent, the particle is introduced into a cell in which the gp46 antigen and Tax antigen of HTLV-1 are desirably expressed (for example, HEK293 cell and a cell derived therefrom (HEK293T cell, FreeStyle 293 cell, Expi293 cell), CHO cell, C2C12 mouse myoblast, immortalized mouse dendritic cell (MutuDC1940)). In this manner, the gp46 antigen and Tax antigen of HTLV-1 can be expressed *in vitro.*

Expression of the gp46 antigen and Tax antigen of HTLV-1 can be analyzed by detecting the gp46 antigen protein and Tax antigen protein of HTLV-1 in a sample by western blotting or detecting a peptide fragment specific to the gp46 antigen and Tax antigen of HTLV-1 by mass spectrometry.

In the present invention, "treatment" refers to symptomatic recovery, remission, symptomatic relief and/or delaying the exacerbation of a disease in a patient having an infection caused by, e.g., a virus or bacterium, or a disease (for example, precancer lesion and cancer) caused by the infection.

In the present invention, "prevention" refers to reducing an incidence rate of a disease due to an infection with, e.g., a virus or bacterium. The prevention includes reducing the risk of progression of a disease due to an infection with, e.g., a virus or a bacterium, or reducing exacerbation of a disease. Since the particle of the present invention induces a protective immune response, the particle exerts an effect on the prevention and/or treatment of a disease as mentioned above.

It is expected that the particle of the present invention is used as drugs for prevent the onset of and treating disease caused by HTLV-1 in HTLV-1 infected persons. Examples of the disease caused by HTLV-1 include adult T cell leukemia/lymphoma (ATLL), HTLV-1 associated myelopathy (HAM) and HTLV-1 uveitis (HU).

## Examples

Now, the present invention will be more specifically described by way of Examples. Note that, these Examples are just for explaining the present invention and thus will not be construed as limiting the range of the present invention.

In Examples, the following brevity codes are sometimes used.

SU gp46: represents surface-unit gp46 of HTLV-1 envelope protein.
gp46-Fib: represents a fusion protein of gp46 and the C-terminal region of Fibritin.
dgp62-Fib: represents a fusion protein of a protein, which is obtained by removing a transmembrane region and intracellular region from gp62, and the C-terminal region of Fibritin.
sec Tax mutant: represents a fusion protein of a mutation-introduced Tax protein and a secretory signal protein.

[Example 1] Preparation of SU gp46 mRNA-001

(1) Preparation of template DNA for *in-vitro* transcription (IVT) of SU gp46

For preparing template DNA for use in *in-vitro* transcription (IVT), SU gp46 DNA was amplified by PCR and then purified. A DNA fragment (SEQ ID NO: 1), which contains a sequence prepared by ligating a T7 promoter sequence, the 5'-UTR sequence of a human β-globin, KOZAK sequence, SU gp46, the 3'-UTR sequence of human β-globin and PolyA sequence, sequentially in this order, was introduced into a plasmid. To Nuclease-free water (547.2 μL) in which the plasmid (8 ng) was dissolved, 10X Buffer for KOD-Plus-Ver. 2 (80 μL, Toyobo Co., Ltd., catalog # KOD-211), 2 mM dNTP mix (80 μL, Toyobo Co., Ltd., catalog # KOD-211), 25 mM $MgSO_4$ (48 μL, Toyobo Co., Ltd., catalog # KOD-211), a 50 μM sense primer (4.8 μL, SEQ ID NO: 2), a 10 μM antisense primer (24 μL, SEQ ID NO: 3) and KOD Plus polymerase (16 μL, Toyobo Co., Ltd., catalog # KOD-211) were added. The mixture was incubated at 98°C for one minute and subjected to 20 times of a cycle consisting of a reaction at 98°C for 5 seconds, a reaction at 55°C for 15 seconds and a reaction at 68°C for 90 seconds, and further incubated at 68°C for one minute to amplify DNA. After completion of the reaction, the template DNA (SEQ ID NO: 4) was purified by Wizard SV Gel and PCR Clean-Up System (Promega catalog # A9281).

(2) Preparation of SU gp46 mRNA-001 by *in-vitro* transcription

First, 365.1 μg/mL template DNA (4.38 μL) obtained in Example 1-(1), 100 mM CleanCap AG (8 μL, TriLink catalog # N-7113), 100 mM ATP (8 μL, Hongene catalog # R1331), 100 mM GTP (8 μL, Hongene catalog # R2331), 100 mM CTP (8 μL, Hongene catalog # R3331), 100 mM N1-methylpseudoUTP (8 μL, Hongene catalog # R5-027), Nuclease-free water (67.62 μL, Thermo Fisher catalog # AM9937), T7 Transcription 5X buffer (32 μL, Promega catalog # P140X), Enzyme mix and T7 RNA Polymerase (16 μL, Promega catalog # P137X) were mixed and incubated at 37°C for 4 hours. RQ1 RNase-Free DNase (8 μL, Promega catalog # M6101) was added in the mixture and the resultant mixture was incubated at 37°C for 15 min. An 8 M LiCl solution (80 μL, Sigma-Aldrich catalog # L7026) was mixed with

the mixture and allowed to stand still at -30°C, overnight. After the mixture was centrifuged (4°C, 5200 × g, 35 min.), the supernatant was discarded. To the residue, 70% ethanol was added. The mixture was centrifuged (4°C, 5200 × g, 10 min.) and then the supernatant was discarded. The residue was air-dried. The obtained residue was dissolved in Nuclease-free water (500 μL) and purified by RNeasy Midi kit (Qiagen catalog # 75144) in accordance with the manual attached to the kit. The obtained solution (750 μL), rApid Alkaline Phosphatase (Roche catalog # 04 898 141001) buffer (85 μL), and an enzyme (32 μL) were mixed and incubated at 37°C for 30 min. and then 75°C for 2 min. The obtained solution was purified by RNeasy Midi kit (Qiagen catalog # 75144) in accordance with the manual attached to the kit to obtain the desired mRNA. The same experimental operation was repeated twice in total. The mRNA solutions obtained were combined to obtain the desired mRNA. The mRNA thus obtained has the sequence of SEQ ID NO: 17.

[Example 2] Preparation of gp46-Fib mRNA-002

(1) Preparation of template DNA for *in-vitro* transcription (IVT) of gp46-Fib

To prepare template DNA for use in *in-vitro* transcription (IVT), gp46-Fib DNA was amplified by PCR and then purified. A DNA fragment (SEQ ID NO: 5), which contains a sequence prepared by ligating a T7 promoter sequence, the 5'-UTR sequence of human β-globin, KOZAK sequence, gp46-Fib, the 3'-UTR sequence of human β-globin and PolyA sequence, sequentially in this order, was introduced into a plasmid. To Nuclease-free water (547.2 μL) in which the plasmid (8 ng) was dissolved, 10X Buffer for KOD-Plus-Ver. 2 (80 μL, Toyobo Co., Ltd., catalog # KOD-211), 2 mM dNTP mix (80 μL, Toyobo Co., Ltd., catalog # KOD-211), 25 mM $MgSO_4$ (48 μL, Toyobo Co., Ltd., catalog # KOD-211), a 50 μM sense primer (4.8 μL, SEQ ID NO: 2), a 10 μM antisense primer (24 μL, SEQ ID NO: 3) and KOD Plus polymerase (16 μL, Toyobo Co., Ltd., catalog # KOD-211), were added. The mixture was incubated at 98°C for one minute, and then, subjected to 20 times of a cycle consisting of a reaction at 98°C for 5 seconds, a reaction at 55°C for 15 seconds and a reaction at 68°C for 90 seconds, and further incubated at 68°C for one minute to amplify DNA. After completion of the reaction, the template DNA (SEQ ID NO: 6) was purified by Wizard SV Gel and PCR Clean-Up System (Promega catalog # A9281).

(2) Preparation of gp46-Fib mRNA-002 by *in-vitro* transcription

First, 345.3 μg/mL template DNA (4.63 μL) obtained in Example 2-(1), 100 mM CleanCap AG (8 μL, TriLink catalog # N-7113), 100 mM ATP (8 μL, Hongene catalog # R1331), 100 mM GTP (8 μL, Hongene catalog # R2331), 100 mM CTP (8 μL, Hongene catalog # R3331), 100 mM N1-methylpseudoUTP (8 μL, Hongene catalog # R5-027), Nuclease-free water (67.37 μL, Thermo Fisher catalog # AM9937), T7 Transcription 5X buffer (32 μL, Promega catalog # P140X), Enzyme mix and T7 RNA Polymerase (16 μL, Promega catalog # P137X) were mixed and incubated at 37°C for 4 hours. RQ1 RNase-Free DNase (8 μL, Promega catalog # M6101) was added in the mixture and the resultant mixture was incubated at 37°C for 15 min. An 8 M LiCl solution (80 μL, Sigma-Aldrich catalog # L7026) was mixed with the mixture and allowed to stand still at -30°C, overnight. After the mixture was centrifuged (4°C, 5200 × g, 35 min.), the supernatant was discarded. To the residue, 70% ethanol was added. The mixture was centrifuged (4°C, 5200 × g, 10 min.), and then, the supernatant was discarded. The residue was air-dried. The obtained residue was dissolved in Nuclease-free water (500 μL), and then, purified by RNeasy Midi kit (Qiagen catalog # 75144) in accordance with the manual attached to the kit. The obtained solution (750 μL), rApid Alkaline Phosphatase (Roche catalog # 04 898 141 001) buffer (85 μL), and an enzyme (32 μL) were mixed and incubated at 37°C for 30 min. and then, 75°C for 2 min. The obtained solution was purified by RNeasy Midi kit (Qiagen catalog # 75144) in accordance with the manual attached to the kit to obtain the desired mRNA. The same experimental operation was repeated twice in total. The mRNA solutions obtained were combined to obtain the desired mRNA. The mRNA thus obtained has the sequence of SEQ ID NO: 18.

[Example 3] Preparation of dgp62-Fib mRNA-003

(1) Preparation of template DNA for *in-vitro* transcription (IVT) of dgp62-Fib

To prepare template DNA for use in *in-vitro* transcription (IVT), dgp62-Fib DNA was amplified by PCR and then purified. A DNA fragment (SEQ ID NO: 7), which contains a sequence prepared by ligating a T7 promoter sequence, the 5'-UTR sequence of human β-globin, KOZAK sequence, dgp62-Fib, the 3'-UTR sequence of human β-globin and PolyA sequence, sequentially in this order, was introduced into a plasmid. To Nuclease-free water (547.2 μL) in which the plasmid (8 ng) was dissolved, 10X Buffer for KOD-Plus-Ver. 2 (80 μL, Toyobo Co., Ltd., catalog # KOD-211), 2 mM dNTP mix (80 μL, Toyobo Co., Ltd., catalog # KOD-211), 25 mM $MgSO_4$ (48 μL, Toyobo Co., Ltd., catalog # KOD-211), a 50 μM sense primer (4.8 μL, SEQ ID NO: 2), a 10 μM antisense primer (24 μL, SEQ ID NO: 3), and KOD Plus polymerase (16 μL, Toyobo Co., Ltd., catalog # KOD-211), were added. The mixture was incubated at 98°C for one

minute and subjected to 20 times of a cycle consisting of a reaction at 98°C for 5 seconds, a reaction at 55°C for 15 seconds and a reaction at 68°C for 90 seconds and further incubated at 68°C for one minute to amplify DNA. After completion of the reaction, template DNA (SEQ ID NO: 8) was purified by Wizard SV Gel and PCR Clean-Up System (Promega catalog # A9281).

(2) Preparation of dgp62-Fib mRNA-003 by *in-vitro* transcription

First, 366.7 μg/mL template DNA (4.36 μL) obtained in Example 3-(1), 100 mM CleanCap AG (8 μL, TriLink catalog # N-7113), 100 mM ATP (8 μL, Hongene catalog # R1331), 100 mM GTP (8 μL, Hongene catalog # R2331), 100 mM CTP (8 μL, Hongene catalog # R3331), 100 mM N1-methylpseudoUTP (8 μL, Hongene catalog # R5-027), Nuclease-free water (67.64 μL, Thermo Fisher catalog # AM9937), T7 Transcription 5X buffer (32 μL, Promega catalog # P140X) and Enzyme mix and T7 RNA Polymerase (16 μL, Promega catalog # P137X) were mixed and incubated at 37°C for 4 hours. RQ1 RNase-Free DNase (8 μL, Promega catalog # M6101) was added in the mixture and the resultant mixture was incubated at 37°C for 15 min. An 8 M LiCl solution (80 μL, Sigma-Aldrich catalog # L7026) was mixed with the mixture and allowed to stand still at -30°C, overnight. After the mixture was centrifuged (4°C, 5200 × g, 35 min.), the supernatant was discarded. To the residue, 70% ethanol was added and the mixture was centrifuged (4°C, 5200 × g, 10 min.). The supernatant was discarded and the residue was air-dried. The obtained residue was dissolved in Nuclease-free water (500 μL) and purified by RNeasy Midi kit (Qiagen catalog # 75144) in accordance with the manual attached to the kit. The obtained solution (750 μL), rApid Alkaline Phosphatase (Roche catalog # 04 898 141 001) buffer (85 μL), and an enzyme (32 μL) were mixed and incubated at 37°C for 30 min and then 75 °C for 2 min. The obtained solution was purified by RNeasy Midi kit (Qiagen catalog # 75144) in accordance with the manual attached to the kit to obtain the desired mRNA. The same experimental operation was repeated twice in total. The mRNA solutions obtained were combined to obtain the desired mRNA. The mRNA thus obtained has the sequence of SEQ ID NO: 19.

[Example 4] Preparation of sec Tax mutant mRNA-004

(1) Preparation of template DNA for *in-vitro* transcription (IVT) of sec Tax mutant

To prepare template DNA for use in *in-vitro* transcription (IVT), sec Tax DNA was amplified by PCR and then purified. A DNA fragment (SEQ ID NO: 9), which contains a sequence prepared by ligating a T7 promoter sequence, the 5'-UTR sequence of human β-globin, KOZAK sequence, sec Tax, the 3'-UTR sequence of human β-globin and PolyA sequence, sequentially in this order, was introduced into a plasmid. To Nuclease-free water (547.2 μL) in which the plasmid (8 ng) was dissolved, 10X Buffer for KOD-Plus-Ver. 2 (80 μL, Toyobo Co., Ltd., catalog # KOD-211), 2 mM dNTP mix (80 μL, Toyobo Co., Ltd., catalog # KOD-211), 25 mM $MgSO_4$ (48 μL, Toyobo Co., Ltd., catalog # KOD-211), a 50 μM sense primer (4.8 μL, SEQ ID NO: 2), a 10 μM antisense primer (24 μL, SEQ ID NO: 3) and KOD Plus polymerase (16 μL, Toyobo Co., Ltd., catalog # KOD-211), were added. The mixture was incubated at 98°C for one minute and subjected to 20 times of a cycle consisting of a reaction at 98°C for 5 seconds, a reaction at 55°C for 15 seconds and a reaction at 68°C for 90 seconds, and further incubated at 68°C for one minute to amplify DNA. After completion of the reaction, template DNA (SEQ ID NO: 10) was purified by Wizard SV Gel and PCR Clean-Up System (Promega catalog # A9281).

(2) Preparation of sec Tax mutant mRNA-004 by *in-vitro* transcription

First, 401.1 μg/mL template DNA (3.99 μL) obtained in Example 4-(1), 100 mM CleanCap AG (8 μL, TriLink catalog # N-7113), 100 mM ATP (8 μL, Hongene catalog # R1331), 100 mM GTP (8 μL, Hongene catalog # R2331), 100 mM CTP (8 μL, Hongene catalog # R3331), 100 mM N1-methylpseudoUTP (8 μL, Hongene catalog # R5-027), Nuclease-free water (68.01 μL, Thermo Fisher catalog # AM9937), T7 Transcription 5X buffer (32 μL, Promega catalog # P140X), Enzyme mix and T7 RNA Polymerase (16 μL, Promega catalog # P137X) were mixed and incubated at 37°C for 4 hours. RQ1 RNase-Free DNase (8 μL, Promega catalog # M6101) was added in the mixture and the resultant mixture was incubated at 37°C for 15 min. An 8 M LiCl solution (80 μL, Sigma-Aldrich catalog # L7026) was mixed with the mixture and allowed to stand still at -30°C, overnight. After the mixture was centrifuged (4°C, 5200 × g, 35 min.), the supernatant was discarded. To the residue, 70% ethanol was added. The mixture was centrifuged (4°C, 5200 × g, 10 min.) and then the supernatant was discarded. The residue was air-dried. The obtained residue was dissolved in Nuclease-free water (500 μL) and purified by RNeasy Midi kit (Qiagen catalog # 75144) in accordance with the manual attached to the kit. The obtained solution (750 μL), rApid Alkaline Phosphatase (Roche catalog # 04 898 141001) buffer (85 μL), and an enzyme (32 μL) were mixed and incubated at 37°C for 30 min. and then 75°C for 2 min. The obtained solution was purified by RNeasy Midi kit (Qiagen catalog # 75144) in accordance with the manual attached to the kit to obtain the desired mRNA. The same experimental operation was repeated twice in total. The mRNA solutions obtained

were combined to obtain the desired mRNA. The mRNA thus obtained has the sequence of SEQ ID NO: 20.

[Example 5] Preparation of SU gp46 mRNA-005

(1) Preparation of template DNA for *in-vitro* transcription (IVT) of SU gp46

Preparation was carried out in the same manner as in Example 1-(1).

(2) Preparation of SU gp46 mRNA-005 by *in-vitro* transcription

First, 339 μg/mL template DNA (89 μL) obtained in Example 5-(1), 100 mM CleanCap AG (150 μL, TriLink catalog # T-7113), 100 mM ATP (150 μL, Hongene catalog # R1331), 100 mM GTP (150 μL, Hongene catalog # R2331), 100 mM CTP (150 μL, Hongene catalog # R3331), 100 mM N1-methyl-pseudouridine 5'-triphosphate (150 μL, Hongene catalog # R5-027), Nuclease-free water (1261 μL, Qiagen catalog # 129114), T7 Transcription 5X buffer (600 μL, Promega catalog # P140X), and Enzyme mix, and T7 RNA Polymerase (300 μL, Promega catalog # P137X) were mixed and incubated at 37°C for 2 hours. RQ1 RNase-Free DNase (30 μL, Promega catalog # M6101) was added in the mixture and the resultant mixture was incubated at 37°C for 15 min. An 8 M LiCl solution (1500 μL, Sigma-Aldrich catalog # L7026) was mixed with the mixture and allowed to stand still at -20°C, overnight. After the mixture was centrifuged (4°C, 5250 × g, 30 min.), the supernatant was discarded. To the residue, 70% ethanol was added and the mixture was centrifuged (4°C, 5250 × g, 10 min.). The supernatant was discarded and the residue was air-dried. The obtained residue was dissolved in Nuclease-free water and purified by RNeasy Maxi kit (Qiagen catalog # 75162) in accordance with the manual attached to the kit. The obtained eluate (12 mL, 12.0 mg in terms of UV), Nuclease-free water (120 μL), rApid Alkaline Phosphatase (Roche catalog # 04 898 141 001) buffer (1400 μL), and an enzyme (480 μL) were mixed and incubated at 37°C for 30 min. After completion of incubation at 75°C for 2 min., an 8M LiCl solution (7000 μL, Sigma-Aldrich catalog # L7026) was added to the mixture. The mixture was allowed to stand still at -20°C, overnight. After the mixture was centrifuged (4°C, 5250 × g, 30 min.), the supernatant was discarded. To the residue, 70% ethanol was added. The mixture was centrifuged (4°C, 5250 × g, 10 min.) and then the supernatant was discarded. The residue was air-dried. The obtained residue was dissolved in Nuclease-free water (2 mL, 9.9 mg in terms of UV). The mRNA obtained was separated/purified by reversed-phase chromatography (Column: PLRP-S, 4000Å, 10 μm, 10 × 200 mm (Agilent), Buffer A: 5% acetonitrile, 400 mM triethylamine acetate (pH7.0), Buffer B: 25% acetonitrile, 400 mM triethylamine acetate (pH7.0), Gradient B%: 27.5% to 35% (20 min.), Flow rate: 5 mL/min, Temperature: 80°C). The desired fractions were collected and desalted by ultrafiltration (Amicon Ultra-15 (MWCO: 30 kDa))(3.4 mg in terms of UV).

The mRNA obtained has the sequence of SEQ ID NO: 17. The mRNA was analyzed by LabChip GX Touch Standard RNA Reagent Kit (PerkinElmer catalog #CLS960010) and confirmed to have the desired length.

[Example 6] Preparation of sec Tax mutant mRNA-006

(1) Preparation of template DNA for *in-vitro* transcription (IVT) of sec Tax mutant

Preparation was carried out in the same manner as in Example 4-(1).

(2) Preparation of sec Tax mutant mRNA-006 by *in-vitro* transcription

First, 336 μg/mL template DNA (89 μL) obtained in Example 6-(1), 100 mM CleanCap AG (150 μL, TriLink catalog # T-7113), 100 mM ATP (150 μL, Hongene catalog # R1331), 100 mM GTP (150 μL, Hongene catalog # R2331), 100 mM CTP (150 μL, Hongene catalog # R3331), 100 mM N1-methyl-pseudouridine 5'-triphosphate (150 μL, Hongene catalog # R5-027), Nuclease-free water (1261 μL, Qiagen catalog # 129114), T7 Transcription 5X buffer (600 μL, Promega catalog # P140X), and Enzyme mix, and T7 RNA Polymerase (300 μL, Promega catalog # P137X) were mixed and incubated at 37°C for 2 hours. RQ1 RNase-Free DNase (30 μL, Promega catalog # M6101) was added in the mixture and the resultant mixture was incubated at 37°C for 15 min. An 8 M LiCl solution (1500 μL, Sigma-Aldrich catalog # L7026) was mixed with the mixture and allowed to stand still at -20°C, overnight. After the mixture was centrifuged (4°C, 5250 × g, 30 min.), the supernatant was discarded. To the residue, 70% ethanol was added and the mixture was centrifuged (4°C, 5250 × g, 10 min.). The supernatant was discarded and the residue was air-dried. The obtained residue was dissolved in Nuclease-free water, and then, purified by RNeasy Maxi kit (Qiagen catalog # 75162) in accordance with the manual attached to the kit. The obtained eluate (12 mL, 12.6 mg in terms of UV), Nuclease-free water (100 μL), rApid Alkaline Phosphatase (Roche catalog # 04 898 141 001) buffer (1400 μL) and an enzyme (500 μL) were mixed and incubated at 37°C for 30 min. After completion of incubation at 75°C for 2 min., an 8M LiCl solution (7000 μL, Sigma-Aldrich catalog # L7026) was added to the mixture. The mixture was allowed to stand still at -20°C overnight. After the

mixture was centrifuged (4°C, 5250 × g, 30 min.), the supernatant was discarded. To the residue, 70% ethanol was added. The mixture was centrifuged (4°C, 5250 × g, 10 min.) and then the supernatant was discarded. The residue was air-dried. The obtained residue was dissolved in Nuclease-free water (2 mL, 10.9 mg in terms of UV). The mRNA obtained was separated/purified by reversed-phase chromatography (Column: PLRP-S, 4000Å, 10 μm, 10 × 200 mm (Agilent), Buffer A: 5% acetonitrile, 400 mM triethylamine acetate (pH7.0), Buffer B: 25% acetonitrile, 400 mM triethylamine acetate (pH7.0), Gradient B%: 27.5% to 35% (20 min.), Flow rate: 5 mL/min, Temperature: 80°C). The desired fractions were collected and desalted by ultrafiltration (Amicon Ultra-15 (MWCO: 30 kDa))(3.7 mg in terms of UV).

The mRNA obtained has the sequence of SEQ ID NO: 20. The mRNA was analyzed by LabChip GX Touch Standard RNA Reagent Kit (PerkinElmer catalog #CLS960010) and confirmed to have the desired length.

[Example 7] Preparation of gp46-Fib mRNA-007

(1) Preparation of template DNA for *in-vitro* transcription (IVT) of gp46-Fib

Preparation was carried out in the same manner as in Example 2-(1).

(2) Preparation of gp46-Fib mRNA-007 by *in-vitro* transcription

First, 397 μg/mL template DNA (63 μL) obtained in Example 7-(1), 100 mM CleanCap AG (50 μL, TriLink catalog # T-7113), 100 mM ATP (50 μL, Hongene catalog # R1331), 100 mM GTP (50 μL, Hongene catalog # R2331), 100 mM CTP (50 μL, Hongene catalog # R3331), 100 mM N1-methyl-pseudouridine 5'-triphosphate (50 μL, Hongene catalog # R5-027), Nuclease-free water (387 μL, Qiagen catalog # 129114), T7 Transcription 5X buffer (200 μL, Promega catalog # P140X), and Enzyme mix and T7 RNA Polymerase (100 μL, Promega catalog # P137X) were mixed and incubated at 37°C for 4 hours. RQ1 RNase-Free DNase (25 μL, Promega catalog # M6101) was added in the mixture and the resultant mixture was incubated at 37°C for 15 min. An 8 M LiCl solution (500 μL, Sigma-Aldrich catalog # L7026) was mixed with the mixture and allowed to stand still at -20°C, overnight. After the mixture was centrifuged (4°C, 5250 × g, 30 min.), the supernatant was discarded. To the residue (pellet), 70% ethanol was added and the mixture was centrifuged (4°C, 5250 × g, 30 min.). The supernatant was discarded and the residue (pellet) was air-dried. The obtained residue was dissolved in Nuclease-free water, and thereafter, subjected to purification process performed by the RNeasy Maxi kit (Qiagen catalog # 75162) in accordance with the manual attached to the kit. The obtained eluate (3 mL, 3.6 mg in terms of UV), Nuclease-free water (332 μL), rApid Alkaline Phosphatase (Roche catalog # 04 898 141 001) buffer (450 μL), and an enzyme (718 μL) were mixed and incubated at 37°C for 30 min. The mixture was incubated at 75°C for 2 min., and thereafter, subjected to a purification process performed by RNeasy Maxi kit in accordance with the manual attached to the kit to obtain the desired mRNA (4 mL, 2.8 mg in terms of UV).

The mRNA obtained has the sequence of SEQ ID NO: 18. The mRNA was analyzed by LabChip GX Touch Standard RNA Reagent Kit (PerkinElmer catalog #CLS960010) and confirmed to have the desired length.

[Example 8] Preparation of sec Tax mutant mRNA-008

(1) Preparation of template DNA for *in-vitro* transcription (IVT) of sec Tax mutant

Preparation was carried out in the same manner as in Example 4-(1).

(2) Preparation of sec Tax mutant mRNA-008 by *in-vitro* transcription

First, 391 μg/mL template DNA (64 μL) obtained in Example 8-(1), 100 mM CleanCap AG (50 μL, TriLink catalog # T-7113), 100 mM ATP (50 μL, Hongene catalog # R1331), 100 mM GTP (50 μL, Hongene catalog # R2331), 100 mM CTP (50 μL, Hongene catalog # R3331), 100 mM N1-methyl-pseudouridine 5'-triphosphate (50 μL, Hongene catalog # R5-027), Nuclease-free water (386 μL, Qiagen catalog # 129114), T7 Transcription 5X buffer (200 μL, Promega catalog # P140X), Enzyme mix, and T7 RNA Polymerase (100μL Promega catalog # P137X) were mixed and incubated at 37°C for 4 hours. RQ1 RNase-Free DNase (25 μL, Promega catalog # M6101) was added in the mixture and the resultant mixture was incubated at 37°C for 15 min. An 8 M LiCl solution (500 μL, Sigma-Aldrich catalog # L7026) was mixed with the mixture and allowed to stand still at -20°C, overnight. After the mixture was centrifuged (4°C, 5250 × g, 30 min.), the supernatant was discarded. To the residue, 70% ethanol was added and the mixture was centrifuged (4°C, 5250 × g, 10 min.). The supernatant was discarded and the residue was air-dried. The obtained residue was dissolved in Nuclease-free water, and thereafter, subjected to a purification process performed by the RNeasy Maxi kit (Qiagen catalog # 75162) in accordance with the manual attached to the kit. The obtained eluate (3 mL, 3.5 mg in terms of UV), Nuclease-free water (359 μL), rApid Alkaline Phosphatase (Roche catalog # 04 898 141 001) buffer (450 μL) and an

enzyme (691 μL) were mixed and incubated at 37°C for 30 min. The mixture was incubated at 75°C for 2 min., and thereafter, subjected to a purification process performed by RNeasy Maxi kit in accordance with the manual attached to the kit to obtain the desired mRNA (4 mL, 2.8 mg in terms of UV).

The mRNA obtained has the sequence of SEQ ID NO: 20. The mRNA was analyzed by LabChip GX Touch Standard RNA Reagent Kit (PerkinElmer catalog #CLS960010) and confirmed to have the desired length.

[Examples 9 to 24] Preparation of nucleic-acid lipid particles encapsulating mRNA molecules obtained in Examples 1 to 8

(1) Preparation of a nucleic-acid lipid particle encapsulating mRNA

Distearoylphosphatidylcholine (1,2-distearoyl-sn-glycero-3-phosphocholine: hereinafter referred to as "DSPC", NOF CORPORATION), cholesterol (hereinafter referred to as "Chol", Sigma-Aldrich, Inc.), (7R,9Z,26Z,29R)-18-({[3-(dimethylamino)propoxy]carbonyl}oxy)pentatriaconta-9,26-diene-7,29-diyl diacetate (compound disclosed in Example 23 of WO2015/005253) (hereinafter referred to as "LP1") or (7R,9Z)-18-({[3-(dimethylamino)propyloxy]carbonyl}oxy)octacosa-9-en-7-yl acetate (compound disclosed in Example 28 of WO2015/005253) (hereinafter referred to as "LP2"), and 1,2-dimyristoyl-sn-glycerol methoxypolyethylene glycol (hereinafter referred to as "PEG-DMG", NOF CORPORATION, SUNBRIGHT GM-020, a molecular weight of polyethylene glycol: about 2000) were dissolved in ethanol in accordance with the molar ratios listed in Table 1 and so as to have a total lipid concentration of 5 mM.

On the other hand, the mRNA molecules obtained in Examples 1 to 8 were diluted with a citrate buffer (20 mM Citrate Buffer, pH4.0) to prepare dilution solutions.

The lipid solution prepared above and each of the mRNA solutions were mixed in microchannels so as to have the total lipid weight ratio to the mRNA listed in Table 1 and the volume ratio thereof of 1: 3 by use of NanoAssemblr BenchTop (Precision Nanosystems Inc.) to obtain coarse dispersions of nucleic-acid lipid particles. The dispersions of the nucleic-acid lipid particles were dialyzed against about 25 to 50X buffer for 12 to 18 hours (Float-A-Lyzer G2, MWCO: 1,000 kD, Spectra/Por) to remove ethanol. In this manner, purified dispersions of nucleic-acid lipid particles encapsulating mRNA were obtained.

Note that, LP1 was synthesized in accordance with the method disclosed in Example 23 of WO2015/005253; whereas, LP2 in accordance with the method disclosed in Example 28 of WO2015/005253.

(2) Property evaluation of nucleic-acid lipid particle encapsulating mRNA

The dispersions containing the nucleic-acid lipid particles prepared in the above step (1) were subjected to property evaluation. Methods for evaluating individual properties will be described below.

(2-1) Encapsulation rate of mRNA

The encapsulation rate of mRNA was determined by Quant-iT RiboGreen RNA Assay kit (Invitrogen) in accordance with the package insert.

More specifically, mRNA in each of the dispersions of nucleic-acid lipid particles was measured in the presence and absence of 0.015% Triton X-100 surfactant. The encapsulation rate was calculated in accordance with the following formula:

$$\{([\text{mRNA amount in the presence of surfactant}] - [\text{mRNA amount in the absence of surfactant})/[\text{mRNA amount in the presence of surfactant}\} \times 100\ (\%)$$

(2-2) The ratio of mRNA and lipid

The amounts of mRNA in the dispersions of nucleic-acid lipid particles were measured in any one of the following methods.

A nucleic-acid lipid particle dispersion was diluted with 1.0% Triton X-100 and subjected to measurement by reversed-phase chromatography (System: Agilent 1260 series, Column: Bioshell A400 Protein C4 (10 cm × 4.6 mm, 3.4 μm) (SUPELCO), Buffer A: 0.1 M triethylamine acetate (pH7.0), Buffer B: acetonitrile, (B%): 5-50% (0-15 min.), Flow Rate: 1 mL/min, Temperature: 70°C, Detection: 260 nm).

The nucleic-acid lipid particle dispersion was diluted/dissolved with 90% methanol and subjected to measurement of mRNA amount in nucleic-acid lipid particles by a UV-visible spectrophotometer (manufactured by PerkinElmer Co., Ltd., LAMBDA (trademark) 465). The concentration of mRNA was calculated in accordance with the following formula:

$$\{[\text{Absorbance at 260 nm}]-[\text{Absorbance at 350 nm}]\} \times 40 \times \text{dilution ratio } (\mu g/mL)$$

The amount of lipid contained in each of the nucleic-acid lipid particle dispersions was determined by reversed-phase chromatography (System: DIONEX UltiMate 3000, Column: XSelect CSH C18 (130Å, 3.5 μm, 3.0 mm × 150 mm) (Waters catalog # 186005263), Buffer A: 0.2% formic acid, Buffer B: 0.2% formic acid, methanol, (B%): 75-100% (0-6 min.), 100% (6-15 min.), Flow Rate: 0.45 mL/min, Temperature: 50°C, Detection: Corona CAD (Charged Aerosol Detector)).

The weight ratio of the total lipid to mRNA was calculated in accordance with the following formula:

$$[\text{Total lipid concentration}]/[\text{mRNA concentration}] \text{ (wt/wt)}$$

(2-3) Average particle size

The particle sizes of the nucleic-acid lipid particles were measured by Zeta Potential/Particle Sizer NICOMPTM 380ZLS (PARTICLE SIZING SYSTEMS). The average particle size in Tables represents a volume average particle size. A deviation is represented by "± or less"

The property evaluations are listed in Table 2.

[Table 1]

| Example No. | mRNA | DSPC | Chol | LP | PEG-DMG | Lipids/mRNA (wt/wt) |
|---|---|---|---|---|---|---|
| Example 9 | Example 1 | 12.5% | 41% | 45% (LP1) | 1.5% | 20 |
| Example 10 | Example 2 | 12.5% | 41% | 45% (LP1) | 1.5% | 20 |
| Example 11 | Example 3 | 12.5% | 41% | 45% (LP1) | 1.5% | 20 |
| Example 12 | Example 4 | 12.5% | 41% | 45% (LP1) | 1.5% | 20 |
| Example 13 | Example 5 | 12.5% | 41% | 45% (LP1) | 1.5% | 20 |
| Example 14 | Example 6 | 12.5% | 41% | 45% (LP1) | 1.5% | 20 |
| Example 15 | Example 7 | 17.5% | 26% | 55% (LP2) | 1.5% | 20 |
| Example 16 | Example 8 | 17.5% | 26% | 55%(LP2) | 1.5% | 20 |
| Example 17 | Example 7 | 22.5% | 21% | 55% (LP2) | 1.5% | 20 |
| Example 18 | Example 8 | 22.5% | 21% | 55% (LP2) | 1.5% | 20 |
| Example 19 | Example 7 | 17.5% | 21% | 60% (LP2) | 1.5% | 20 |
| Example 20 | Example 8 | 17.5% | 21% | 60% (LP2) | 1.5% | 20 |
| Example 21 | Example 7 | 22.5% | 16% | 60% (LP2) | 1.5% | 20 |
| Example 22 | Example 8 | 22.5% | 16% | 60% (LP2) | 1.5% | 20 |
| Example 23 | Example 7 | 12.5% | 41% | 45% (LP1) | 1.5% | 20 |
| Example 24 | Example 8 | 12.5% | 41% | 45% (LP1) | 1.5% | 20 |

[Table 2]

| Example No. | Encapsulation rate (%) | Particle size (nm) |
|---|---|---|
| Example 9 | 97% | 126±39 |
| Example 10 | 95% | 138±39 |
| Example 11 | 96% | 133±49 |
| Example 12 | 96% | 124±35 |
| Example 13 | 97% | 111±37 |

(continued)

| Example No. | Encapsulation rate (%) | Particle size (nm) |
|---|---|---|
| Example 14 | 98% | $108 \pm 24$ |
| Example 15 | 99% | $135 \pm 41$ |
| Example 16 | 99% | $133 \pm 18$ |
| Example 17 | 97% | $138 \pm 17$ |
| Example 18 | 97% | $133 \pm 35$ |
| Example 19 | 99% | $123 \pm 48$ |
| Example 20 | 98% | $123 \pm 20$ |
| Example 21 | 96% | $127 \pm 42$ |
| Example 22 | 96% | $129 \pm 45$ |
| Example 23 | 99% | $133 \pm 49$ |
| Example 24 | 99% | $137 \pm 33$ |

From the above results, it was found that 95% or more of the mRNA are encapsulated in the nucleic-acid lipid particles, and that the nucleic-acid lipid particles have an average particle size of about 100 nm to about 140 nm.

(Experimental Example 1)

Construction of plasmid for LTR reporter assay

DNA fragments encoding a HTLV-1 Tax wild type (Uniprot Accession Number: P03409), a Tax mutant, and a secretory Tax mutant, which was prepared by adding an IgE secretory signal to the Tax mutant, were synthesized by an outsourcing company, Eurofin. Each of the DNA fragments was cloned by inserting it into a NheI/NotI site of pcDNA3.1+ vector (Thermo Scientific Inc.). DNA fragments encoding HIV-1 LTR and HTLV-1 LTR were synthesized by an outsourcing company, Eurofin. Each of the DNA fragments was cloned by inserting it into a KpnI/XhoI site of pGL4.17 vector (Promega).

HIV-1 LTR and HTLV-1 LTR reporter assay

A plasmid (350 ng) expressing the Tax wild type, Tax mutant, or secretory Tax mutant, an HIV-1 LTR reporter plasmid or a HTLV-1 LTR reporter plasmid (100 ng), and 50 ng of pRG-RK plasmid (Promega) for use in the correction of transfection efficiency were prepared. HEK293T cells were transfected with the plasmids (500 ng in total) by use of transIT-LT1 gene introduction reagent (Takara). Fouty-eight hours after completion of transfection, the expression level of firefly luciferase and the expression level of Renilla luciferase were measured by Dual-Glo (registered trademark) Luciferase Assay System (Promega). The ratio of firefly luciferase expression level/Renilla luciferase expression level was calculated to evaluate the transcriptional activities of HIV-1 LTR and HTLV-1 LTR.

(Experimental Example 2)

Administration of lipid particles (Example 12) to C3H mice

C3H/HeJJcl mice were obtained from CLEA Japan, Inc and acclimatized. To sterilized musculus triceps femoris of mice under anesthesia, lipid particles (Example 12) were administered at a dose of 5 $\mu$g mRNA/20 $\mu$L/mouse at intervals of 2 weeks. The first administration was made to the right leg and the second administration to the left leg. The concentration of lipid particles was controlled with a buffer and the buffer was administered to a negative control group.

Preparation of the serum and splenocytes

Two weeks after the first administration of the lipid particles and one week after the second administration, a blood sample was taken from the mice under anesthesia. The blood samples were each allowed to stand still at normal

temperature for one hour or more and centrifuged at 3000 RPM for 10 min and collected. The spleen was excised from each of the dead mice by exsanguination under anesthesia. The spleen was ground by the mesh of Cell Strainer (FALCON) and the gasket of a syringe (TERUMO) and added in RPMI 1640 (Nacalai Tesque) to prepare a single-cell suspension. The cells were centrifugally collected. The supernatant was discarded and hemolysis (red blood cell lysis) was carried out with DB Pharm Lyse Lysing buffer (BECTON DICKINSON). The red blood cells lysed were centrifuged and resuspended with RPMI 1640. The cell suspension was passed through mini Cell Strainers (HIGH-TECH CORPORATION, Cat. HT-AMS-14002). After centrifugation, the supernatant was discarded. The cells were resuspended and the density of the cells was measured. After centrifugation, the supernatant was discarded. The density of the cells was adjusted with a cell culture solution (RPMI 1640 containing 10% Fetal Bovine Serum (inactivated and passed through a filter, HyClone), 1% Penicillin-Streptomycin Mixed Solution (Nacalai Tesque), 1% Sodium Pyruvate (gibco), 1% MEM Non-Essential Amino Acids (gibco), 1% HEPES Buffer Solution (gibco) and 1% StemSure Monothioglycerol Solution (FUJIFILM)). Note that, the centrifugation was carried out at 1500 RPM at 4°C for 5 min.

Level of Tax-specific CTL induction

The mouse splenocytes ($3 \times 10^6$ cells) prepared were centrifuged and the supernatant was discarded. A step of resuspending the cells in 1X Dulbecco's Phosphate Buffered Saline (gibco), centrifuging the resuspension and discarding the supernatant was repeated twice. Thereafter, TruStain FcX Antibody (BioLegend) diluted 20 fold with 1X PBS was added to the cells and the mixture was allowed to stand still at normal temperature for 5 min. Five μL of $H\text{-}2D^K$ HTLV-1 Tax38-46 Tetramer-ARLHTHALL-PE (MBL) was added to each sample. The samples were incubated at 37°C for 15 min in a 5% $CO_2$. APC/Fire (trademark) 750 anti-mouse CD3 Antibody (BioLegend) and Anti-CD8 (Mouse) mAb-FITC (MBL), which were diluted 100 fold on ice, were added to the samples, which were allowed to stand still in a dark place for 30 min. The samples were centrifuged and the supernatant was discarded. The cells were washed twice with 1X PBS. Finally, the cells were resuspended with 400 μL of PBS. The cells labeled with FACSVerse (BD) were detected and analyzed by FlowJo (confirmation). Note that, the centrifugation was carried out at 1500 RPM at 4°C for 5 min.

Anti-Tax antibody titer in mouse blood

For using Tax (MYBiosource) recombinant protein as an immobilized antigen, it was added in the wells of a 96-well flat bottom plate and allowed to stand still at 4°C, overnight. A dilution series for a standard curve was prepared by 3-fold serial dilution of Mouse IgG (SouthernBiotech) from the highest concentration of 0.25 μg/mL to obtain 7-stepwise dilution solutions. Individual wells were washed and a blocking solution was added to the wells and allowed to stand still at normal temperature for one hour. The serum samples were prepared with a blocking solution by 4-fold serial dilution from the highest concentration of a 100-fold dilution sample to obtain 7-stepwise dilution solutions. The plate treated with the blocking solution was washed, and a diluted sample was added in the wells of the plate and allowed to stand still at normal temperature for one hour. A detection antibody, Goat Anti-Mouse IgG, and Human ads-HRP (SouthernBiotech) were diluted 3000 fold with the blocking solution and added in the washed wells. One hour later, the plate was washed and then TMB Microwell Peroxidase Substrate System (seracare) was added in the wells. The plate was allowed to stand still for 2 to 3 minutes. The reaction was terminated with TMB Stop Solution (KPL, Cat. 51500-0021). Absorbance was measured at 540 nm and 450 nm by a plate reader. An anti-gp46 antibody titer and anti-Tax antibody titer were calculated by using the corrected absorbance, which was obtained by subtracting the absorbance at 540 nm from the absorbance at 450 nm, in analysis. Note that, as the blocking solution, 1X Dulbecco's Phosphate Buffered Saline (gibco) containing 1% Bovine Serum Albumin (Sigma) and 0.05% Tween (BIO-RAD) was used. Washing was carried out three times with 1X Dulbecco's Phosphate Buffered Saline ((10X) gibco) containing 0.05% Tween.

(Experimental Examples 3 to 5)

Administration to cynomolgus monkey

To the upper arms of monkeys, the lipid particles of Example 13 and Example 14 were administered once per two weeks, in total 4 times. The first administration was made to the right upper arm and the following administrations were made alternately to the left and right arms. The lipid particles of Example 13 and Example 14 each contained an equal amount of 25 μg mRNA and the lipid particles were administered at a dose of 50 μg mRNA/200 μL/body per time.

(Experimental Example 3)

Anti gp46 and Tax antibody titer in monkey blood

A recombinant gp46 protein (RayBiotech) and a recombination Tax protein (MY Biosource) were added in the wells of 96-well plates and allowed to stand still at 4°C overnight to immobilize them. Thereafter, the plates were washed by a plate washer (three times with a PBS wash solution containing 0.05% Tween 20) and treated with a blocking solution (PBS containing 1% BSA and 0.05% Tween 20) to perform blocking. The monkey plasma sample dilution series was prepared with a blocking solution by 4-fold serial dilution from the highest concentration of a 100-fold dilution sample to obtain 7-stepwise dilution solutions. A dilution series for a standard curve for monkey IgG concentration was prepared by 3-fold serial dilution of a Monkey IgG solution (provided by a manufacturer) from the highest concentration of 0.25 μg/mL with the blocking solution to obtain 8-stepwise dilution solutions. The sample dilution solution and the dilution solution for the standard curve were added and allowed to stand still at room temperature for one hour. Washing was carried out by a plate washer. As a detection antibody, HRP-labeled anti-monkey IgG antibody (Sigma-Aldrich) was diluted 4000 fold with the blocking solution, added in the wells of the plates and allowed to stand still at room temperature for one hour. After washing was carried out by a plate washer, TMB Microwell Peroxidase Substrate System (SERACARE Life Sciences) was added and allowed to stand still for 10 min. As the reaction termination solution, TMB Stop Solution (SERACARE Life Sciences) was used. Absorbance was measured at a wavelength of 450 nm and a reference wavelength of 540 nm by a plate reader. The corrected absorbance (Delta), which was obtained by subtracting the absorbance at 540 nm from the absorbance at 450 nm, was used in analysis. A calibration curve was prepared based on the monkey IgG concentration in the standard curve and Delta, by use of Nonlinear Regression: 4 Parameters. Based on the calibration curve, the dilution rates of measurement samples and Delta, the anti-gp46 and Tax antibody concentration of the samples were calculated.

(Experimental Example 4)

Cell-mediated immune response specific to gp46 and Tax in monkeys

PBMCs, which were isolated from monkey peripheral blood with Ficoll (Cytiva), were suspended in RPMI Complete culture medium (10% FBS [Sigma-Aldrich], 1% PS [Penicilin-Streptomycin Mixed Solution, Nacalai Tesque], 1 mM Sodium Pyruvate [Thermo Fisher Scientific], 10 mM HEPES [Thermo Fisher Scientific], 1X StemSure[manufactured by FUJIFILM Wako Pure Chemical Corporation], and 1X MEM Non-Essential Amino Acids Solution [Thermo Fisher Scientific]) so as to have a density of $2 \times 10^6$ cells/mL, and seeded in the wells of IFN-γ ELISpot plate (MABTech) at a rate of 100 μL/well. Thereafter, Tax epitope peptide pool (Eurofins), which was prepared with RPMI Complete culture medium to have a final concentration of 0.1% (v/v) or gp46 recombinant protein (RayBiotech), which was prepared to have a final concentration of 1 μg/mL, was added at a rate of 100 μL/well and cultured at 37°C in a 5% $CO_2$ condition for 48 hours. Antigen-specific IFN-γ-producing cells were detected by Monkey IFN-y ELISpot$^{PLUS}$ kit (HRP) (MABTech). The number of the antigen-specific IFN-y producing cells was counted by ELISPOT analyzer (CTL).

(Experimental Example 5)

Anti-HTLV-1 neutralization activity in monkey blood

Using Spin column based Antibody Purification Kit (cosmo bio), total IgG was purified from the monkey plasma collected/prepared two weeks after the 4th administration. To YT#1 cell line of HTLV-1 infected cells, purified IgG was added, and then, HTLV-1 uninfected cell line, CEM cells, were mixed at a predetermined ratio of the CEM cells to the YT#1 cells of 1: 1. The serum of a patient was added to the co-culture system and cultured overnight. Thereafter, the rate of syncytia formed was measured by microscopic visualization.

(Experimental Example 6)

Total IgG antibody titer specific to gp46 in mouse blood

For using a gp46 (Ray Biotech Inc.) recombinant protein as an immobilized antigen, the protein was added in the wells of a 96-well flat bottom plate and allowed to stand still at 4°C, overnight. A dilution series for a standard curve was prepared by 3-fold serial dilution of Mouse IgG (SouthernBiotech) from the highest concentration of 0.25 μg/mL to obtain 7-stepwise dilution solutions. Individual wells were washed and a blocking solution was added to the wells and allowed to stand still at normal temperature for one hour. The serum samples were prepared with a blocking solution by

4-fold serial dilution from the highest concentration of a 100-fold dilution sample to obtain 7-stepwise dilution solutions. The plate treated with the blocking solution was washed, and a diluted sample was added in the wells of the plate and allowed to stand still at normal temperature for one hour. Detection antibody, Goat AntiMouse IgG, and Human ads-HRP (SouthernBiotech) were diluted 3000 fold with the blocking solution and added in the washed wells. One hour later, the wells were washed, and then, TMB Microwell Peroxidase Substrate System (seracare) was added in the wells and allowed to stand still for 2 to 3 minutes. The reaction was terminated with TMB Stop Solution (KPL, Cat. 51500-0021). Absorbance was measured at 540 nm and 450 nm by a plate reader. An anti-gp46 antibody titer and anti-Tax antibody titer were calculated by using the corrected absorbance, which was obtained by subtracting the absorbance at 540 nm from the absorbance at 450 nm, in analysis. Note that, the blocking solution was 1X Dulbecco's Phosphate Buffered Saline (gibco) containing 1% Bovine Serum Albumin (Sigma) and 0.05% Tween (BIO-RAD). Washing was carried out three times with 1X Dulbecco's Phosphate Buffered Saline ((10X) gibco) containing 0.05% Tween.

(Experimental Example 7)

Cell-mediated immune response specific to gp46 and Tax in mice

The mouse splenocytes prepared were seeded in the wells of a 96-well U-bottom plate (FALCON) at a density of $10^6$ cells/well. Then, the mouse splenocytes were stimulated with gp46 and Tax pooled peptides (Eurofins) controlled so as to have a final concentration of 10 μg/mL, at 37°C in a 5% $CO_2$ (atmosphere). Twenty-four hours later, the culture supernatant was recovered, and the production amounts of IFN-y (R&D Systems) and IL-2 (R&D Systems) were measured in accordance with the protocol of the kits.

(Experimental Example 8)

Analysis for protein expression

The lipid particles of Examples 9, 10, 11 and 12 each were added to CHO-S cells (Thermo Fisher Scientific) such that the concentration of mRNA in the culture medium became 3 μg/mL. Seventy-two hours after the addition, the culture supernatant and a cell pellet were recovered. To the cell pellet, M-PER (trademark) Tissue Protein Extraction Reagent (Thermo Scientific) was added. The mixture was sufficiently stirred by a vortex and allowed to stand still at normal temperature for 10 min. The mixture was again stirred by a vortex and centrifuged (3000 RPM, 4°C, 10 min.). Thereafter, a cell lysate was collected. The gp46 protein in the culture supernatant and in the cell lysate was detected by Western blotting.

(Results of Experimental Example 1)

Screening of Tax antigen design based on transcriptional activities of HIV-1 LTR and HTLV-1 LTR as indexes

HIV-1 LTR transcriptional activity and HTLV-1 LTR transcriptional activity of the Tax wild type, Tax mutant, and secretory Tax mutant were evaluated (Figure 1). As a result, it was confirmed that the HIV-1 LTR transcriptional activity and HTLV-1 LTR transcriptional activity of the Tax mutant is lower than those of the Tax wild type. The HIV-1 LTR transcriptional activity and HTLV-1 LTR transcriptional activity of the secretory Tax mutant were equivalent to those of the negative control. From the results, it was suggested that the secretory Tax mutant exhibiting the lowest HIV-1 LTR transcriptional activity and HTLV-1 LTR transcriptional activity is a vaccine-antigen candidate having the lowest carcinogenicity.

(Results of Experimental Example 2)

CTL induction level and anti-Tax antibody titer in the blood by the lipid particle of Example 12

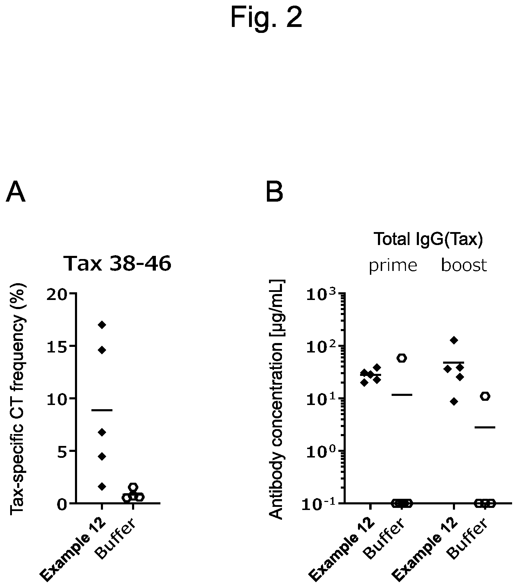

The CTL induction level in C3H mice administered with the lipid particle of Example 12 was evaluated (Figure 2). As a result, Tax-specific CTL and anti-Tax antibody response in the blood were induced by the lipid particle of Example 12.

(Results of Experimental Example 3)

Anti gp46 and Tax antibody titer in monkey blood

The anti-gp46 antibody response and anti-Tax antibody response in the blood induced by a lipid particle preparation, which was prepared by adding lipid particles of Example 13 and Example 14 in the same amounts, were evaluated (Figure 3). As a result, the anti-gp46 antibody titer and anti-Tax antibody titer in the blood were high after the 2nd administration (4W) and the 3rd administration (6W) compared to before administration (0W).

(Results of Experimental Example 4)

The level of IFN-γ-producing cells specific to gp46 and Tax in monkey PBMCs

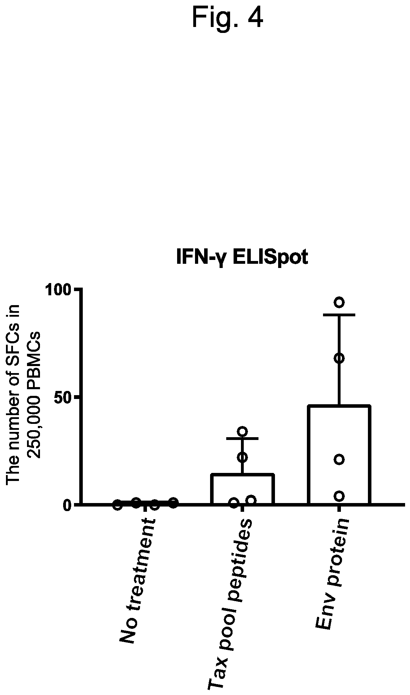

The anti-gp46 antibody response and anti-Tax antibody response in the blood induced by the mixed preparation of the lipid particles of Example 13 and Example 14 were evaluated (Figure 4). As the results of an examination carried out by use of PBMCs, which were collected/prepared from the blood taken 7 weeks after the 4th administration, it was confirmed that gp46-specific and Tax-specific IFN-γ-producing cells were induced in a group administered with the mixed preparation of the lipid particles of Example 5 and Example 6.

(Results of Experimental Example 5)

Anti HTLV-1 neutralization activity in monkey blood

The anti-HTLV-1 neutralization activity in the blood induced by the mixed preparation of the lipid particles of Example 13 and Example 14 was evaluated (Figure 5). As the results of an examination carried out by use of total IgG in the blood, which was prepared from the blood collected and purified 2 weeks after the 4th administration, the anti-HTLV-1 neutralization activity in the blood was confirmed in 3 of 4 monkeys in a lipid particle administration group, compared to the negative control group.

(Results of Experimental Example 6)

Anti gp46 antibody titer in mouse blood

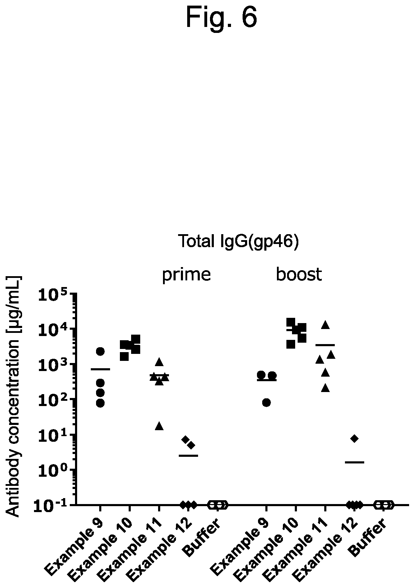

Anti-gp46 antibody response in the blood induced by administration of each of the lipid particles of Example 9, Example 10, Example 11 and Example 12 was evaluated (Figure 6). As a result, a high anti-gp46 antibody response in the blood was confirmed in the administration groups of the lipid particles of Example 9, Example 10 and Example 11, compared to the negative control group.

(Results of Experimental Example 7)

Cell-mediated immune response specific to gp46 and Tax in C57BL/6 mice

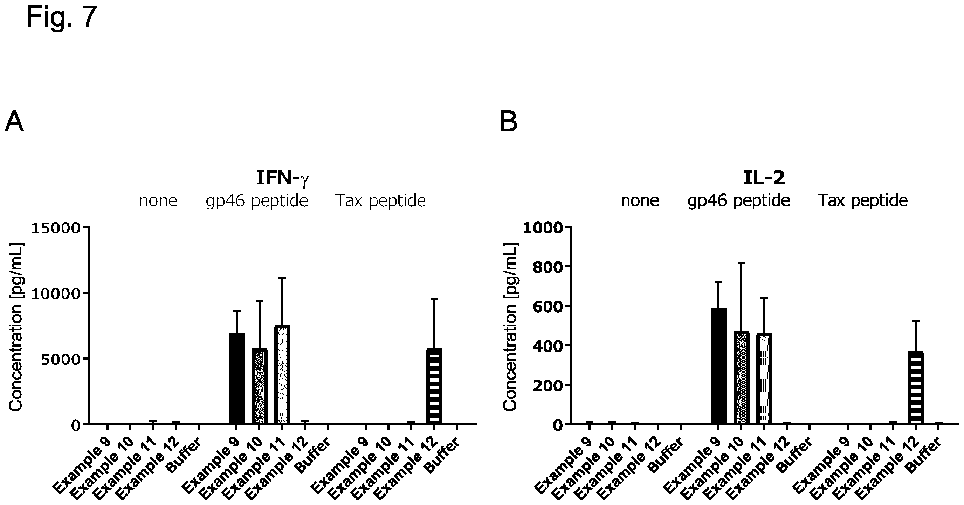

The gp46-specific and IL-2-specific cell-mediated immunity induced by administration of each of the lipid particles of Example 9, Example 10, Example 11 and Example 12 were evaluated based on IFN-y production level and IL-2 production level as indexes (Figure 7). As a result, a high-level gp46-specific cell-mediated immune response was confirmed in the lipid particle administration groups of Example 9, Example 10 and Example 11, compared to the negative control group. In the lipid particle administration group of Example 12, a high-level Tax-specific cell-mediated immune response was confirmed, compared to the negative control group.

(Results of Experimental Example 8)

Antigen protein expression level by lipid particles of Example 9 to Example 12

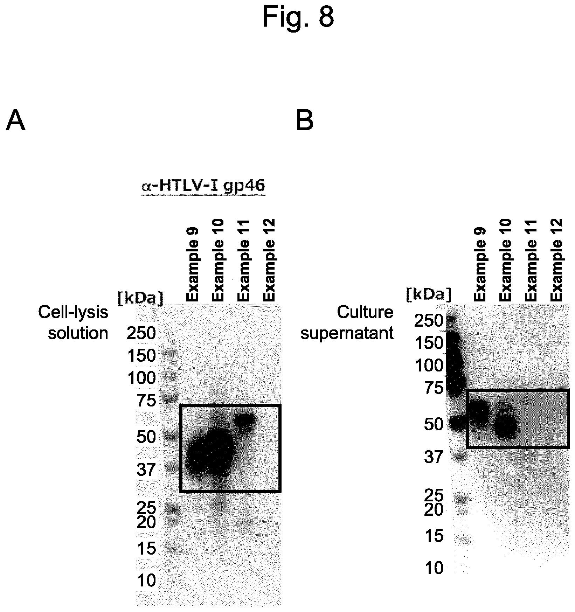

In the culture supernatants of CHO-S cells treated with the lipid particles of Example 9 to Example 12 and cell lysates, the expression levels of antigen proteins expressed by the lipid particles were evaluated (Figure 8). As a result, it was confirmed that antigen proteins, to which the anti-gp46 antibodies expressed by individual lipid particles bind, are expressed. Note that, the size of the gp46 protein was 39 kDa.

[Examples 25 to 29]

Preparation of gp46-Fib mRNA

(1) Preparation of template DNA for *in-vitro* transcription (IVT) of gp46-Fib

Plasmids were constructed for preparing template DNA for use in *in-vitro* transcription (IVT). Plasmids were prepared by introducing DNA fragments (SEQ ID NOs: 21 to 25) each containing a sequence prepared by ligating a T7 promoter sequence, a 5'-UTR sequence of human β-globin, KOZAK sequence, a coding region of gp46-Fib, 3'-UTR sequence of human β-globin and a polyA sequence, sequentially in the order (Examples 25 to 29 were carried out by using plasmids prepared by introducing DNA fragments of SEQ ID NOs: 21 to 25, respectively).

(2) Linearization of template DNA

In Nuclease-free water (268 μL), in which the plasmid (300 μg) obtained in the above step (1) was dissolved, a 10X NE Buffer 3.1 and BspQI (32 μL, New England Biolabs catalog # R0712L) were added. The mixture was incubated at 50°C for 16 hours, and then, 80°C for 20 minutes. To this, ethanol (900 μL) and a 3 mol/L sodium acetate solution (30 μL) were mixed. The mixture was allowed to stand still at -80°C for 4 hours. Centrifugation (4°C, 15000 × g, 30 min.) was carried out and the supernatant was discarded. To the residue, 70% ethanol was added. The suspension was centrifugated (4°C, 15000 × g, 10 min.). The supernatant was discarded and the residue was air-dried. The obtained residue was dissolved in Nuclease-free water to prepare a 500 μg/mL solution.

(3) Preparation of gp46-Fib mRNA by *in-vitro* transcription

The 500 μg/mL template DNA (15 μL) obtained in the above step (2), 100 mM CleanCap AG (15 μL, TriLink catalog # N-7113), 100 mM ATP (15 μL, Hongene catalog # R1331), 100 mM GTP (15 μL, Hongene catalog # R2331), 100 mM 5-Me-CTP (15 μL, Hongene catalog # R3-029), 100 mM 5-Me-UTP (15 μL, Hongene catalog # R5-104), Nuclease-free water (113 μL), 5 × IVT buffer (60 μL, 400 mM HEPES-KOH pH7.5, 400 mM DTT, 120 mM MgCl2, 10 mM Spermidine), T7 RNA Polymerase (15 μL, Promega catalog # P407X), RNase Inhibitor (15 μL, Promega catalog # P261X) and Pyrophosphatase (7.5 μL, Hongene catalog # ON-025) were mixed. The mixture was incubated at 37°C for 4 hours. RQ1 RNase-Free DNase (7.5 μL, Promega catalog # M6101) was added in the mixture and the resultant mixture was incubated at 37°C for 30 min. To the mixture, a 10 M ammonium acetate solution (100 μL) was added. The mixture was allowed to stand still at -20°C for 2 hours. After the mixture was centrifuged (4°C, 15000 × g, 30 min.), the supernatant was discarded. To the residue, 70% ethanol was added. The suspension was centrifugated (4°C, 15000 × g, 10 min.). The supernatant was discarded and the residue was air-dried. The obtained residue was dissolved in Nuclease-free water and purified with NucleoSpin (Macherey-Nagel catalog # 740948.50) to obtain the desired mRNA.

The obtained mRNA molecules have the sequences of SEQ ID NOs: 31 to 35, a Cap-1 structure at the 5'-end and 5-methylcytidine and 5-methyluridine in place of cytidine and uridine, respectively. It was confirmed that the mRNA molecules have desired lengths based on the analysis by LabChip GX Touch Standard RNA Reagent Kit (PerkinElmer catalog #CLS960010).

[Examples 30 to 34]

Preparation of nucleic-acid lipid particles encapsulating mRNA obtained in Examples 25 to 29

(1) Preparation of nucleic-acid lipid particle encapsulating mRNA

The nucleic-acid lipid particles listed in Table 3 were prepared in the same manner as in Examples 9 to 24 except that the mRNA molecules obtained in Examples 25 to 29 were respectively used in place of the mRNA molecules obtained in Examples 1 to 8.

(2) Property evaluation of nucleic-acid lipid particle encapsulating mRNA

The properties of the nucleic-acid lipid particles were evaluated in the same manner as in Examples 9 to 24. The results are listed in Table 4.

[Table 3]

| Example No. | mRNA | DSPC | Chol | LP | PEG-DMG | Lipids/mRNA (wt/wt) |
|---|---|---|---|---|---|---|
| Example 35 | Example 25 | 17.5% | 21% | 60% (LP2) | 1.5% | 25 |
| Example 36 | Example 26 | 17.5% | 21% | 60% (LP2) | 1.5% | 25 |
| Example 37 | Example 27 | 17.5% | 21% | 60% (LP2) | 1.5% | 25 |
| Example 38 | Example 28 | 17.5% | 21% | 60% (LP2) | 1.5% | 25 |
| Example 39 | Example 29 | 17.5% | 21% | 60% (LP2) | 1.5% | 25 |
| Example 40 | Example 30 | 17.5% | 21% | 60% (LP2) | 1.5% | 25 |

[Table 4]

| Example No. | Encapsulation rate (%) | Particle size (nm) |
|---|---|---|
| Example 35 | 95% | 107±13 |
| Example 36 | 96% | 130±28 |
| Example 37 | 96% | 111±22 |
| Example 38 | 96% | 103±16 |
| Example 39 | 96% | 108±13 |
| Example 40 | 97% | 102±28 |

(Experimental Example 9)

Analysis for expression of gp46 protein

The nucleic-acid lipid particles of Examples 30 to 34 were added to CHO-S cells (Thermo Fisher Scientific) such that the mRNA concentration of a medium became 3 μg/mL. As the negative control, a buffer of the same volume as the liquid used in the particles of Examples 35 to 39 was added. Three days after addition of the particles, the culture supernatant was recovered. The gp46 protein in the culture supernatant was detected by diluting the culture supernatant 10X with D-PBS, immobilizing the supernatant onto a 96 half-well plate and applying Enzyme-Linked Immunosorbent Assay (ELISA) using the serum of a mouse administered with the (nucleic acid) lipid particles of Example 10.

(Results of Experimental Example 9)

Ability of nucleic-acid lipid particles of Examples 30 to 34 to induce expression of gp46

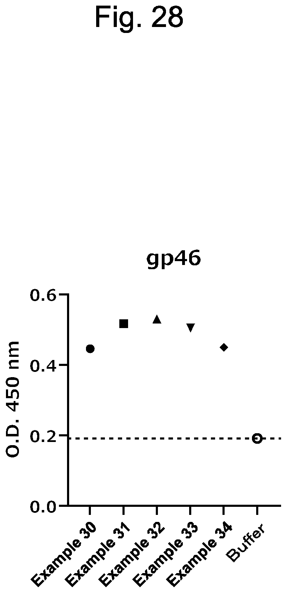

The expression levels of gp46 protein in the culture supernatants of CHO-S cells treated with nucleic-acid lipid particles of Examples 30 to 34, were evaluated. As a result, the CHO-S cells treated with nucleic-acid lipid particles of Examples 30 to 34 expressed gp46 protein (Figure 28).

Industrial Applicability

The present invention can be used prevention and/or treatment of infection with HTLV-1.

[Sequence Listing Free text]

SEQ ID NO: 1: Nucleotide sequence of template plasmid DNA for IVT of SU gp46
SEQ ID NO: 2: Nucleotide sequence of a sense primer
SEQ ID NO: 3: Nucleotide sequence of an antisense primer
SEQ ID NO: 4: Nucleotide sequence of template DNA for *in-vitro* transcription (IVT) of SU gp46

SEQ ID NO: 5: Nucleotide sequence of template plasmid DNA for IVT of HTLV-I gp46-Fib
SEQ ID NO: 6: Nucleotide sequence of template DNA for *in-vitro* transcription (IVT) of gp46-Fib
SEQ ID NO: 7: Nucleotide sequence of template plasmid DNA for IVT of HTLV-I dgp62-Fib
SEQ ID NO: 8: Nucleotide sequence of template DNA for *in-vitro* transcription (IVT) of dgp62-Fib
SEQ ID NO: 9: Nucleotide sequence of template plasmid DNA for IVT of HTLV-I sec Tax mutant
SEQ ID NO: 10: Nucleotide sequence of template DNA for *in-vitro* transcription (IVT) of sec Tax mutant
SEQ ID NO: 11: Amino acid sequence of Tax wild type
SEQ ID NO: 12: Amino acid sequence of Tax mutant (T130A/L131S/L319R/L320S)
SEQ ID NO: 13: Amino acid sequence of secretory Tax mutant (T130A/L131S/L319R/L320S)
SEQ ID NO: 14: Amino acid sequence of gp46
SEQ ID NO: 15: Amino acid sequence of gp46-Fib
SEQ ID NO: 16: Amino acid sequence of dgp62-Fib
SEQ ID NO: 17: mRNA of SU gp46
SEQ ID NO: 18: mRNA of gp46-Fib
SEQ ID NO: 19: mRNA of dgp62-Fib
SEQ ID NO: 20: mRNA of sec Tax mutant
SEQ ID NO: 21: nucleotide sequence of the template DNA for gp46-Fib + polyA95
SEQ ID NO: 22: Nucleotide sequence of template DNA for gp46-Fib + polyA80
SEQ ID NO: 23: Nucleotide sequence of template DNA for gp46-Fib + polyA60
SEQ ID NO: 24: Nucleotide sequence of template DNA for gp46-Fib + polyA40
SEQ ID NO: 25: Nucleotide sequence of template DNA for gp46-Fib + polyA20
SEQ ID NO: 26: Nucleotide sequence of template DNA for a sec Tax mutant + polyA95
SEQ ID NO: 27: Nucleotide sequence of template DNA for a sec Tax mutant + polyA80
SEQ ID NO: 28: Nucleotide sequence of template DNA for a sec Tax mutant + polyA60
SEQ ID NO: 29: Nucleotide sequence of template DNA for a sec Tax mutant + polyA40
SEQ ID NO: 30: Nucleotide sequence of template DNA for a sec Tax mutant + polyA20
SEQ ID NO: 31: mRNA sequence of gp46-Fib + polyA95
SEQ ID NO: 32: mRNA sequence of gp46-Fib + polyA80
SEQ ID NO: 33: mRNA sequence of gp46-Fib + polyA60
SEQ ID NO: 34: mRNA sequence of gp46-Fib + polyA40
SEQ ID NO: 35: mRNA sequence of gp46-Fib + polyA20
SEQ ID NO: 36: mRNA sequence of sec Tax mutant + polyA95
SEQ ID NO: 37: mRNA sequence of sec Tax mutant + polyA80
SEQ ID NO: 38: mRNA sequence of sec Tax mutant + polyA60
SEQ ID NO: 39: mRNA sequence of sec Tax mutant + polyA40
SEQ ID NO: 40: mRNA sequence of sec Tax mutant + polyA20

All publications, patents and patent applications cited in the specification are incorporated herein in their entireties by reference.

## Claims

1. A lipid particle encapsulating a nucleic acid capable of expressing a gp46 antigen or a Tax antigen of human T-cell leukemia virus type 1 (HTLV-1), wherein the lipid comprises a cationic lipid represented by general formula (Ia):

[Formula 1]

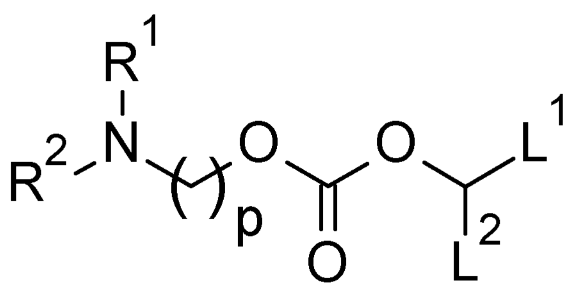

(Ia)

or a pharmaceutically acceptable salt thereof,
wherein

$R^1$ and $R^2$ each independently represent a $C_1$-$C_3$ alkyl group;
$L^1$ represents a $C_{17}$-$C_{19}$ alkenyl group optionally having one or more $C_2$-$C_4$ alkanoyloxy groups;
$L^2$ represents a $C_{10}$-$C_{19}$ alkyl group optionally having one or more $C_2$-$C_4$ alkanoyloxy groups, or a $C_{10}$-$C_{19}$ alkenyl group optionally having one or more $C_2$-$C_4$ alkanoyloxy groups; and p is 3 or 4.

2. The particle according to Claim 1, wherein, in general formula (Ia), $R^1$ and $R^2$ both are methyl groups.

3. The particle according to claim 1 or 2, wherein, in general formula (Ia), "p" is 3.

4. The particle according to any one of claims 1 to 3, wherein, in general formula (Ia), $L^1$ is a $C_{17}$-$C_{19}$ alkenyl group optionally having one or more acetoxy groups.

5. The particle according to any one of claims 1 to 4, wherein in general formula (Ia), $L^2$ is a $C_{10}$-$C_{12}$ alkyl group optionally having one or more acetoxy groups, or a $C_{10}$-$C_{19}$ alkenyl group optionally having one or more acetoxy groups.

6. The particle according to any one of claims 1 to 4, wherein in general formula (Ia), $L^2$ is a $C_{10}$-$C_{12}$ alkyl group optionally having one or more acetoxy groups or a $C_{17}$-$C_{19}$ alkenyl group optionally having one or more acetoxy groups.

7. The particle according to any one of claims 1 to 6, wherein in general formula (Ia), $L^1$ is an (R)-11-acetyloxy-cis-8-heptadecenyl group, a cis-8-heptadecenyl group, or an (8Z,11Z)-heptadecadienyl group.

8. The particle according to any one of claims 1 to 7, wherein in general formula (Ia), $L^2$ is a decyl group, a cis-7-decenyl group, a dodecyl group, or an (R)-11-acetyloxy-cis-8-heptadecenyl group.

9. The particle according to claim 1, wherein the cationic lipid is represented by the following structural formula:

[Formula 2]

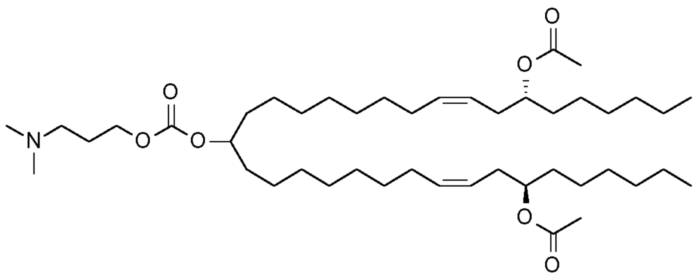

10. The particle according to claim 1, wherein the cationic lipid is represented by the following structural formula:

[Formula 3]

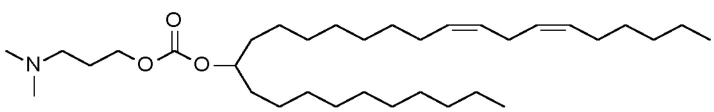

11. The particle according to claim 1, wherein the cationic lipid is represented by the following structural formula:

[Formula 4]

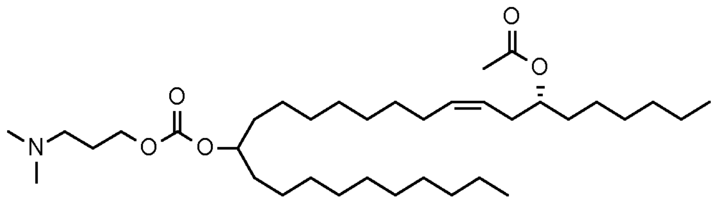

12. The particle according to claim 9 or 10, wherein the lipid further comprises an amphipathic lipid, a sterol and a PEG lipid.

13. The particle according to claim 11, wherein the lipid further comprises an amphipathic lipid, a sterol and a PEG lipid.

14. The particle according to claim 12, wherein the amphipathic lipid is at least one selected from the group consisting of distearoylphosphatidylcholine, dioleoylphosphatidylcholine and dioleoyl phosphatidylethanolamine.

15. The particle according to claim 13, wherein the amphipathic lipid is at least one selected from the group consisting of distearoylphosphatidylcholine, dioleoylphosphatidylcholine and dioleoyl phosphatidylethanolamine.

16. The particle according to claim 12 or 14, wherein the sterol is cholesterol.

17. The particle according to claim 13 or 15, wherein the sterol is cholesterol.

18. The particle according to any one of claims 12, 14 and 16, wherein the PEG lipid is 1,2-dimyristoyl-sn-glycerol methoxypolyethylene glycol and/or N-[methoxypoly(ethylene glycol)2000]carbamoyl]-1,2-dimyristyloxypropyl-3-amine.

19. The particle according to any one of claims 13, 15 and 17, wherein the PEG lipid is 1,2-dimyristoyl-sn-glycerol methoxypolyethylene glycol and/or N-[methoxypoly(ethylene glycol)2000]carbamoyl]-1,2-dimyristyloxypropyl-3-amine.

20. The particle according to any one of claims 12 to 19, wherein the lipid composition of the amphipathic lipid, the sterol, the cationic lipid, and the PEG lipid is as follows: 5 to 25% of the amphipathic lipid, 10 to 55% of the sterol, 40 to 65% of the cationic lipid, and 1 to 5% of the PEG lipid on a molar basis.

21. The particle according to claim 20, wherein the amphipathic lipid is contained in a ratio of 10 to 25%.

22. The particle according to any one of claims 12, 14, 16 and 18 wherein the lipid composition of the amphipathic lipid, the sterol, the cationic lipid, and the PEG lipid is as follows: 5 to 15% of the amphipathic lipid, 35 to 50% of the sterol, 40 to 55% of the cationic lipid, and 1 to 3% of the PEG lipid on a molar basis.

23. The particle according to claim 22, wherein the amphipathic lipid, sterol, cationic lipid and PEG lipid are contained in a ratio of 10 to 15%, 35 to 45%, 40 to 50%, and 1 to 2.5%, respectively.

24. The particle according to claim 23, wherein the PEG lipid is contained in a ratio of 1 to 2%.

25. The particle according to any one of claims 13, 15, 17 and 19, wherein the lipid composition of the amphipathic lipid, the sterol, the cationic lipid, and the PEG lipid is as follows: 10 to 25% of the amphipathic lipid, 10 to 50% of the sterol, 40 to 65% of the cationic lipid, and 1 to 3% of the PEG lipid on a molar basis.

26. The particle according to claim 25, wherein the sterol, cationic lipid and PEG lipid are contained in a ratio of 10 to 45%, 42.5 to 65%, and 1 to 2.5%, respectively.

27. The particle according to claim 26, wherein the PEG lipid is contained in a ratio of 1 to 2%.

28. The particle according to any one of claims 20 to 27, wherein a weight ratio of the total lipid to the nucleic acid is

from 15 to 30.

**29.** The particle according to claim 28, wherein the weight ratio of the total lipid to the nucleic acid is from 15 to 25.

**30.** The particle according to claim 29, wherein the weight ratio of the total lipid to the nucleic acid is from 17.5 to 22.5.

**31.** The particle according to any one of claims 1 to 30, wherein the gp46 antigen of human T-cell leukemia virus type 1 (HTLV-1) is a fusion protein with an oligomerization domain.

**32.** The particle according to claim 31, wherein the oligomerization domain is fibritin.

**33.** The particle according to claim 31 or 32, wherein the gp46 antigen of human T-cell leukemia virus type 1 (HTLV-1) consists of an amino acid sequence having an identity of at least 95% with the amino acid sequence of SEQ ID NO: 14.

**34.** The particle according to any one of claims 1 to 33, wherein the Tax antigen of human T-cell leukemia virus type 1 (HTLV-1) has at least one mutation selected from the group consisting of T130A, L131S, L319R and L320S relative to the amino acid sequence of SEQ ID NO: 11, and the amino acid sequence consisting of amino acids except the mutant amino acids has an identity of at least 95% with the amino acid sequence of SEQ ID NO: 11.

**35.** The particle according to claim 34, wherein the Tax antigen of human T-cell leukemia virus type 1 (HTLV-1) has mutations of T130A, L131S, L319R and L320S relative to the amino acid sequence of SEQ ID NO: 11, and the amino acid sequence consisting of amino acids except the mutant amino acids has an identity of at least 95% with the amino acid sequence of SEQ ID NO: 11.

**36.** The particle according to any one of claims 1 to 35, wherein the Tax antigen of human T-cell leukemia virus type 1 (HTLV-1) is a fusion protein with a signal peptide.

**37.** The particle according to claim 36, wherein the signal peptide is a peptide consisting of the sequence from positions 1 to 18 in the amino acid sequence of SEQ ID NO: 13.

**38.** The particle according to any one of claims 31 to 37, wherein nucleic acid capable of expressing the gp46 antigen or Tax antigen of human T-cell leukemia virus type 1 (HTLV-1) is mRNA containing a cap structure (Cap), a 5'-noncoding region (5'-UTR), a coding region of the gp46 antigen or Tax antigen, a 3'-noncoding region (3'-UTR) and poly A tail (polyA).

**39.** The particle according to claim 38, wherein the sequence of the nucleic acid capable of expressing the gp46 antigen of human T-cell leukemia virus type 1 (HTLV-1) consists of a nucleotide sequence having an identity of at least 90% with the sequence of SEQ ID NO: 17 or 18.

**40.** The particle according to claim 38, wherein the sequence of the nucleic acid capable of expressing the Tax antigen of human T-cell leukemia virus type 1 (HTLV-1) consists of a nucleotide sequence having an identity of at least 90% with the sequence of SEQ ID NO: 20.

**41.** The particle according to any one of claims 1 to 40, wherein the nucleic acid comprises at least one modified nucleotide.

**42.** The particle according to claim 41, wherein the modified nucleotide comprises at least one of a pyrimidine nucleotide substituted at position 5 and/or pseudouridine optionally substituted at position 1.

**43.** The particle according to claim 41, wherein the modified nucleotide comprises at least one selected from the group consisting of 5-methylcytidine, 5-methoxyuridine, 5-methyluridine, pseudouridine, and a 1-alkylpseudouridine.

**44.** The particle according to claim 41, wherein the modified nucleotide comprises at least one selected from the group consisting of 5-methylcytidine, 5-methyluridine, and 1-methylpseudouridine.

**45.** The particle according to any one of claims 1 to 44, having an average particle size of 30 to 300 nm.

**46.** Use of the particle according to any one of claims 1 to 45, for preparing a composition for preventing and/or treating

infection with human T-cell leukemia virus type 1 (HTLV-1).

**47.** A composition containing the particle according to any one of claims 1 to 45.

**48.** The composition according to claim 47 for expressing the gp46 antigen or Tax antigen of human T-cell leukemia virus type 1 (HTLV-1) *in vivo* or *in vitro.*

**49.** The composition according to claim 47 or 48 for use as a medicament.

**50.** The composition according to claim 49 for inducing an immune response to human T-cell leukemia virus type 1 (HTLV-1).

**51.** The composition according to claim 49 or 50 for preventing and/or treating infection with human T-cell leukemia virus type 1 (HTLV-1).

**52.** The composition according to claim 49 or 50 for preventing the onset of and/or treating a disease caused by HTLV-1 selected from the group consisting of adult T cell leukemia/lymphoma (ATLL), HTLV-1 associated myelopathy (HAM) and HTLV-1 uveitis (HU), in an HTLV-1 infected person.

**53.** A method for expressing the gp46 antigen or Tax antigen of human T-cell leukemia virus type 1 (HTLV-1) *in vitro,* comprising introducing the composition according to any one of claims 47 to 50 into a cell.

**54.** A method for expressing the gp46 antigen or Tax antigen of human T-cell leukemia virus type 1 (HTLV-1) *in vivo*, comprising administering the composition according to any one of claims 47 to 51 to a mammal.

**55.** A method for inducing an immune response to human T-cell leukemia virus type 1 (HTLV-1), comprising administering the composition according to claim 49 or 50 to a mammal.

**56.** A method for preventing and/or treating infection with human T-cell leukemia virus type 1 (HTLV-1), comprising administering the composition according to any one of claims 49 to 52 to a mammal.

**57.** A method for preventing the onset of and/or treating a disease caused by HTLV-1 selected from the group consisting of adult T cell leukemia/lymphoma (ATLL), HTLV-1 associated myelopathy (HAM) and HTLV-1 uveitis (HU), comprising administering the composition according to any one of claims 49 to 52 to a mammal.

Fig. 1

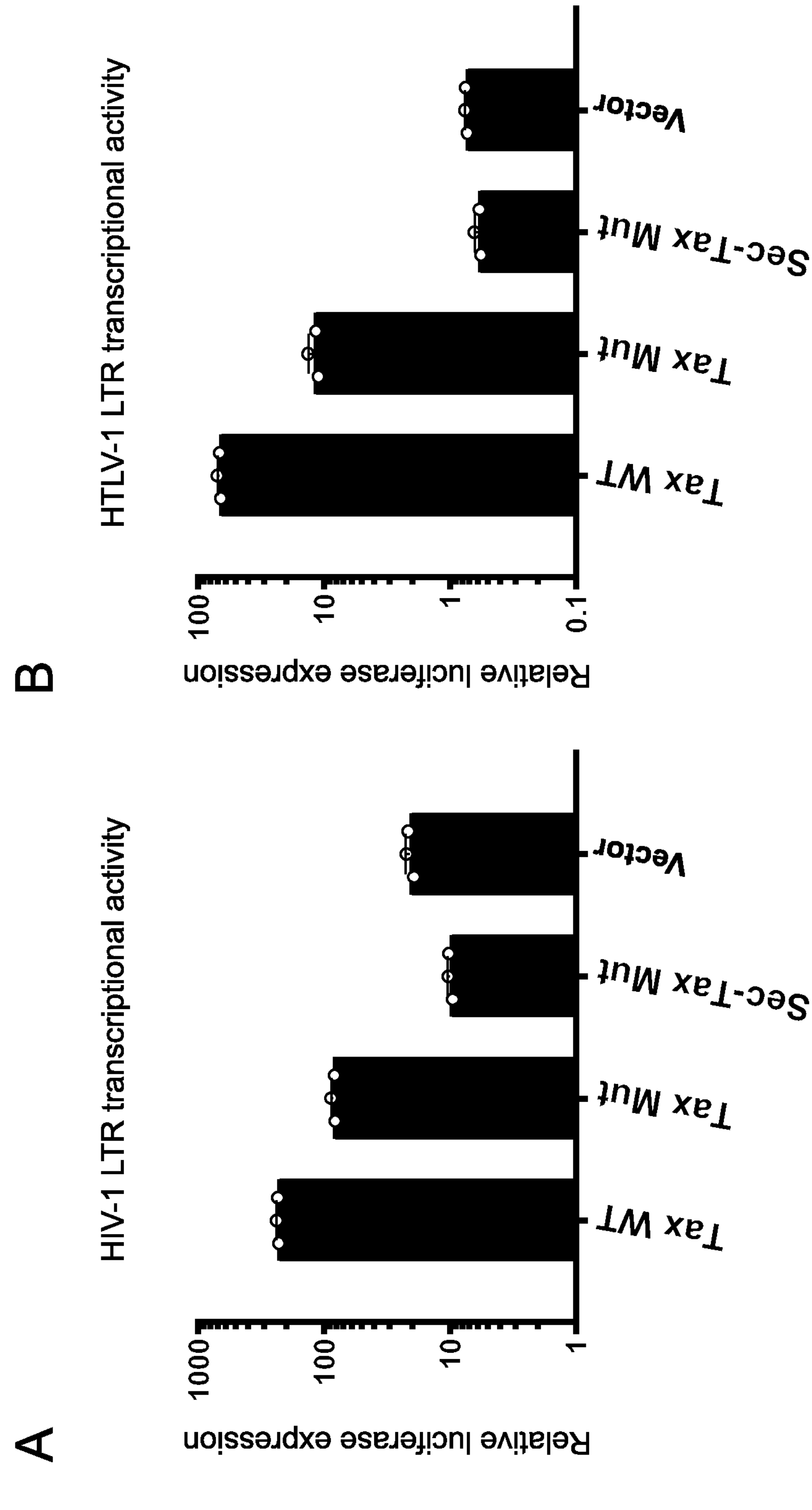

A
HIV-1 LTR transcriptional activity
Relative luciferase expression
1000
100
10
1
Tax WT
Tax Mut
Sec-Tax Mut
Vector
B
HTLV-1 LTR transcriptional activity
Relative luciferase expression
100
10
1
0.1
Tax WT
Tax Mut
Sec-Tax Mut
Vector

Fig. 2

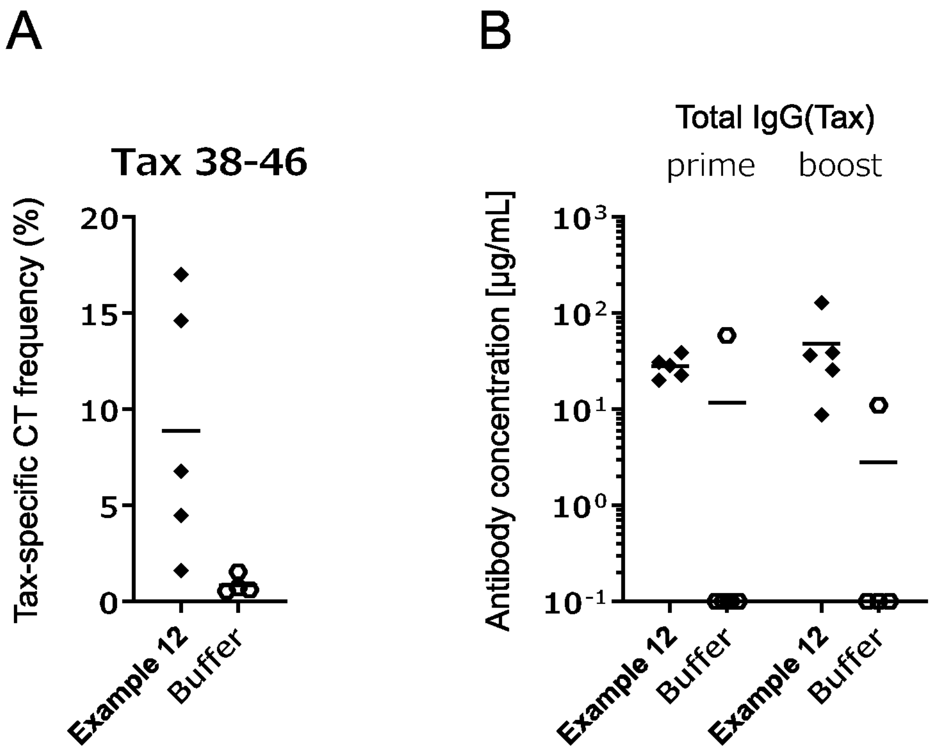
A
Tax 38-46
Tax-specific CT frequency (%)
20
15
10
5
0
Example 12
Buffer
B
Total IgG(Tax)
prime
boost
Antibody concentration [μg/mL]
$10^3$
$10^2$
$10^1$
$10^0$
$10^{-1}$
Example 12
Buffer
Example 12
Buffer

Fig. 3

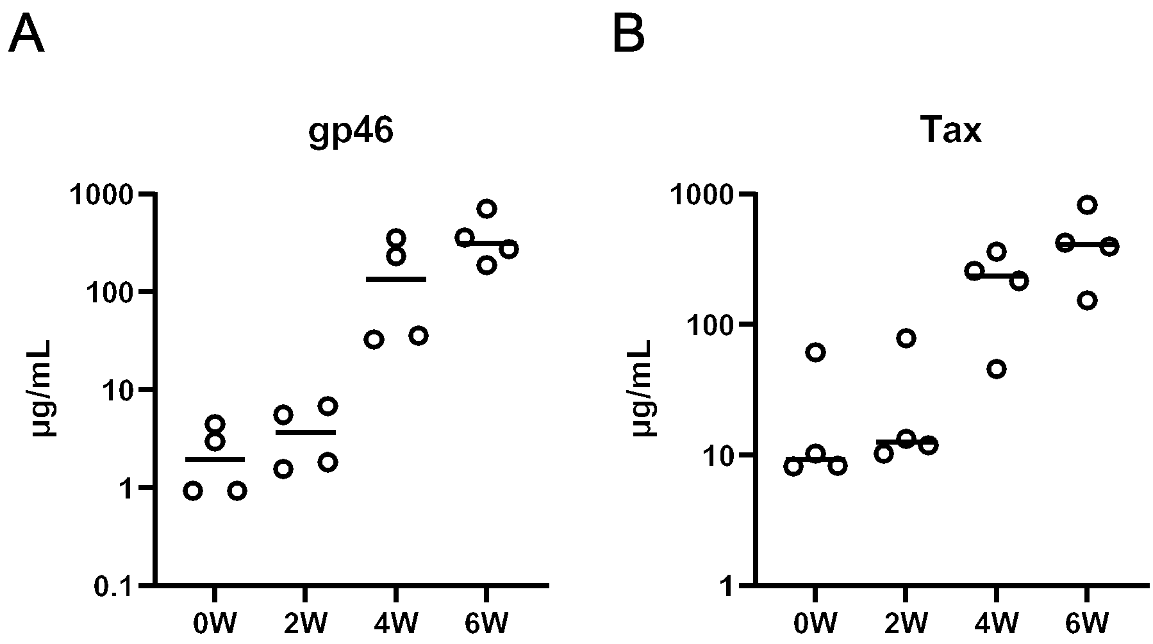
A
B
gp46
Tax
1000
100
10
1
0.1
μg/mL
0W
2W
4W
6W
1000
100
10
1
μg/mL
0W
2W
4W
6W

Fig. 4

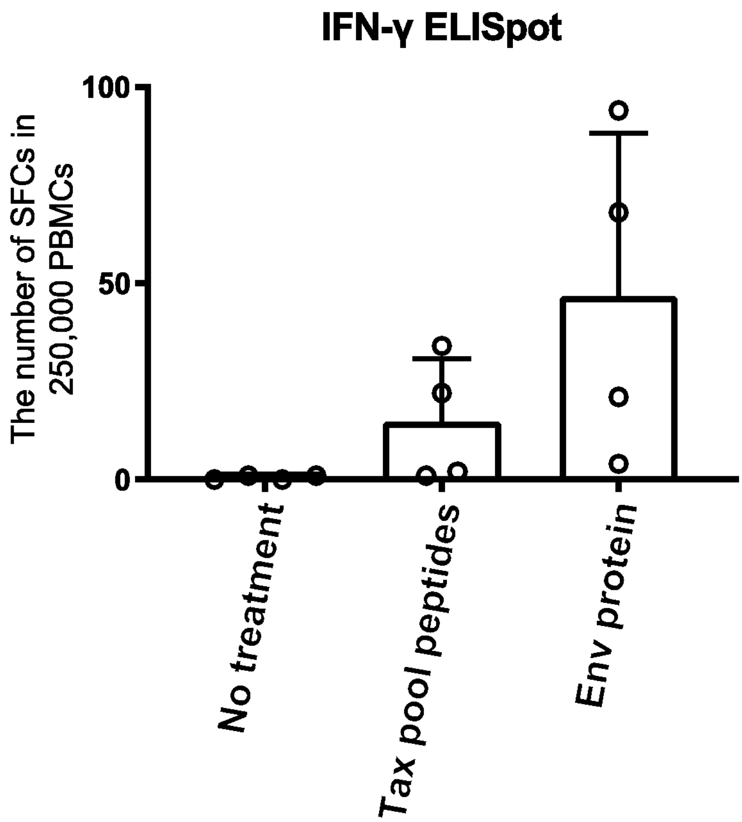
IFN-γ ELISpot
The number of SFCs in 250,000 PBMCs
100
50
0
No treatment
Tax pool peptides
Env protein

Fig. 5

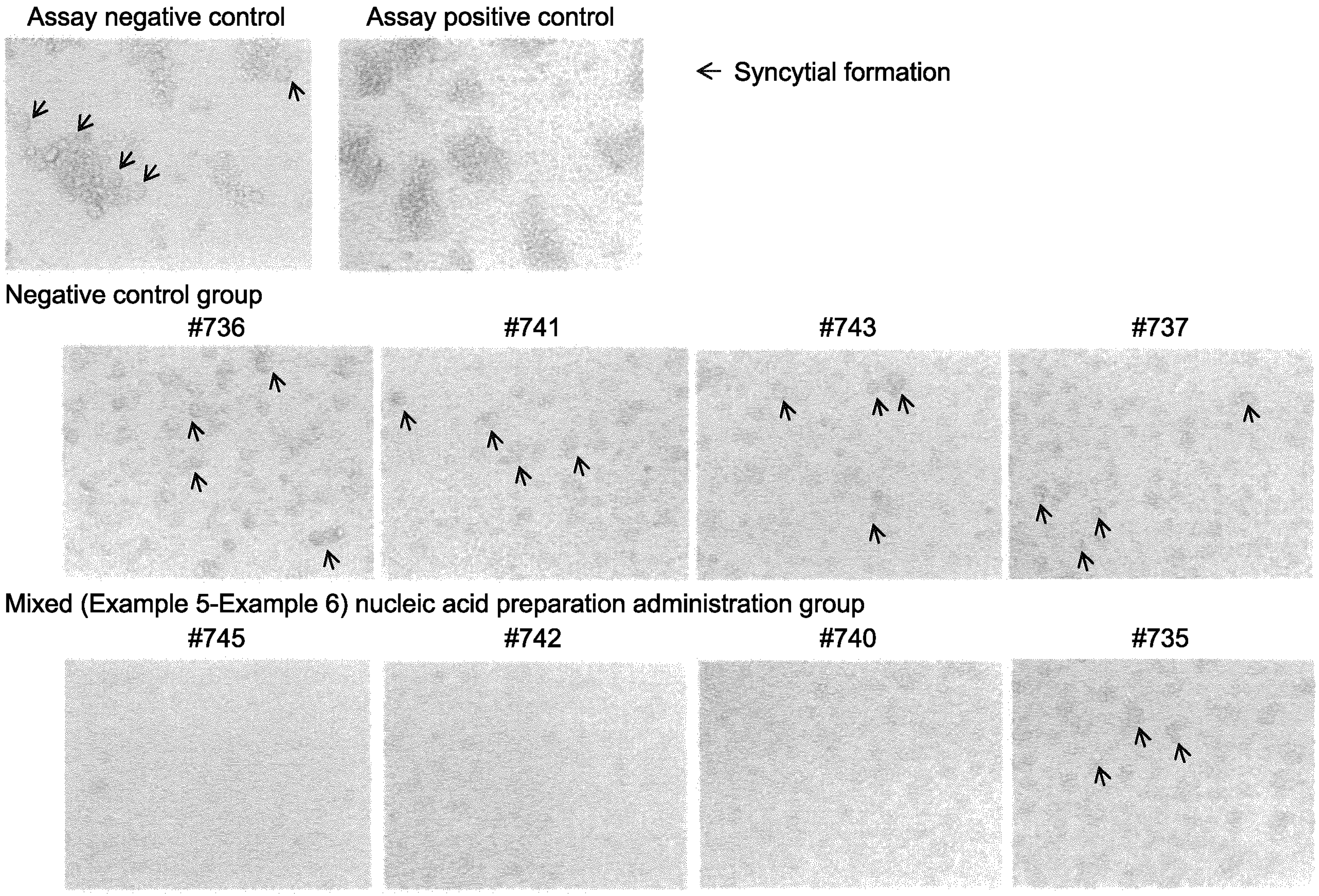
Assay negative control
Assay positive control
Syncytial formation
Negative control group
#736
#741
#743
#737
Mixed (Example 5-Example 6) nucleic acid preparation administration group
#745
#742
#740
#735

Fig. 6

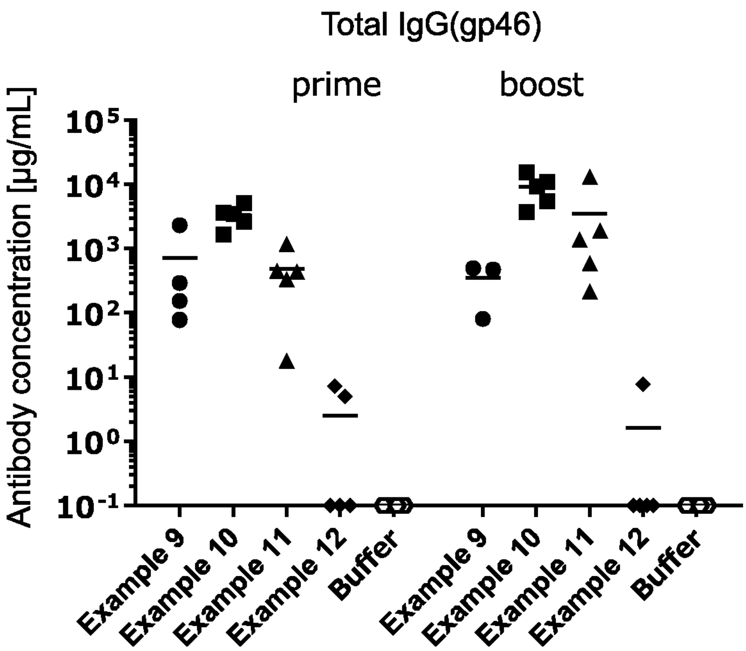
Total IgG(gp46)
prime
boost
Antibody concentration [µg/mL]
10⁵
10⁴
10³
10²
10¹
10⁰
10⁻¹
Example 9
Example 10
Example 11
Example 12
Buffer
Example 9
Example 10
Example 11
Example 12
Buffer

Fig. 7

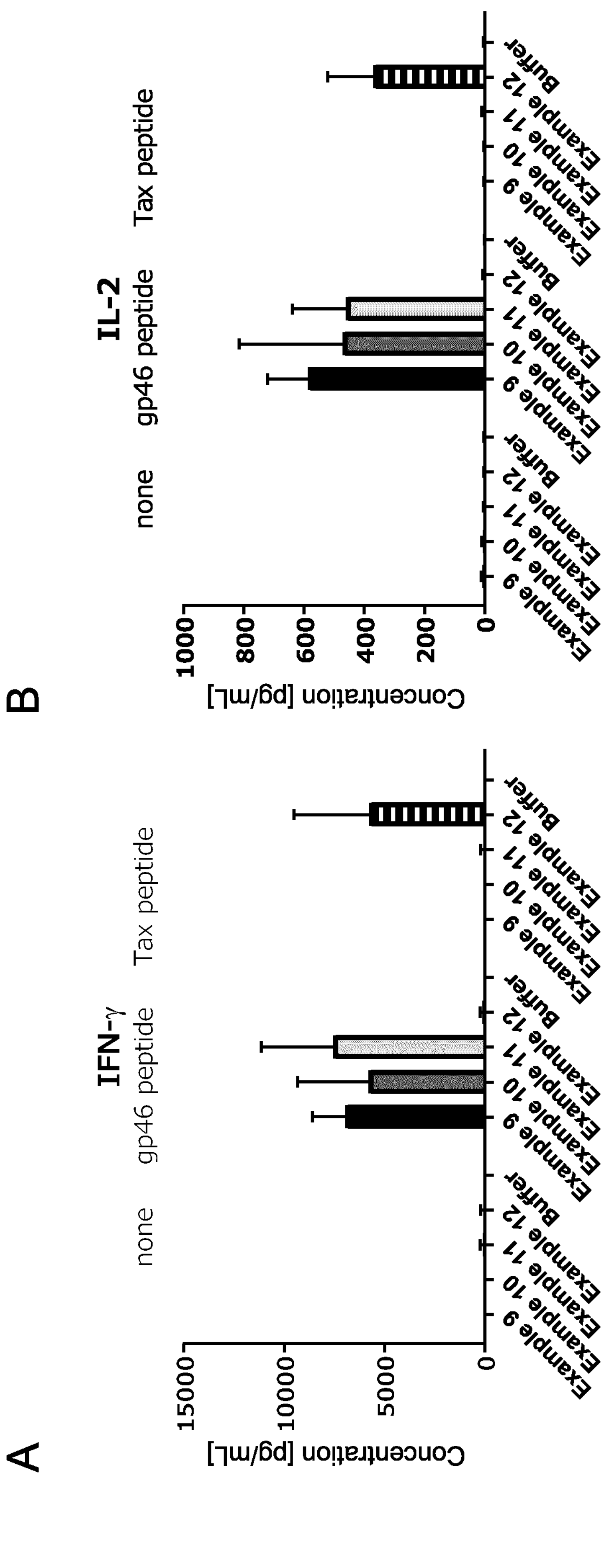
A
IFN-γ
none
gp46 peptide
Tax peptide
Concentration [pg/mL]
15000
10000
5000
0
Example 9
Example 10
Example 11
Example 12
Buffer
B
IL-2
none
gp46 peptide
Tax peptide
Concentration [pg/mL]
1000
800
600
400
200
0
Example 9
Example 10
Example 11
Example 12
Buffer

Fig. 8

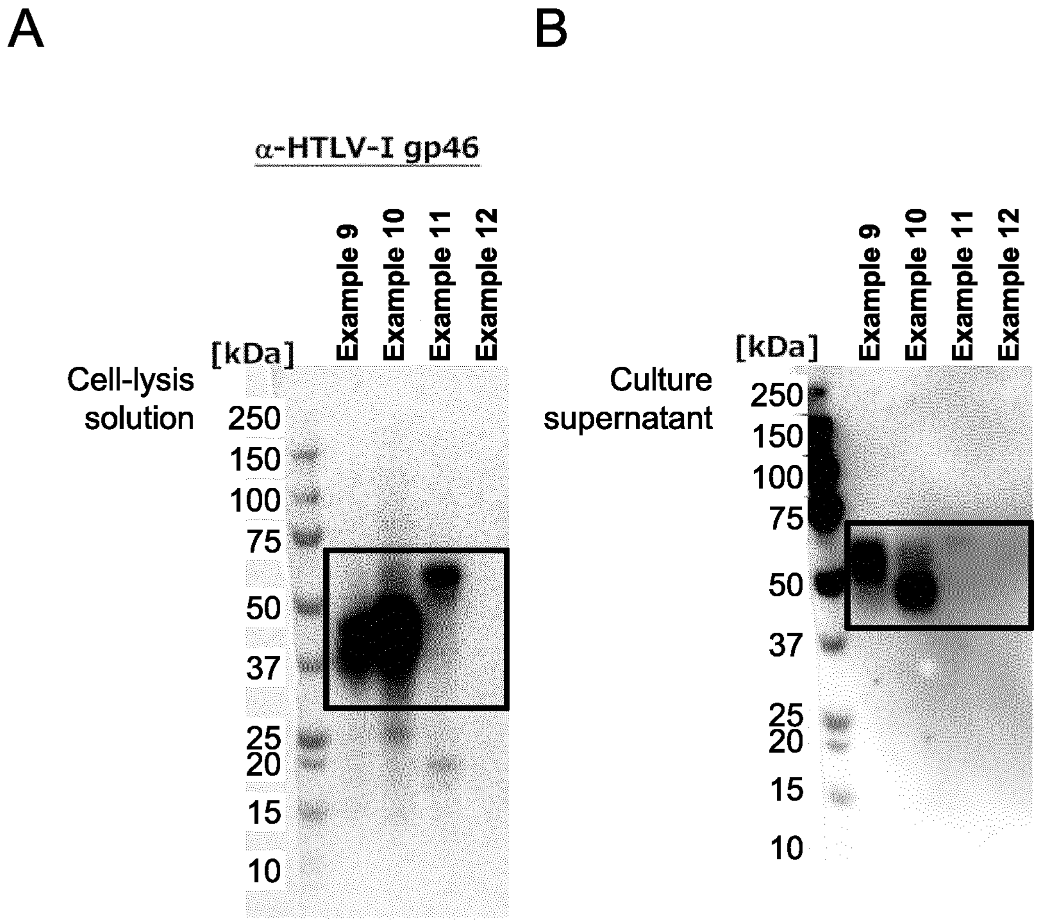
A
B
α-HTLV-I gp46
Cell-lysis solution
[kDa]
250
150
100
75
50
37
25
20
15
10
Example 9
Example 10
Example 11
Example 12
Culture supernatant
[kDa]
250
150
100
75
50
37
25
20
15
10
Example 9
Example 10
Example 11
Example 12

# Fig. 9

**Template plasmid DNA for HTLV-I SU gp46 IVT**

gctagcgtaatacgactcactataaggagacccaagctacatttgcttctgacaca
actgtgttcactagcaacctcaaacagacaccgccaccatgggcaagttcctggcc
acactgatcctgttcttccagttctgcccccctgatcttcggcgactacagccccag
ctgctgcaccctgacaatcggcgtgagcagctaccacagcaagccctgcaaccccg
cccagcccgtgtgcagctggaccctggacctgctggccctgagcgccgaccaggcc
ctgcagccccccctgccccaacctggtgagctacagcagctaccacgccacctacag
cctgtacctgttcccccactggaccaagaagcccaacagaaacggcggaggctact
acagcgccagctacagcgacccctgcagcctgaagtgcccctacctgggctgccag
agctggacatgcccctacacaggcgccgtgagcagcccctactggaagttccagca
cgacgtgaacttcacccaagaggtgagccggctgaacatcaacctgcacttcagca
agtgcggcttcccattcagcctgctggtggacgcccccggctacgaccccatctgg
ttcctgaacaccgagcccagccagctgcccccaacagccccacccctgctgcccca
cagcaacctggaccacatcctggaacccagcatcccctggaagagcaagctgctga
cactggtgcagctgaccctgcagagcaccaactacacctgcatcgtgtgcatcgac
cgggccagcctgagcacatggcacgtgctgtacagccccaacgtgagcgtgcccag
cagcagcagcacacccctgctgtaccccagcctggcactgcccgcccccccacctga
cactgcccttcaactggacccactgcttcgacccccagatccaggccatcgtgagc
agcccatgccacaacagcctgatcctgcccccattcagcctgagccccgtgcccac
actgggcagcagaagcagaagatgagctcgctttcttgctgtccaatttctattaa
aggttcctttgttccctaagtccaactactaaactgggggatattatgaagggcct
tgagcatctggattctgcctaataaaaacatttattttcattgcaaaaaaaaaaaa
aaaaaaaaaaaaaaaaaaaaaaaaaaaaaaaaaaaaaaaaaaaaaaaaaaaaaaaa
aaaaaaaaaaaaaaaaaaaaaaaaaaaaaaaaaaagagcactagt
(SEQ ID NO: 1)

GCTAGC(NheI site): Nucleotide No. 1-6
T7 promoter sequence: Nucleotide No. 8-24
G: Transcription initiation point: Nucleotide No. 25
5'-UTR sequence(including transcription initiation point and KOZAK sequence of nucleotide Nos. 89-94): Nucleotide No. 25-94
Coding region of SU gp46: Nucleotide No. 95-1033
3'-UTR sequence: Nucleotide No. 1034-1165
polyA: Nucleotide No. 1166-1267
ACTAGT(SpeI site): Nucleotide No. 1272-1277

Fig. 10

**sense primer**

**TAATACGACTCACTATAAGGAGAC**
**(SEQ ID NO: 2)**

**antisense primer**

**TTTTTTTTTTTTTTTTTTTTTTTTTTTTTTTTTTTTTTTTTTTTTTTTTTTTTTTT**
**TTTTTTTTTTTTTTTTTTTTTTTTTTTTTTTTTTTTTTTTTTTTGCAATGAAAATA**
**AATGTTTTTTATTAGGC**
**(SEQ ID NO: 3)**

# Fig. 11

**Template plasmid DNA for SU gp46 in vitro transcription (IVT)**

**taatacgactcactataaggagacccaagctacatttgcttctgacacaactgtgt
tcactagcaacctcaaacagacaccgccaccatgggcaagttcctggccacactga
tcctgttcttccagttctgcccccctgatcttcggcgactacagccccagctgctgc
accctgacaatcggcgtgagcagctaccacagcaagccctgcaaccccgcccagcc
cgtgtgcagctggaccctggacctgctggccctgagcgccgaccaggccctgcagc
ccccctgccccaacctggtgagctacagcagctaccacgccacctacagcctgtac
ctgttccccccactggaccaagaagcccaacagaaacggcggaggctactacagcgc
cagctacagcgacccctgcagcctgaagtgcccctacctgggctgccagagctgga
catgcccctacacaggcgccgtgagcagcccctactggaagttccagcacgacgtg
aacttcacccaagaggtgagccggctgaacatcaacctgcacttcagcaagtgcgg
cttcccattcagcctgctggtggacgcccccggctacgaccccatctggttcctga
acaccgagcccagccagctgcccccaacagccccacccctgctgccccacagcaac
ctggaccacatcctggaacccagcatcccctggaagagcaagctgctgacactggt
gcagctgaccctgcagagcaccaactacacctgcatcgtgtgcatcgaccgggcca
gcctgagcacatggcacgtgctgtacagccccaacgtgagcgtgcccagcagcagc
agcacacccctgctgtaccccagcctggcactgcccgcccccacctgacactgcc
cttcaactggacccactgcttcgacccccagatccaggccatcgtgagcagcccat
gccacaacagcctgatcctgcccccattcagcctgagccccgtgcccacactgggc
agcagaagcagaagatgagctcgctttcttgctgtccaatttctattaaaggttcc
tttgttccctaagtccaactactaaactgggggatattatgaagggccttgagcat
ctggattctgcctaataaaaacatttattttcattgcaaaaaaaaaaaaaaaaaaaaaaa
aaaaaaaaaaaaaaaaaaaaaaaaaaaaaaaaaaaaaaaaaaaaaaaaaaaaaaaaaaaa
aaaaaaaaaaaaaaaaaaaaaaaaaaaa
(SEQ ID NO: 4)**

# Fig. 12

**Template plasmid DNA for HTLV-I gp46-Fib IVT**

**gctagcgtaatacgactcactataaggagacccaagctacatttgcttctgacaca
actgtgttcactagcaacctcaaacagacaccgccaccatgggcaagttcctggcc
acactgatcctgttcttccagttctgcccccctgatcttcggcgactacagccccag
ctgctgcaccctgacaatcggcgtgagcagctaccacagcaagccctgcaaccccg
cccagcccgtgtgcagctggaccctggacctgctggccctgagcgccgaccaggcc
ctgcagccccccctgccccaacctggtgagctacagcagctaccacgccacctacag
cctgtacctgttcccccactggaccaagaagcccaacagaaacggcggaggctact
acagcgccagctacagcgacccctgcagcctgaagtgcccctacctgggctgccag
agctggacatgcccctacacaggcgccgtgagcagcccctactggaagttccagca
cgacgtgaacttcacccaagaggtgagccggctgaacatcaacctgcacttcagca
agtgcggcttcccattcagcctgctggtggacgcccccggctacgaccccatctgg
ttcctgaacaccgagcccagccagctgcccccaacagccccacccctgctgcccca
cagcaacctggaccacatcctggaacccagcatcccctggaagagcaagctgctga
cactggtgcagctgaccctgcagagcaccaactacacctgcatcgtgtgcatcgac
cgggccagcctgagcacatggcacgtgctgtacagccccaacgtgagcgtgcccag
cagcagcagcacacccctgctgtaccccagcctggcactgcccgcccccacctga
cactgcccttcaactggacccactgcttcgaccccagatccaggccatcgtgagc
agcccatgccacaacagcctgatcctgcccccattcagcctgagccccgtgcccac
actcggcagcggcagcggcagcggctacatccccgaggcccccagagacggccagg
cctacgtcagaaaggacggcgaatgggtgctgctgagcaccttcctgtgagctcgc
tttcttgctgtccaatttctattaaaggttcctttgttccctaagtccaactacta
aactgggggatattatgaagggccttgagcatctggattctgcctaataaaaaaca
tttattttcattgcaaaaaaaaaaaaaaaaaaaaaaaaaaaaaaaaaaaaaaaaaa
aaaaaaaaaaaaaaaaaaaaaaaaaaaaaaaaaaaaaaaaaaaaaaaaaaaaaaaa
aaaagagcactagt
(SEQ ID NO: 5)**

**GCTAGC(NheI site): Nucleotide No. 1-6
T7 promoter sequence: Nucleotide No. 8-24
G: Transcription initiation point: Nucleotide No. 25
5'-UTR sequence(including transcription initiation point and KOZAK sequence of nucleotide Nos. 89-94): Nucleotide No. 25-94
Coding region of gp46-Fib: Nucleotide No. 95-1114
3'-UTR sequence: Nucleotide No. 1115-1246
polyA: Nucleotide No. 1247-1348
ACTAGT(SpeI site): Nucleotide No. 1353-1358**

# Fig. 13

**Template plasmid DNA for gp46-Fib in vitro transcription (IVT)**

**taatacgactcactataaggagacccaagctacatttgcttctgacacaactgtgt**
**tcactagcaacctcaaacagacaccgccaccatgggcaagttcctggccacactga**
**tcctgttcttccagttctgcccccctgatcttcggcgactacagccccagctgctgc**
**accctgacaatcggcgtgagcagctaccacagcaagccctgcaaccccgcccagcc**
**cgtgtgcagctggaccctggacctgctggccctgagcgccgaccaggccctgcagc**
**cccccctgccccaacctggtgagctacagcagctaccacgccacctacagcctgtac**
**ctgttcccccactggaccaagaagcccaacagaaacggcggaggctactacagcgc**
**cagctacagcgacccctgcagcctgaagtgcccctacctgggctgccagagctgga**
**catgcccctacacaggcgccgtgagcagcccctactggaagttccagcacgacgtg**
**aacttcacccaagaggtgagccggctgaacatcaacctgcacttcagcaagtgcgg**
**cttcccattcagcctgctggtggacgcccccggctacgaccccatctggttcctga**
**acaccgagcccagccagctgcccccaacagccccacccctgctgccccacagcaac**
**ctggaccacatcctggaacccagcatcccctggaagagcaagctgctgacactggt**
**gcagctgaccctgcagagcaccaactacacctgcatcgtgtgcatcgaccgggcca**
**gcctgagcacatggcacgtgctgtacagccccaacgtgagcgtgcccagcagcagc**
**agcacacccctgctgtaccccagcctggcactgcccgcccccacctgacactgcc**
**cttcaactggacccactgcttcgacccccagatccaggccatcgtgagcagcccat**
**gccacaacagcctgatcctgcccccattcagcctgagccccgtgcccacactcggc**
**agcggcagcggcagcggctacatccccgaggcccccagagacggccaggcctacgt**
**cagaaaggacggcgaatgggtgctgctgagcaccttcctgtgagctcgctttcttg**
**ctgtccaatttctattaaaggttcctttgttccctaagtccaactactaaactggg**
**ggatattatgaagggccttgagcatctggattctgcctaataaaaaacatttattt**
**tcattgcaaaaaaaaaaaaaaaaaaaaaaaaaaaaaaaaaaaaaaaaaaaaaaaaaa**
**aaaaaaaaaaaaaaaaaaaaaaaaaaaaaaaaaaaaaaaaaaaaaaaaaaaaaaa**
**(SEQ ID NO: 6)**

# Fig. 14

**Template plasmid DNA for HTLV-I dgb62-FIb IVT**

gctagcgtaatacgactcactataaggagacccaagctacatttgcttctgacaca
actgtgttcactagcaacctcaaacagacaccgccaccatgggcaagttcctggcc
acactgatcctgttcttccagttctgcccccctgatcttcggcgactacagccccag
ctgctgcaccctgacaatcggcgtgagcagctaccacagcaagccctgcaaccccg
cccagcccgtgtgcagctggaccctggacctgctggccctgagcgccgaccaggcc
ctgcagccccccctgccccaacctggtgagctacagcagctaccacgccacctacag
cctgtacctgttcccccactggaccaagaagcccaacagaaacggcggaggctact
acagcgccagctacagcgacccctgcagcctgaagtgcccctacctgggctgccag
agctggacatgcccctacacaggcgccgtgagcagcccctactggaagttccagca
cgacgtgaacttcacccaagaggtgagccggctgaacatcaacctgcacttcagca
agtgcggcttcccattcagcctgctggtggacgcccccggctacgaccccatctgg
ttcctgaacaccgagcccagccagctgcccccaacagccccacccctgctgcccca
cagcaacctggaccacatcctggaacccagcatcccctggaagagcaagctgctga
cactggtgcagctgaccctgcagagcaccaactacacctgcatcgtgtgcatcgac
cgggccagcctgagcacatggcacgtgctgtacagccccaacgtgagcgtgcccag
cagcagcagcacacccctgctgtaccccagcctggcactgcccgcccccacctga
cactgcccttcaactggacccactgcttcgacccccagatccaggccatcgtgagc
agcccatgccacaacagcctgatcctgcccccattcagcctgagccccgtgcccac
actcggcagcggaagcggaagcgcagtgcccgtggccgtgtggctggtgagcgccc
tggccatgggagccggcgtggcaggcggaatcacaggcagcatgagcctggccagc
ggcaagagcctgctgcacgaggtggacaaggacatcagccagctgacccaggccat
cgtgaagaaccacaagaacctgctgaagatcgcccagtacgccgcccagaacagaa
gaggactggacctgctgttctgggagcaaggcggactgtgcaaagccctccaagag
cagtgcagattccccaacatcaccaacagccacgtgcccatcctgcaagagcggcc
cccactggaaaacagagtgctgacaggatggggcctgaactgggacctgggactga
gccagtgggccagagaggccggctacatccccgaggcccccagagacggacaggcc
tacgtcagaaaggacggcgaatgggtgctgctgagcaccttcctgtgagctcgctt
tcttgctgtccaatttctattaaaggttcctttgttccctaagtccaactactaaa
ctgggggatattatgaagggccttgagcatctggattctgcctaataaaaaacatt
tattttcattgcaaaaaaaaaaaaaaaaaaaaaaaaaaaaaaaaaaaaaaaaaaaa
aaaaaaaaaaaaaaaaaaaaaaaaaaaaaaaaaaaaaaaaaaaaaaaaaaaaaaaa
aagagcactagt
(SEQ ID NO: 7)

GCTAGC(NheI site): Nucleotide No. 1-6
T7 promoter sequence: Nucleotide No. 8-24
G: Transcription initiation point: Nucleotide No. 25
5'-UTR sequence(including transcription initiation point and KOZAK sequence of nucleotide Nos. 89-94): Nucleotide No. 25-94
Coding region of dgp62-Fib: Nucleotide No. 95-1504
3'-UTR sequence: Nucleotide No. 1505-1636
polyA: Nucleotide No. 1637-1738
ACTAGT(SpeI site): Nucleotide No. 1743-1748

# Fig. 15

**Template plasmid DNA for dgb62-FIb in vitro transcription (IVT)**

taatacgactcactataaggagacccaagctacatttgcttctgacacaactgtgt
tcactagcaacctcaaacagacaccgccaccatgggcaagttcctggccacactga
tcctgttcttccagttctgcccccctgatcttcggcgactacagccccagctgctgc
accctgacaatcggcgtgagcagctaccacagcaagccctgcaaccccgcccagcc
cgtgtgcagctggaccctggacctgctggccctgagcgccgaccaggccctgcagc
ccccctgccccaacctggtgagctacagcagctaccacgccacctacagcctgtac
ctgttcccccactggaccaagaagcccaacagaaacggcggaggctactacagcgc
cagctacagcgacccctgcagcctgaagtgcccctacctgggctgccagagctgga
catgcccctacacaggcgccgtgagcagcccctactggaagttccagcacgacgtg
aacttcacccaagaggtgagccggctgaacatcaacctgcacttcagcaagtgcgg
cttcccattcagcctgctggtggacgcccccggctacgaccccatctggttcctga
acaccgagcccagccagctgcccccaacagccccacccctgctgccccacagcaac
ctggaccacatcctggaacccagcatcccctggaagagcaagctgctgacactggt
gcagctgaccctgcagagcaccaactacacctgcatcgtgtgcatcgaccgggcca
gcctgagcacatggcacgtgctgtacagccccaacgtgagcgtgcccagcagcagc
agcacacccctgctgtaccccagcctggcactgcccgcccccacctgacactgcc
cttcaactggacccactgcttcgacccccagatccaggccatcgtgagcagcccat
gccacaacagcctgatcctgcccccattcagcctgagccccgtgcccacactcggc
agcggaagcggaagcgcagtgcccgtggccgtgtggctggtgagcgccctggccat
gggagccggcgtggcaggcggaatcacaggcagcatgagcctggccagcggcaaga
gcctgctgcacgaggtggacaaggacatcagccagctgacccaggccatcgtgaag
aaccacaagaacctgctgaagatcgcccagtacgccgcccagaacagaagaggact
ggacctgctgttctgggagcaaggcggactgtgcaaagccctccaagagcagtgca
gattccccaacatcaccaacagccacgtgcccatcctgcaagagcggcccccactg
gaaaacagagtgctgacaggatggggcctgaactgggacctgggactgagccagtg
ggccagagaggccggctacatccccgaggcccccagagacggacaggcctacgtca
gaaaggacggcgaatgggtgctgctgagcaccttcctgtgagctcgctttcttgct
gtccaatttctattaaaggttcctttgttccctaagtccaactactaaactgggg
atattatgaagggccttgagcatctggattctgcctaataaaaaacatttattttc
attgcaaaaaaaaaaaaaaaaaaaaaaaaaaaaaaaaaaaaaaaaaaaaaaaaaaaa
aaaaaaaaaaaaaaaaaaaaaaaaaaaaaaaaaaaaaaaaaaaaaaaaaaaa
**(SEQ ID NO: 8)**

# Fig. 16

**Template plasmid DNA for HTLV-I sec Tax mutant IVT**

**gctagcgtaatacgactcactataaggagacccaagctacatttgcttctgacaca
actgtgttcactagcaacctcaaacagacaccgccaccatggactggacctggatc
ctgttcctggtggccgccgccacaagagtgcacagcgcacacttcccaggcttcgg
ccagagcctgctgttcggctaccccgtgtacgtgttcggcgactgcgtgcaaggcg
actggtgccccatcagcggcggcctgtgcagcgccagactgcacagacacgccctg
ctggccacatgccccgagcaccagatcacctgggaccccatcgacggcagagtgat
cggaagcgccctgcagttcctgatccccagactgcccagcttccccacacagagaa
ccagcaagaccctgaaggtgctgacccccccatcacacacaccacacccaacatc
cccccaagcttcctgcaggccatgcggaagtacagccccttcagaaacggctacat
ggaacccacactgggccagcacctgcccgccagcagcttccccgaccccggactga
ggccccagaacctgtacacactgtggggcggcagcgtcgtgtgcatgtacctgtac
cagctgagcccaccaatcacatggcccctgctgccccacgtgatcttctgccaccc
cggacagctgggcgccttcctgaccaacgtgccctacaagcggatcgaggaactgc
tgtacaagatcagcctgaccacaggcgccctgatcatcctgcccgaggactgcctg
cccaccacactgttccagcccgccagagcccccgtgacactgaccgcctggcagaa
cggcctgctgccattccacagcaccctgacaacacccggcctgatctggaccttca
ccgacggcacacccatgatcagcggcccctgccccaaggacggccagcccagcctg
gtgctgcagagcagcagcttcatcttccacaagttccagaccaaggcctaccaccc
cagcttcctgctgagccacggactgatccagtacagcagcttccacagcctgcacc
tgctgttcgaagagtacacaaacatccccatcagccggagcttcaacgagaaagag
gccgacgacaacgaccacgagccccagatcagccccggcggactggaacccccag
cgagaagcacttccgggaaaccgaagtgtgagctcgctttcttgctgtccaatttc
tattaaaggttcctttgttccctaagtccaactactaaactgggggatattatgaa
gggccttgagcatctggattctgcctaataaaaaacatttattttcattgcaaaaa
aaaaaaaaaaaaaaaaaaaaaaaaaaaaaaaaaaaaaaaaaaaaaaaaaaaaaaaa
aaaaaaaaaaaaaaaaaaaaaaaaaaaaaaaaaaaaaaaaagagcactagt
(SEQ ID NO: 9)**

**GCTAGC(NheI site): Nucleotide No. 1-6
T7 promoter sequence: Nucleotide No. 8-24
G: Transcription initiation point: Nucleotide No. 25
5'-UTR sequence(including transcription initiation point and KOZAK sequence of nucleotide Nos. 89-94): Nucleotide No. 25-94
Coding region of sec Tax mutant: Nucleotide No. 95-1207
3'-UTR sequence: Nucleotide No. 1208-1339
polyA: Nucleotide No. 1340-1441
ACTAGT(SpeI site): Nucleotide No. 1446-1451**

# Fig. 17

**Template plasmid DNA for sec Tax mutant in vitro transcription (IVT)**

taatacgactcactataaggagacccaagctacatttgcttctgacacaactgtgt
tcactagcaacctcaaacagacaccgccaccatggactggacctggatcctgttcc
tggtggccgccgccacaagagtgcacagcgcacacttcccaggcttcggccagagc
ctgctgttcggctaccccgtgtacgtgttcggcgactgcgtgcaaggcgactggtg
ccccatcagcggcggcctgtgcagcgccagactgcacagacacgccctgctggcca
catgccccgagcaccagatcacctgggaccccatcgacggcagagtgatcggaagc
gccctgcagttcctgatccccagactgcccagcttccccacacagagaaccagcaa
gaccctgaaggtgctgacccccccatcacacacaccacacccaacatcccccaa
gcttcctgcaggccatgcggaagtacagccccttcagaaacggctacatggaaccc
acactgggccagcacctgcccgccagcagcttccccgaccccggactgaggcccca
gaacctgtacacactgtggggcggcagcgtcgtgtgcatgtacctgtaccagctga
gcccaccaatcacatggcccctgctgccccacgtgatcttctgccaccccggacag
ctgggcgccttcctgaccaacgtgccctacaagcggatcgaggaactgctgtacaa
gatcagcctgaccacaggcgccctgatcatcctgcccgaggactgcctgcccacca
cactgttccagcccgccagagcccccgtgacactgaccgcctggcagaacggcctg
ctgccattccacagcaccctgacaacacccggcctgatctggaccttcaccgacgg
cacacccatgatcagcggcccctgccccaaggacggccagcccagcctggtgctgc
agagcagcagcttcatcttccacaagttccagaccaaggcctaccaccccagcttc
ctgctgagccacggactgatccagtacagcagcttccacagcctgcacctgctgtt
cgaagagtacacaaacatccccatcagccggagcttcaacgagaaagaggccgacg
acaacgaccacgagccccagatcagccccggcggactggaacccccagcgagaag
cacttccgggaaaccgaagtgtgagctcgctttcttgctgtccaatttctattaaa
ggttcctttgttccctaagtccaactactaaactggggatattatgaagggcctt
gagcatctggattctgcctaataaaaacatttattttcattgcaaaaaaaaaaaa
aaaaaaaaaaaaaaaaaaaaaaaaaaaaaaaaaaaaaaaaaaaaaaaaaaaa
aaaaaaaaaaaaaaaaaaaaaaaaaaaaaaaa
(SEQ ID NO: 10)

Fig. 18

**Tax wild-type amino acid sequence**

MAHFPGFGQSLLFGYPVYVFGDCVQGDWCPISGGLCSARLHRHALLATCPEHQITW
DPIDGRVIGSALQFLIPRLPSFPTQRTSKTLKVLTPPITHTTPNIPPSFLQAMRKY
SPFRNGYMEPTLGQHLPTLSFPDPGLRPQNLYTLWGGSVVCMYLYQLSPPITWPLL
PHVIFCHPGQLGAFLTNVPYKRIEELLYKISLTTGALIIILPEDCLPTTLFQPARAP
VTLTAWQNGLLPFHSTLTTPGLIWTFTDGTPMISGPCPKDGQPSLVLQSSSFIFHK
FQTKAYHPSFLLSHGLIQYSSFHSLHLLFEEYTNIPISLLFNEKEADDNDHEPQIS
PGGLEPPSEKHFRETEV
(SEQ ID NO: 11)

# Fig. 19

**Tax mutant (T130A/L131S/L319R/L320S) amino acid sequence**

**MAHFPGFGQSLLFGYPVYVFGDCVQGDWCPISGGLCSARLHRHALLATCPEHQITW
DPIDGRVIGSALQFLIPRLPSFPTQRTSKTLKVLTPPITHTTPNIPPSFLQAMRKY
SPFRNGYMEPTLGQHLPASSFPDPGLRPQNLYTLWGGSVVCMYLYQLSPPITWPLL
PHVIFCHPGQLGAFLTNVPYKRIEELLYKISLTTGALIIILPEDCLPTTLFQPARAP
VTLTAWQNGLLPFHSTLTTPGLIWTFTDGTPMISGPCPKDGQPSLVLQSSSFIFHK
FQTKAYHPSFLLSHGLIQYSSFHSLHLLFEEYTNIPISRSFNEKEADDNDHEPQIS
PGGLEPPSEKHFRETEV
(SEQ ID NO: 12)**

# Fig. 20

**Secretory Tax mutant (T130A/L131S/L319R/L320S) amino acid sequence**

**MDWTWILFLVAAATRVHSAHFPGFGQSLLFGYPVYVFGDCVQGDWCPISGGLCSAR
LHRHALLATCPEHQITWDPIDGRVIGSALQFLIPRLPSFPTQRTSKTLKVLTPPIT
HTTPNIPPSFLQAMRKYSPFRNGYMEPTLGQHLPASSFPDPGLRPQNLYTLWGGSV
VCMYLYQLSPPITWPLLPHVIFCHPGQLGAFLTNVPYKRIEELLYKISLTTGALII
LPEDCLPTTLFQPARAPVTLTAWQNGLLPFHSTLTTPGLIWTFTDGTPMISGPCPK
DGQPSLVLQSSSFIFHKFQTKAYHPSFLLSHGLIQYSSFHSLHLLFEEYTNIPISR
SFNEKEADDNDHEPQISPGGLEPPSEKHFRETEV
(SEQ ID NO: 13)**

**MDWTWILFLVAAATRVHS:Signal sequence**

Fig. 21

**gp46 amino acid sequence**

**MGKFLATLILFFQFCPLIFGDYSPSCCTLTIGVSSYHSKPCNPAQPVCSWTLDLLA
LSADQALQPPCPNLVSYSSYHATYSLYLFPHWTKKPNRNGGGYYSASYSDPCSLKC
PYLGCQSWTCPYTGAVSSPYWKFQHDVNFTQEVSRLNINLHFSKCGFPFSLLVDAP
GYDPIWFLNTEPSQLPPTAPPLLPHSNLDHILEPSIPWKSKLLTLVQLTLQSTNYT
CIVCIDRASLSTWHVLYSPNVSVPSSSSTPLLYPSLALPAPHLTLPFNWTHCFDPQ
IQAIVSSPCHNSLILPPFSLSPVPTLGSRSRR
(SEQ ID NO: 14)**

Fig. 22

**gp46-Fib amino acid sequence**

**MGKFLATLILFFQFCPLIFGDYSPSCCTLTIGVSSYHSKPCNPAQPVCSWTLDLLA
LSADQALQPPCPNLVSYSSYHATYSLYLFPHWTKKPNRNGGGYYSASYSDPCSLKC
PYLGCQSWTCPYTGAVSSPYWKFQHDVNFTQEVSRLNINLHFSKCGFPFSLLVDAP
GYDPIWFLNTEPSQLPPTAPPLLPHSNLDHILEPSIPWKSKLLTLVQLTLQSTNYT
CIVCIDRASLSTWHVLYSPNVSVPSSSSTPLLYPSLALPAPHLTLPFNWTHCFDPQ
IQAIVSSPCHNSLILPPFSLSPVPTLGSGSGSGYIPEAPRDGQAYVRKDGEWVLLS
TFL
(SEQ ID NO: 15)**

**GYIPEAPRDGQAYVRKDGEWVLLSTFL:Fibritin**

# Fig. 23

**dgp62 amino acid sequence**

**MGKFLATLILFFQFCPLIFGDYSPSCCTLTIGVSSYHSKPCNPAQPVCSWTLDLLA**
**LSADQALQPPCPNLVSYSSYHATYSLYLFPHWTKKPNRNGGGYYSASYSDPCSLKC**
**PYLGCQSWTCPYTGAVSSPYWKFQHDVNFTQEVSRLNINLHFSKCGFPFSLLVDAP**
**GYDPIWFLNTEPSQLPPTAPPLLPHSNLDHILEPSIPWKSKLLTLVQLTLQSTNYT**
**CIVCIDRASLSTWHVLYSPNVSVPSSSSTPLLYPSLALPAPHLTLPFNWTHCFDPQ**
**IQAIVSSPCHNSLILPPFSLSPVPTLGSGSGSAVPVAVWLVSALAMGAGVAGGITG**
**SMSLASGKSLLHEVDKDISQLTQAIVKNHKNLLKIAQYAAQNRRGLDLLFWEQGGL**
**CKALQEQCRFPNITNSHVPILQERPPLENRVLTGWGLNWDLGLSQWAREAGYIPEA**
**PRDGQAYVRKDGEWVLLSTFL**
**(SEQ ID NO: 16)**

**GYIPEAPRDGQAYVRKDGEWVLLSTFL:Fibritin**

# Fig. 24

**SU gp46 mRNA**

aggagacccaagcuacauuugcuucugacacaacuguguucacuagcaaccucaaa
cagacaccgccaccaugggcaaguuccuggccacacugauccuguucuuccaguuc
ugcccccugaucuucggcgacuacagccccagcugcugcacccugacaaucggcgu
gagcagcuaccacagcaagcccugcaaccccgcccagcccgugugcagcuggaccc
uggaccugcuggcccugagcgccgaccaggcccugcagcccccugccccaaccug
gugagcuacagcagcuaccacgccaccuacagccuguaccuguucccccacuggac
caagaagcccaacagaaacggcggaggcuacuacagcgccagcuacagcgaccccu
gcagccugaagugccccuaccugggcugccagagcuggacaugccccuacacaggc
gccgugagcagccccuacuggaaguuccagcacgacgugaacuucacccaagaggu
gagccggcugaacaucaaccugcacuucagcaagugcggcuucccauucagccugc
ugguggacgcccccggcuacgaccccaucugguuccugaacaccgagcccagccag
cugcccccaacagccccaccccugcugccccacagcaaccuggaccacauccugga
acccagcauccccuggaagagcaagcugcugacacuggugcagcugacccugcaga
gcaccaacuacaccugcaucgugugcaucgaccgggccagccugagcacauggcac
gugcuguacagccccaacgugagcgugcccagcagcagcagcacaccccugcugua
ccccagccuggcacugcccgcccccaccugacacugcccuucaacuggacccacu
gcuucgacccccagauccaggccaucgugagcagcccaugccacaacagccugauc
cugcccccauucagccugagccccgugcccacacugggcagcagaagcagaagaug
agcucgcuuucuugcuguccaauuucuauuaaagguuccuuuguucccuaagucca
acuacuaaacugggggauauuaugaagggccuugagcaucuggauucugccuaaua
aaaaacauuuauuuucauugcaaaaaaaaaaaaaaaaaaaaaaaaaaaaaaaaaaaaaaa
aaaaaaaaaaaaaaaaaaaaaaaaaaaaaaaaaaaaaaaaaaaaaaaaaaaaaaaaaaaa
aaaaaaaaa
(SEQ ID NO: 17)

# Fig. 25

**gp46-Fib mRNA**

**aggagacccaagcuacauuugcuucugacacaacuguguucacuagcaaccucaaa
cagacaccgccaccaugggcaaguuccuggccacacugauccuguucuuccaguuc
ugcccccugaucuucggcgacuacagccccagcugcugcacccugacaaucggcgu
gagcagcuaccacagcaagcccugcaaccccgcccagcccgugugcagcuggaccc
uggaccugcuggcccugagcgccgaccaggcccugcagcccccccugccccaaccug
gugagcuacagcagcuaccacgccaccuacagccuguaccuguucccccacuggac
caagaagcccaacagaaacggcggaggcuacuacagcgccagcuacagcgaccccu
gcagccugaagugccccuaccugggcugccagagcuggacaugccccuacacaggc
gccgugagcagccccuacuggaaguuccagcacgacgugaacuucacccaagaggu
gagccggcugaacaucaaccugcacuucagcaagugcggcuucccauucagccugc
ugguggacgcccccggcuacgaccccaucugguuccugaacaccgagcccagccag
cugcccccaacagccccaccccugcugccccacagcaaccuggaccacauccugga
acccagcauccccuggaagagcaagcugcugacacuggugcagcugacccugcaga
gcaccaacuacaccugcaucgugugcaucgaccgggccagccugagcacauggcac
gugcuguacagccccaacgugagcgugcccagcagcagcagcacaccccugcugua
ccccagccuggcacugcccgcccccaccugacacugcccuucaacuggacccacu
gcuucgacccccagauccaggccaucgugagcagcccaugccacaacagccugauc
cugcccccauucagccugagccccgugcccacacucggcagcggcagcggcagcgg
cuacauccccgaggcccccagagacggccaggccuacgucagaaaggacggcgaau
gggugcugcugagcaccuuccugugagcucgcuuucuugcuguccaauuucuauua
aagguuccuuuguucccuaaguccaacuacuaaacugggggauauuaugaagggcc
uugagcaucuggauucugccuaauaaaaaacauuuauuuucauugcaaaaaaaaaa
aaaaaaaaaaaaaaaaaaaaaaaaaaaaaaaaaaaaaaaaaaaaaaaaaaaaaaaa
aaaaaaaaaaaaaaaaaaaaaaaaaaaaaaaaaa
(SEQ ID NO: 18)**

# Fig. 26

**dgp62-Fib mRNA**

aggagacccaagcuacauuugcuucugacacaacuguguucacuagcaaccucaaa
cagacaccgccaccaugggcaaguuccuggccacacugauccuguucuuccaguuc
ugccccugaucuucggcgacuacagccccagcugcugcacccugacaaucggcgu
gagcagcuaccacagcaagcccugcaaccccgcccagcccgugugcagcuggaccc
uggaccugcuggcccugagcgccgaccaggcccugcagcccccugccccaaccug
gugagcuacagcagcuaccacgccaccuacagccuguaccuguuccccacuggac
caagaagcccaacagaaacggcggaggcuacuacagcgccagcuacagcgaccccu
gcagccugaagugccccuaccugggcugccagagcuggacaugccccuacacaggc
gccgugagcagccccuacuggaaguuccagcacgacgugaacuucacccaagaggu
gagccggcugaacaucaaccugcacuucagcaagugcggcuucccauucagccugc
uggcggacgcccccggcuacgaccccaucugguuccugaacaccgagcccagccag
cugcccccaacagccccaccccugcugccccacagcaaccuggaccacauccugga
acccagcauccccuggaagagcaagcugcugacacuggugcagcugacccugcaga
gcaccaacuacaccugcaucgugugcaucgaccgggccagccugagcacauggcac
gugcuguacagccccaacgugagcgugcccagcagcagcagcacaccccugcugua
ccccagccuggcacugcccgcccccaccugacacugcccuucaacuggacccacu
gcuucgacccccagauccaggccaucgugagcagcccaugccacaacagccugauc
cugcccccauucagccugagccccgugcccacacucggcagcggaagcggaagcgc
agugcccguggccgugguggcuggugagcgcccuggccaugggagccggcguggcag
gcggaaucacaggcagcaugagccuggccagcggcaagagccugcugcacgaggug
gacaaggacaucagccagcugacccaggccaucgugaagaaccacaagaaccugcu
gaagaucgcccaguacgccgcccagaacagaagaggacuggaccugcuguucuggg
agcaaggcggacugugcaaagcccuccaagagcagugcagauuccccaacaucacc
aacagccacgugcccauccugcaagagcggcccccacuggaaaacagagugcugac
aggauggggccugaacugggaccugggacugagccaguggccagagaggccggcu
acauccccgaggcccccagagacggacaggccuacgucagaaaggacggcgaaugg
gugcugcugagcaccuuccugugagcucgcuuucuugcuguccaauuucuauuaaa
gguuccuuuguucccuaaguccaacuacuaaacugggggauauuaugaagggccuu
gagcaucuggauucugccuaauaaaaaacauuuauuuucauugcaaaaaaaaaaaaaaaa
aaaaaaaaaaaaaaaaaaaaaaaaaaaaaaaaaaaaaaaaaaaaaaaaaaaaaaaaaaaa
aaaaaaaaaaaaaaaaaaaaaaaaaaaaaaaaaa
(SEQ ID NO: 19)

# Fig. 27

**sec Tax mutant mRNA**

aggagacccaagcuacauuugcuucugacacaacuguguucacuagcaaccucaaa
cagacaccgccaccauggacuggaccuggauccuguuccugguggccgccgccaca
agagugcacagcgcacacuucccaggcuucggccagagccugcuguucggcuaccc
cguguacguguucggcgacugcgugcaaggcgacuggugccccaucagcggcggcc
ugugcagcgccagacugcacagacacgcccugcuggccacaugccccgagcaccag
aucaccugggaccccaucgacggcagagugaucggaagcgcccugcaguuccugau
ccccagacugcccagcuuccccacacagagaaccagcaagacccugaaggugcuga
cccccccaucacacacaccacacccaacaucccccaagcuuccugcaggccaug
cggaaguacagccccuucagaaacggcuacauggaacccacacugggccagcaccu
gcccgccagcagcuuccccgaccccggacugaggccccagaaccuguacacacugu
ggggcggcagcgucgugugcauguaccuguaccagcugagcccaccaaucacaugg
ccccugcugccccacgugaucuucugccaccccggacagcugggcgccuuccugac
caacgugcccuacaagcggaucgaggaacugcuguacaagaucagccugaccacag
gcgcccugaucauccugcccgaggacugccugcccaccacacuguuccagcccgcc
agagcccccgugacacugaccgccuggcagaacggccugcugccauuccacagcac
ccugacaacacccggccugaucuggaccuucaccgacggcacacccaugaucagcg
gccccugccccaaggacggccagcccagccuggugcugcagagcagcagcuucauc
uuccacaaguuccagaccaaggccuaccaccccagcuuccugcugagccacggacu
gauccaguacagcagcuuccacagccugcaccugcuguucgaagaguacacaaaca
uccccaucagccggagcuucaacgagaaagaggccgacgacaacgaccacgagccc
cagaucagccccggcggacuggaacccccagcgagaagcacuuccgggaaaccga
agugugagcucgcuuucuugcuguccaauuucuauuaaagguuccuuuguucccua
aguccaacuacuaaacuggggggauauuaugaagggccuugagcaucuggauucugc
cuaauaaaaaacauuuauuuucauugcaaaaaaaaaaaaaaaaaaaaaaaaaaaaaaa
aaaaaaaaaaaaaaaaaaaaaaaaaaaaaaaaaaaaaaaaaaaaaaaaaaaaaaaaaaaa
aaaaaaaaaaaaaaaa
(SEQ ID NO: 20)

Fig. 28

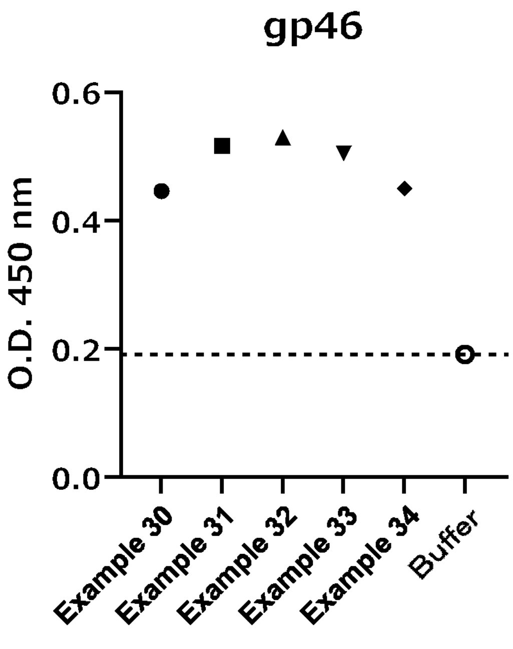
gp46
0.6
0.4
0.2
0.0
O.D. 450 nm
Example 30
Example 31
Example 32
Example 33
Example 34
Buffer

# Fig. 29

**Template DNA nucleotide sequence for gp46-Fib  polyA95**

gctagcgtaatacgactcactataaggagacccaagctacatttgcttctgacaca
actgtgttcactagcaacctcaaacagacaccgccaccatgggcaagttcctggcc
acactgatcctgttcttccagttctgccccctgatcttcggcgactacagccccag
ctgctgcaccctgacaatcggcgtgagcagctaccacagcaagccctgcaaccccg
cccagcccgtgtgcagctggaccctggacctgctggccctgagcgccgaccaggcc
ctgcagccccccctgccccaacctggtgagctacagcagctaccacgccacctacag
cctgtacctgttcccccactggaccaagaagcccaacagaaacggcggaggctact
acagcgccagctacagcgacccctgcagcctgaagtgcccctacctgggctgccag
agctggacatgcccctacacaggcgccgtgagcagcccctactggaagttccagca
cgacgtgaacttcacccaagaggtgagccggctgaacatcaacctgcacttcagca
agtgcggcttcccattcagcctgctggtggacgcccccggctacgaccccatctgg
ttcctgaacaccgagcccagccagctgcccccaacagccccacccctgctgcccca
cagcaacctggaccacatcctggaacccagcatcccctggaagagcaagctgctga
cactggtgcagctgaccctgcagagcaccaactacacctgcatcgtgtgcatcgac
cgggccagcctgagcacatggcacgtgctgtacagccccaacgtgagcgtgcccag
cagcagcagcacacccctgctgtaccccagcctggcactgcccgcccccacctga
cactgcccttcaactggacccactgcttcgacccccagatccaggccatcgtgagc
agcccatgccacaacagcctgatcctgcccccattcagcctgagccccgtgcccac
actcggcagcggcagcggcagcggctacatccccgaggcccccagagacggccagg
cctacgtcagaaaggacggcgaatgggtgctgctgagcaccttcctgtgagctcgc
tttcttgctgtccaatttctattaaaggttcctttgttccctaagtccaactacta
aactgggggatattatgaagggccttgagcatctggattctgcctaataaaaaca
tttattttcattgcaaaaaaaaaaaaaaaaaaaaaaaaaaaaaaaaaaaa
aaaaaaaaaaaaaaaaaaaaaaaaaaaaaaaaaaaaaaaaaaaaaaacga
agagc
(SEQ ID NO: 21)

gctagc(NheI site): Nucleotide No. 1-6
T7 promoter sequence: Nucleotide No. 8-24
a: Transcription initiation point: Nucleotide No. 25
5'-UTR sequence(including transcription initiation point and KOZAK sequence of nucleotide Nos. 89-94): Nucleotide No. 25-94
Coding region of gp46-Fib: Nucleotide No. 95-1114
3'-UTR sequence: Nucleotide No. 1115-1246
Poly A sequence(A95): Nucleotide No. 1247-1341

# Fig. 30

**Template DNA nucleotide sequence for gp46-Fib polyA80**

**gctagcgtaatacgactcactataaggagacccaagctacatttgcttctgacaca
actgtgttcactagcaacctcaaacagacaccgccaccatgggcaagttcctggcc
acactgatcctgttcttccagttctgcccctgatcttcggcgactacagccccag
ctgctgcaccctgacaatcggcgtgagcagctaccacagcaagccctgcaaccccg
cccagcccgtgtgcagctggaccctggacctgctggccctgagcgccgaccaggcc
ctgcagcccccctgccccaacctggtgagctacagcagctaccacgccacctacag
cctgtacctgttcccccactggaccaagaagcccaacagaaacggcggaggctact
acagcgccagctacagcgacccctgcagcctgaagtgcccctacctgggctgccag
agctggacatgcccctacacaggcgccgtgagcagcccctactggaagttccagca
cgacgtgaacttcacccaagaggtgagccggctgaacatcaacctgcacttcagca
agtgcggcttcccattcagcctgctggtggacgcccccggctacgaccccatctgg
ttcctgaacaccgagcccagccagctgcccccaacagccccacccctgctgcccca
cagcaacctggaccacatcctggaacccagcatcccctggaagagcaagctgctga
cactggtgcagctgaccctgcagagcaccaactacacctgcatcgtgtgcatcgac
cgggccagcctgagcacatggcacgtgctgtacagccccaacgtgagcgtgcccag
cagcagcagcacacccctgctgtaccccagcctggcactgcccgcccccacctga
cactgcccttcaactggacccactgcttcgacccccagatccaggccatcgtgagc
agcccatgccacaacagcctgatcctgcccccattcagcctgagccccgtgcccac
actcggcagcggcagcggcagcggctacatccccgaggcccccagagacggccagg
cctacgtcagaaaggacggcgaatgggtgctgctgagcaccttcctgtgagctcgc
tttcttgctgtccaatttctattaaaggttcctttgttccctaagtccaactacta
aactgggggatattatgaagggccttgagcatctggattctgcctaataaaaaaca
tttattttcattgcaaaaaaaaaaaaaaaaaaaaaaaaaaaaaaaaaaaaaaaaaa
aaaaaaaaaaaaaaaaaaaaaaaaaaaaaaaaaaaaaacgaagagc
(SEQ ID NO: 22)**

**gctagc(NheI site): Nucleotide No. 1-6
T7 promoter sequence: Nucleotide No. 8-24
a: Transcription initiation point: Nucleotide No. 25
5'-UTR sequence(including transcription initiation point and KOZAK sequence of nucleotide Nos. 89-94): Nucleotide No. 25-94
Coding region of gp46-Fib: Nucleotide No. 95-1114
3'-UTR sequence: Nucleotide No. 1115-1246
Poly A sequence(A80): Nucleotide No. 1247-1326**

# Fig. 31

**Template DNA nucleotide sequence for gp46-Fib polyA60**

**gctagcgtaatacgactcactataaggagacccaagctacatttgcttctgacaca
actgtgttcactagcaacctcaaacagacaccgccaccatgggcaagttcctggcc
acactgatcctgttcttccagttctgccccctgatcttcggcgactacagccccag
ctgctgcaccctgacaatcggcgtgagcagctaccacagcaagccctgcaaccccg
cccagcccgtgtgcagctggaccctggacctgctggccctgagcgccgaccaggcc
ctgcagccccccctgccccaacctggtgagctacagcagctaccacgccacctacag
cctgtacctgttcccccactggaccaagaagcccaacagaaacggcggaggctact
acagcgccagctacagcgacccctgcagcctgaagtgcccctacctgggctgccag
agctggacatgcccctacacaggcgccgtgagcagcccctactggaagttccagca
cgacgtgaacttcacccaagaggtgagccggctgaacatcaacctgcacttcagca
agtgcggcttcccattcagcctgctggtggacgcccccggctacgaccccatctgg
ttcctgaacaccgagcccagccagctgcccccaacagccccacccctgctgcccca
cagcaacctggaccacatcctggaacccagcatcccctggaagagcaagctgctga
cactggtgcagctgaccctgcagagcaccaactacacctgcatcgtgtgcatcgac
cgggccagcctgagcacatggcacgtgctgtacagccccaacgtgagcgtgcccag
cagcagcagcacacccctgctgtaccccagcctggcactgcccgcccccacctga
cactgcccttcaactggacccactgcttcgacccccagatccaggccatcgtgagc
agcccatgccacaacagcctgatcctgcccccattcagcctgagccccgtgcccac
actcggcagcggcagcggcagcggctacatccccgaggcccccagagacggccagg
cctacgtcagaaaggacggcgaatgggtgctgctgagcaccttcctgtgagctcgc
tttcttgctgtccaatttctattaaaggttcctttgttccctaagtccaactacta
aactgggggatattatgaagggccttgagcatctggattctgcctaataaaaaca
tttattttcattgcaaaaaaaaaaaaaaaaaaaaaaaaaaaaaaaaaaaaaaaaaa
aaaaaaaaaaaaaaaaaacgaagagc
(SEQ ID NO: 23)**

**gctagc(NheI site): Nucleotide No. 1-6
T7 promoter sequence: Nucleotide No. 8-24
a: Transcription initiation point: Nucleotide No. 25
5'-UTR sequence(including transcription initiation point and KOZAK sequence of nucleotide Nos. 89-94): Nucleotide No. 25-94
Coding region of gp46-Fib: Nucleotide No. 95-1114
3'-UTR sequence: Nucleotide No. 1115-1246
Poly A sequence(A60): Nucleotide No. 1247-1306**

# Fig. 32

**Template DNA nucleotide sequence for gp46-Fib polyA40**

**gctagcgtaatacgactcactataaggagacccaagctacatttgcttctgacaca
actgtgttcactagcaacctcaaacagacaccgccaccatgggcaagttcctggcc
acactgatcctgttcttccagttctgcccccctgatcttcggcgactacagccccag
ctgctgcaccctgacaatcggcgtgagcagctaccacagcaagccctgcaaccccg
cccagcccgtgtgcagctggaccctggacctgctggccctgagcgccgaccaggcc
ctgcagccccccctgccccaacctggtgagctacagcagctaccacgccacctacag
cctgtacctgttcccccactggaccaagaagcccaacagaaacggcggaggctact
acagcgccagctacagcgacccctgcagcctgaagtgcccctacctgggctgccag
agctggacatgcccctacacaggcgccgtgagcagcccctactggaagttccagca
cgacgtgaacttcacccaagaggtgagccggctgaacatcaacctgcacttcagca
agtgcggcttcccattcagcctgctggtggacgcccccggctacgaccccatctgg
ttcctgaacaccgagcccagccagctgcccccaacagccccacccctgctgcccca
cagcaacctggaccacatcctggaacccagcatcccctggaagagcaagctgctga
cactggtgcagctgaccctgcagagcaccaactacacctgcatcgtgtgcatcgac
cgggccagcctgagcacatggcacgtgctgtacagccccaacgtgagcgtgcccag
cagcagcagcacacccctgctgtaccccagcctggcactgcccgcccccacctga
cactgcccttcaactggacccactgcttcgacccccagatccaggccatcgtgagc
agcccatgccacaacagcctgatcctgcccccattcagcctgagccccgtgcccac
actcggcagcggcagcggcagcggctacatccccgaggcccccagagacggccagg
cctacgtcagaaaggacggcgaatgggtgctgctgagcaccttcctgtgagctcgc
tttcttgctgtccaatttctattaaaggttcctttgttccctaagtccaactacta
aactggggatattatgaagggccttgagcatctggattctgcctaataaaaaaca
tttattttcattgcaaaaaaaaaaaaaaaaaaaaaaaaaaaaaaaaaaaaaaaacg
aagagc
(SEQ ID NO: 24)**

**gctagc(NheI site): Nucleotide No. 1-6
T7 promoter sequence: Nucleotide No. 8-24
a: Transcription initiation point: Nucleotide No. 25
5'-UTR sequence(including transcription initiation point and KOZAK sequence of nucleotide Nos. 89-94): Nucleotide No. 25-94
Coding region of gp46-Fib: Nucleotide No. 95-1114
3'-UTR sequence: Nucleotide No. 1115-1246
Poly A sequence(A40): Nucleotide No. 1247-1286**

# Fig. 33

**Template DNA nucleotide sequence for gp46-Fib polyA20**

gctagcgtaatacgactcactataaggagacccaagctacatttgcttctgacaca
actgtgttcactagcaacctcaaacagacaccgccaccatgggcaagttcctggcc
acactgatcctgttcttccagttctgcccccctgatcttcggcgactacagccccag
ctgctgcaccctgacaatcggcgtgagcagctaccacagcaagccctgcaaccccg
cccagcccgtgtgcagctggaccctggacctgctggccctgagcgccgaccaggcc
ctgcagcccccctgccccaacctggtgagctacagcagctaccacgccacctacag
cctgtacctgttcccccactggaccaagaagcccaacagaaacggcggaggctact
acagcgccagctacagcgacccctgcagcctgaagtgcccctacctgggctgccag
agctggacatgcccctacacaggcgccgtgagcagcccctactggaagttccagca
cgacgtgaacttcacccaagaggtgagccggctgaacatcaacctgcacttcagca
agtgcggcttcccattcagcctgctggtggacgcccccggctacgaccccatctgg
ttcctgaacaccgagcccagccagctgcccccaacagccccacccctgctgcccca
cagcaacctggaccacatcctggaacccagcatcccctggaagagcaagctgctga
cactggtgcagctgaccctgcagagcaccaactacacctgcatcgtgtgcatcgac
cgggccagcctgagcacatggcacgtgctgtacagccccaacgtgagcgtgcccag
cagcagcagcacacccctgctgtaccccagcctggcactgcccgcccccacctga
cactgcccttcaactggacccactgcttcgacccccagatccaggccatcgtgagc
agcccatgccacaacagcctgatcctgcccccattcagcctgagccccgtgcccac
actcggcagcggcagcggcagcggctacatccccgaggcccccagagacggccagg
cctacgtcagaaaggacggcgaatgggtgctgctgagcaccttcctgtgagctcgc
tttcttgctgtccaatttctattaaaggttcctttgttccctaagtccaactacta
aactgggggatattatgaagggccttgagcatctggattctgcctaataaaaaaca
tttattttcattgcaaaaaaaaaaaaaaaaaaaacgaagagc
(SEQ ID NO: 25)

gctagc(NheI site): Nucleotide No. 1-6
T7 promoter sequence: Nucleotide No. 8-24
a: Transcription initiation point: Nucleotide No. 25
5'-UTR sequence(including transcription initiation point and KOZAK sequence of nucleotide Nos. 89-94): Nucleotide No. 25-94
Coding region of gp46-Fib: Nucleotide No. 95-1114
3'-UTR sequence: Nucleotide No. 1115-1246
Poly A sequence(A20): Nucleotide No. 1247-1266

# Fig. 34

**Template DNA nucleotide sequence for sec Tax mutant polyA95**

gctagcgtaatacgactcactataaggagacccaagctacatttgcttctgacaca
actgtgttcactagcaacctcaaacagacaccgccaccatggactggacctggatc
ctgttcctggtggccgccgccacaagagtgcacagcgcacacttcccaggcttcgg
ccagagcctgctgttcggctaccccgtgtacgtgttcggcgactgcgtgcaaggcg
actggtgccccatcagcggcggcctgtgcagcgccagactgcacagacacgccctg
ctggccacatgccccgagcaccagatcacctgggaccccatcgacggcagagtgat
cggaagcgccctgcagttcctgatccccagactgcccagcttccccacacagagaa
ccagcaagaccctgaaggtgctgacccccccatcacacacaccacacccaacatc
cccccaagcttcctgcaggccatgcggaagtacagccccttcagaaacggctacat
ggaacccacactgggccagcacctgcccgccagcagcttccccgaccccggactga
ggccccagaacctgtacacactgtggggcggcagcgtcgtgtgcatgtacctgtac
cagctgagcccaccaatcacatggcccctgctgccccacgtgatcttctgccaccc
cggacagctgggcgccttcctgaccaacgtgccctacaagcggatcgaggaactgc
tgtacaagatcagcctgaccacaggcgccctgatcatcctgcccgaggactgcctg
cccaccacactgttccagcccgccagagcccccgtgacactgaccgcctggcagaa
cggcctgctgccattccacagcaccctgacaacacccggcctgatctggaccttca
ccgacggcacacccatgatcagcggcccctgccccaaggacggccagcccagcctg
gtgctgcagagcagcagcttcatcttccacaagttccagaccaaggcctaccaccc
cagcttcctgctgagccacggactgatccagtacagcagcttccacagcctgcacc
tgctgttcgaagagtacacaaacatccccatcagccggagcttcaacgagaaagag
gccgacgacaacgaccacgagccccagatcagccccggcggactggaacccccag
cgagaagcacttccgggaaaccgaagtgtgagctcgctttcttgctgtccaatttc
tattaaaggttcctttgttccctaagtccaactactaaactgggggatattatgaa
gggccttgagcatctggattctgcctaataaaaaacatttattttcattgcaaaaa
aaaaaaaaaaaaaaaaaaaaaaaaaaaaaaaaaaaaaaaaaaaaaaaaaaaaaaaa
aaaaaaaaaaaaaaaaaaaaaaaaaaaaaaaaaacgaagagc
(SEQ ID NO: 26)

gctagc(NheI site): Nucleotide No. 1-6
T7 promoter sequence: Nucleotide No. 8-24
a: Transcription initiation point: Nucleotide No. 25
5'-UTR sequence(including transcription initiation point and KOZAK sequence of nucleotide Nos. 89-94): Nucleotide No. 25-94
Coding region of sec Tax mutant: Nucleotide No. 95-1207
3'-UTR sequence: Nucleotide No. 1208-1339
Poly A sequence(A95): Nucleotide No. 1340-1434

# Fig. 35

**Template DNA nucleotide sequence for sec Tax mutant polyA80**

**gctagcgtaatacgactcactataaggagacccaagctacatttgcttctgacaca**
**actgtgttcactagcaacctcaaacagacaccgccaccatggactggacctggatc**
**ctgttcctggtggccgccgccacaagagtgcacagcgcacacttcccaggcttcgg**
**ccagagcctgctgttcggctaccccgtgtacgtgttcggcgactgcgtgcaaggcg**
**actggtgccccatcagcggcggcctgtgcagcgccagactgcacagacacgccctg**
**ctggccacatgccccgagcaccagatcacctgggaccccatcgacggcagagtgat**
**cggaagcgccctgcagttcctgatccccagactgcccagcttccccacacagagaa**
**ccagcaagaccctgaaggtgctgacccccccatcacacacaccacacccaacatc**
**cccccaagcttcctgcaggccatgcggaagtacagccccttcagaaacggctacat**
**ggaacccacactgggccagcacctgcccgccagcagcttccccgaccccggactga**
**ggccccagaacctgtacacactgtggggcggcagcgtcgtgtgcatgtacctgtac**
**cagctgagcccaccaatcacatggcccctgctgccccacgtgatcttctgccaccc**
**cggacagctgggcgccttcctgaccaacgtgccctacaagcggatcgaggaactgc**
**tgtacaagatcagcctgaccacaggcgccctgatcatcctgcccgaggactgcctg**
**cccaccacactgttccagcccgccagagcccccgtgacactgaccgcctggcagaa**
**cggcctgctgccattccacagcaccctgacaacacccggcctgatctggaccttca**
**ccgacggcacacccatgatcagcggcccctgccccaaggacggccagcccagcctg**
**gtgctgcagagcagcagcttcatcttccacaagttccagaccaaggcctaccaccc**
**cagcttcctgctgagccacggactgatccagtacagcagcttccacagcctgcacc**
**tgctgttcgaagagtacacaaacatccccatcagccggagcttcaacgagaaagag**
**gccgacgacaacgaccacgagccccagatcagccccggcggactggaacccccag**
**cgagaagcacttccgggaaaccgaagtgtgagctcgctttcttgctgtccaatttc**
**tattaaaggttcctttgttccctaagtccaactactaaactgggggatattatgaa**
**gggccttgagcatctggattctgcctaataaaaacatttattttcattgcaaaaa**
**aaaaaaaaaaaaaaaaaaaaaaaaaaaaaaaaaaaaaaaaaaaaaaaaaaaaaaaa**
**aaaaaaaaaaaaaaaaaaacgaagagc**
**(SEQ ID NO: 27)**

**gctagc(NheI site): Nucleotide No. 1-6**
**T7 promoter sequence: Nucleotide No. 8-24**
**a: Transcription initiation point: Nucleotide No. 25**
**5'-UTR sequence(including transcription initiation point and KOZAK sequence of nucleotide Nos. 89-94): Nucleotide No. 25-94**
**Coding region of sec Tax mutant: Nucleotide No. 95-1207**
**3'-UTR sequence: Nucleotide No. 1208-1339**
**Poly A sequence(A80): Nucleotide No. 1340-1419**

# Fig. 36

**Template DNA nucleotide sequence for sec Tax mutant polyA60**

**gctagcgtaatacgactcactataaggagacccaagctacatttgcttctgacaca
actgtgttcactagcaacctcaaacagacaccgccaccatggactggacctggatc
ctgttcctggtggccgccgccacaagagtgcacagcgcacacttcccaggcttcgg
ccagagcctgctgttcggctaccccgtgtacgtgttcggcgactgcgtgcaaggcg
actggtgccccatcagcggcggcctgtgcagcgccagactgcacagacacgccctg
ctggccacatgccccgagcaccagatcacctgggaccccatcgacggcagagtgat
cggaagcgccctgcagttcctgatccccagactgcccagcttccccacacagagaa
ccagcaagaccctgaaggtgctgacccccccatcacacacaccacacccaacatc
cccccaagcttcctgcaggccatgcggaagtacagccccttcagaaacggctacat
ggaacccacactgggccagcacctgcccgccagcagcttccccgaccccggactga
ggccccagaacctgtacacactgtggggcggcagcgtcgtgtgcatgtacctgtac
cagctgagcccaccaatcacatggcccctgctgccccacgtgatcttctgccaccc
cggacagctgggcgccttcctgaccaacgtgccctacaagcggatcgaggaactgc
tgtacaagatcagcctgaccacaggcgccctgatcatcctgcccgaggactgcctg
cccaccacactgttccagcccgccagagcccccgtgacactgaccgcctggcagaa
cggcctgctgccattccacagcaccctgacaacacccggcctgatctggaccttca
ccgacggcacacccatgatcagcggcccctgccccaaggacggccagcccagcctg
gtgctgcagagcagcagcttcatcttccacaagttccagaccaaggcctaccaccc
cagcttcctgctgagccacggactgatccagtacagcagcttccacagcctgcacc
tgctgttcgaagagtacacaaacatccccatcagccggagcttcaacgagaaagag
gccgacgacaacgaccacgagccccagatcagccccggcggactggaacccccag
cgagaagcacttccgggaaaccgaagtgtgagctcgctttcttgctgtccaatttc
tattaaaggttcctttgttccctaagtccaactactaaactgggggatattatgaa
gggccttgagcatctggattctgcctaataaaaacatttattttcattgcaaaaa
aaaaaaaaaaaaaaaaaaaaaaaaaaaaaaaaaaaaaaaaaaaaaaaaaaaaaaac
gaagagc
(SEQ ID NO: 28)**

**gctagc(NheI site): Nucleotide No. 1-6
T7 promoter sequence: Nucleotide No. 8-24
a: Transcription initiation point: Nucleotide No. 25
5'-UTR sequence(including transcription initiation point and KOZAK sequence of nucleotide Nos. 89-94): Nucleotide No. 25-94
Coding region of sec Tax mutant: Nucleotide No. 95-1207
3'-UTR sequence: Nucleotide No. 1208-1339
Poly A sequence(A60): Nucleotide No. 1340-1399**

# Fig. 37

**Template DNA nucleotide sequence for sec Tax mutant polyA40**

**gctagcgtaatacgactcactataaggagacccaagctacatttgcttctgacaca
actgtgttcactagcaacctcaaacagacaccgccaccatggactggacctggatc
ctgttcctggtggccgccgccacaagagtgcacagcgcacacttcccaggcttcgg
ccagagcctgctgttcggctaccccgtgtacgtgttcggcgactgcgtgcaaggcg
actggtgccccatcagcggcggcctgtgcagcgccagactgcacagacacgccctg
ctggccacatgccccgagcaccagatcacctgggaccccatcgacggcagagtgat
cggaagcgccctgcagttcctgatccccagactgcccagcttccccacacagagaa
ccagcaagaccctgaaggtgctgacccccccccatcacacacaccacacccaacatc
cccccaagcttcctgcaggccatgcggaagtacagccccttcagaaacggctacat
ggaacccacactgggccagcacctgcccgccagcagcttccccgaccccggactga
ggccccagaacctgtacacactgtggggcggcagcgtcgtgtgcatgtacctgtac
cagctgagcccaccaatcacatggcccctgctgccccacgtgatcttctgccaccc
cggacagctgggcgccttcctgaccaacgtgccctacaagcggatcgaggaactgc
tgtacaagatcagcctgaccacaggcgccctgatcatcctgcccgaggactgcctg
cccaccacactgttccagcccgccagagcccccgtgacactgaccgcctggcagaa
cggcctgctgccattccacagcaccctgacaacacccggcctgatctggaccttca
ccgacggcacacccatgatcagcggcccctgccccaaggacggccagcccagcctg
gtgctgcagagcagcagcttcatcttccacaagttccagaccaaggcctaccaccc
cagcttcctgctgagccacggactgatccagtacagcagcttccacagcctgcacc
tgctgttcgaagagtacacaaacatccccatcagccggagcttcaacgagaaagag
gccgacgacaacgaccacgagccccagatcagccccggcggactggaaccccccag
cgagaagcacttccgggaaaccgaagtgtgagctcgctttcttgctgtccaatttc
tattaaaggttcctttgttccctaagtccaactactaaactgggggatattatgaa
gggccttgagcatctggattctgcctaataaaaaacatttattttcattgcaaaaa
aaaaaaaaaaaaaaaaaaaaaaaaaaaaaaaaaacgaagagc
(SEQ ID NO: 29)**

**gctagc(NheI site): Nucleotide No. 1-6
T7 promoter sequence: Nucleotide No. 8-24
a: Transcription initiation point: Nucleotide No. 25
5'-UTR sequence(including transcription initiation point and KOZAK sequence of nucleotide Nos. 89-94): Nucleotide No. 25-94
Coding region of sec Tax mutant: Nucleotide No. 95-1207
3'-UTR sequence: Nucleotide No. 1208-1339
Poly A sequence(A40): Nucleotide No. 1340-1379**

# Fig. 38

**Template DNA nucleotide sequence for sec Tax mutant polyA20**

**gctagcgtaatacgactcactataaggagacccaagctacatttgcttctgacaca
actgtgttcactagcaacctcaaacagacaccgccaccatggactggacctggatc
ctgttcctggtggccgccgccacaagagtgcacagcgcacacttcccaggcttcgg
ccagagcctgctgttcggctaccccgtgtacgtgttcggcgactgcgtgcaaggcg
actggtgccccatcagcggcggcctgtgcagcgccagactgcacagacacgccctg
ctggccacatgccccgagcaccagatcacctgggaccccatcgacggcagagtgat
cggaagcgccctgcagttcctgatccccagactgcccagcttccccacacagagaa
ccagcaagaccctgaaggtgctgacccccccatcacacacaccacacccaacatc
cccccaagcttcctgcaggccatgcggaagtacagccccttcagaaacggctacat
ggaacccacactgggccagcacctgcccgccagcagcttccccgaccccggactga
ggccccagaacctgtacacactgtggggcggcagcgtcgtgtgcatgtacctgtac
cagctgagcccaccaatcacatggcccctgctgccccacgtgatcttctgccaccc
cggacagctgggcgccttcctgaccaacgtgccctacaagcggatcgaggaactgc
tgtacaagatcagcctgaccacaggcgccctgatcatcctgcccgaggactgcctg
cccaccacactgttccagcccgccagagcccccgtgacactgaccgcctggcagaa
cggcctgctgccattccacagcaccctgacaacacccggcctgatctggaccttca
ccgacggcacacccatgatcagcggcccctgccccaaggacggccagcccagcctg
gtgctgcagagcagcagcttcatcttccacaagttccagaccaaggcctaccaccc
cagcttcctgctgagccacggactgatccagtacagcagcttccacagcctgcacc
tgctgttcgaagagtacacaaacatccccatcagccggagcttcaacgagaaagag
gccgacgacaacgaccacgagccccagatcagccccggcggactggaacccccag
cgagaagcacttccgggaaaccgaagtgtgagctcgctttcttgctgtccaatttc
tattaaaggttcctttgttccctaagtccaactactaaactgggggatattatgaa
gggccttgagcatctggattctgcctaataaaaaacatttattttcattgcaaaaa
aaaaaaaaaaaaaaacgaagagc
(SEQ ID NO: 30)**

**gctagc(NheI site): Nucleotide No. 1-6
T7 promoter sequence: Nucleotide No. 8-24
a: Transcription initiation point: Nucleotide No. 25
5'-UTR sequence(including transcription initiation point and KOZAK sequence of nucleotide Nos. 89-94): Nucleotide No. 25-94
Coding region of sec Tax mutant: Nucleotide No. 95-1207
3'-UTR sequence: Nucleotide No. 1208-1339
Poly A sequence(A20): Nucleotide No. 1340-1359**

# Fig. 39

**mRNA sequence for gp46-Fib polyA95**

aggagacccaagcuacauuugcuucugacacaacuguguucacuagcaaccucaaa
cagacaccgccaccaugggcaaguuccuggccacacugauccuguucuuccaguuc
ugccccugaucuucggcgacuacagccccagcugcugcacccugacaaucggcgu
gagcagcuaccacagcaagcccugcaaccccgcccagcccgugugcagcuggaccc
uggaccugcuggcccugagcgccgaccaggcccugcagcccccugccccaaccug
gugagcuacagcagcuaccacgccaccuacagccuguaccuguucccccacuggac
caagaagcccaacagaaacggcggaggcuacuacagcgccagcuacagcgaccccu
gcagccugaagugccccuaccugggcugccagagcuggacaugccccuacacaggc
gccgugagcagccccuacuggaaguuccagcacgacgugaacuucacccaagaggu
gagccggcugaacaucaaccugcacuucagcaagugcggcuucccauucagccugc
ugguggacgcccccggcuacgaccccaucugguuccugaacaccgagcccagccag
cugcccccaacagccccaccccugcugccccacagcaaccuggaccacauccugga
acccagcauccccuggaagagcaagcugcugacacuggugcagcugacccugcaga
gcaccaacuacaccugcaucgugugcaucgaccgggccagccugagcacauggcac
gugcuguacagccccaacgugagcgugcccagcagcagcagcacaccccugcugua
ccccagccuggcacugcccgcccccaccugacacugcccuucaacuggacccacu
gcuucgacccccagauccaggccaucgugagcagcccaugccacaacagccugauc
cugcccccauucagccugagccccgugcccacacucggcagcggcagcggcagcgg
cuacauccccgaggcccccagagacggccaggccuacgucagaaaggacggcgaau
gggugcugcugagcaccuuccugugagcucgcuuucuugcuguccaauuucuauua
aagguuccuuuguucccuaaguccaacuacuaaacugggggauauuaugaagggcc
uugagcaucuggauucugccuaauaaaaaacauuuauuuucauugcaaaaaaaaaaaaaa
aaaaaaaaaaaaaaaaaaaaaaaaaaaaaaaaaaaaaaaaaaaaaaaaaaaaaaaaaaaa
aaaaaaaaaaaaaaaaaaaaaaaaaaaaaaa
(SEQ ID NO: 31)

# Fig. 40

**mRNA sequence for gp46-Fib polyA80**

**aggagacccaagcuacauuugcuucugacacaacuguguucacuagcaaccucaaa
cagacaccgccaccaugggcaaguuccuggccacacugauccuguucuuccaguuc
ugccccugaucuucggcgacuacagccccagcugcugcacccugacaaucggcgu
gagcagcuaccacagcaagcccugcaaccccgcccagcccgugugcagcuggaccc
uggaccugcuggcccugagcgccgaccaggcccugcagcccccugccccaaccug
gugagcuacagcagcuaccacgccaccuacagccuguaccuguuccccacuggac
caagaagcccaacagaaacggcggaggcuacuacagcgccagcuacagcgaccccu
gcagccugaagugccccuaccugggcugccagagcuggacaugccccuacacaggc
gccgugagcagccccuacuggaaguuccagcacgacgugaacuucacccaagaggu
gagccggcugaacaucaaccugcacuucagcaagugcggcuucccauucagccugc
ugguggacgcccccggcuacgaccccaucugguuccugaacaccgagcccagccag
cugcccccaacagccccaccccugcugccccacagcaaccuggaccacauccugga
acccagcauccccuggaagagcaagcugcugacacuggugcagcugacccugcaga
gcaccaacuacaccugcaucgugugcaucgaccgggccagccugagcacauggcac
gugcuguacagccccaacgugagcgugcccagcagcagcagcacaccccugcugua
ccccagccuggcacugcccgcccccaccugacacugcccuucaacuggacccacu
gcuucgacccccagauccaggccaucgugagcagcccaugccacaacagccugauc
cugcccccauucagccugagccccgugcccacacucggcagcggcagcggcagcgg
cuacauccccgaggcccccagagacggccaggccuacgucagaaaggacggcgaau
gggugcugcugagcaccuuccugugagcucgcuuucuugcuguccaauuucuauua
aagguuccuuuguucccuaaguccaacuacuaaacugggggauauuaugaagggcc
uugagcaucuggauucugccuaauaaaaaacauuuauuuucauugcaaaaaaaaaa
aaaaaaaaaaaaaaaaaaaaaaaaaaaaaaaaaaaaaaaaaaaaaaaaaaaaaaaaaaaa
aaaaaaaaaaaaaaa
(SEQ ID NO: 32)**

# Fig. 41

**mRNA sequence for gp46-Fib polyA60**

aggagacccaagcuacauuugcuucugacacaacuguguucacuagcaaccucaaa
cagacaccgccaccaugggcaaguuccuggccacacugauccuguucuuccaguuc
ugccccugaucuucggcgacuacagccccagcugcugcacccugacaaucggcgu
gagcagcuaccacagcaagcccugcaaccccgcccagcccgugugcagcuggaccc
uggaccugcuggcccugagcgccgaccaggcccugcagccccccugccccaaccug
gugagcuacagcagcuaccacgccaccuacagccuguaccuguuccccacuggac
caagaagcccaacagaaacggcggaggcuacuacagcgccagcuacagcgaccccu
gcagccugaagugccccuaccugggcugccagagcuggacaugccccuacacaggc
gccgugagcagccccuacuggaaguuccagcacgacgugaacuucacccaagaggu
gagccggcugaacaucaaccugcacuucagcaagugcggcuucccauucagccugc
ugguggacgcccccggcuacgaccccaucugguuccugaacaccgagcccagccag
cugcccccaacagccccaccccugcugccccacagcaaccuggaccacauccugga
acccagcauccccuggaagagcaagcugcugacacuggugcagcugacccugcaga
gcaccaacuacaccugcaucgugugcaucgaccgggccagccugagcacauggcac
gugcuguacagccccaacgugagcgugcccagcagcagcagcacaccccugcugua
ccccagccuggcacugcccgccccccaccugacacugcccuucaacuggacccacu
gcuucgacccccagauccaggccaucgugagcagcccaugccacaacagccugauc
cugcccccauucagccugagccccgugcccacacucggcagcggcagcggcagcgg
cuacauccccgaggcccccagagacggccaggccuacgucagaaaggacggcgaau
gggugcugcugagcaccuuccugugagcucgcuuucuugcuguccaauuucuauua
aagguuccuuuguucccuaaguccaacuacuaaacugggggauauuaugaagggcc
uugagcaucuggauucugccuaauaaaaaacauuuauuuucauugcaaaaaaaaaa
aaaaaaaaaaaaaaaaaaaaaaaaaaaaaaaaaaaaaaaaaaaaaaaaaa
(SEQ ID NO: 33)

# Fig. 42

**mRNA sequence for gp46-Fib polyA40**

aggagacccaagcuacauuugcuucugacacaacuguguucacuagcaaccucaaa
cagacaccgccaccaugggcaaguuccuggccacacugauccuguucuuccaguuc
ugcccccugaucuucggcgacuacagccccagcugcugcacccugacaaucggcgu
gagcagcuaccacagcaagcccugcaaccccgcccagcccgugugcagcuggaccc
uggaccugcuggcccugagcgccgaccaggcccugcagcccccugccccaaccug
gugagcuacagcagcuaccacgccaccuacagccuguaccuguuccccacuggac
caagaagcccaacagaaacggcggaggcuacuacagcgccagcuacagcgaccccu
gcagccugaagugccccuaccugggcugccagagcuggacaugccccuacacaggc
gccgugagcagccccuacuggaaguuccagcacgacgugaacuucacccaagaggu
gagccggcugaacaucaaccugcacuucagcaagugcggcuucccauucagccugc
ugguggacgccccggcuacgaccccaucugguuccugaacaccgagcccagccag
cugcccccaacagccccaccccugcugccccacagcaaccuggaccacauccugga
acccagcauccccuggaagagcaagcugcugacacuggugcagcugacccugcaga
gcaccaacuacaccugcaucgugugcaucgaccgggccagccugagcacauggcac
gugcuguacagccccaacgugagcgugcccagcagcagcagcacaccccugcugua
ccccagccuggcacugcccgcccccaccugacacugcccuucaacuggacccacu
gcuucgacccccagauccaggccaucgugagcagcccaugccacaacagccugauc
cugcccccauucagccugagccccgugcccacacucggcagcggcagcggcagcgg
cuacauccccgaggcccccagagacggccaggccuacgucagaaaggacggcgaau
gggugcugcugagcaccuuccugugagcucgcuuucuugcuguccaauuucuauua
aagguuccuuuguucccuaaguccaacuacuaaacugggggauauuaugaagggcc
uugagcaucuggauucugccuaauaaaaaacauuuauuuucauugcaaaaaaaaaa
aaaaaaaaaaaaaaaaaaaaaaaaaaaaaa
(SEQ ID NO: 34)

# Fig. 43

**mRNA sequence for gp46-Fib  polyA20**

**aggagacccaagcuacauuugcuucugacacaacuguguucacuagcaaccucaaa
cagacaccgccaccaugggcaaguuccuggccacacugauccuguucuuccaguuc
ugcccccugaucuucggcgacuacagccccagcugcugcacccugacaaucggcgu
gagcagcuaccacagcaagcccugcaaccccgcccagcccgugugcagcuggaccc
uggaccugcuggcccugagcgccgaccaggcccugcagcccccugccccaaccug
gugagcuacagcagcuaccacgccaccuacagccuguaccuguucccccacuggac
caagaagcccaacagaaacggcggaggcuacuacagcgccagcuacagcgaccccu
gcagccugaagugccccuaccugggcugccagagcuggacaugccccuacacaggc
gccgugagcagccccuacuggaaguuccagcacgacgugaacuucacccaagaggu
gagccggcugaacaucaaccugcacuucagcaagugcggcuucccauucagccugc
ugguggacgcccccggcuacgaccccaucugguuccugaacaccgagcccagccag
cugcccccaacagccccaccccugcugccccacagcaaccuggaccacauccugga
acccagcauccccuggaagagcaagcugcugacacuggugcagcugacccugcaga
gcaccaacuacaccugcaucgugugcaucgaccgggccagccugagcacauggcac
gugcuguacagccccaacgugagcgugcccagcagcagcagcacaccccugcugua
ccccagccuggcacugcccgcccccaccugacacugcccuucaacuggacccacu
gcuucgacccccagauccaggccaucgugagcagcccaugccacaacagccugauc
cugcccccauucagccugagccccgugcccacacucggcagcggcagcggcagcgg
cuacauccccgaggcccccagagacggccaggccuacgucagaaaggacggcgaau
gggugcugcugagcaccuuccugugagcucgcuuucuugcuguccaauuucuauua
aagguuccuuuguucccuaaguccaacuacuaaacugggggauauuaugaagggcc
uugagcaucuggauucugccuaauaaaaaacauuuauuuucauugcaaaaaaaaaa
aaaaaaaaaa
(SEQ ID NO: 35)**

# Fig. 44

**mRNA sequence for sec Tax mutant polyA95**

aggagacccaagcuacauuugcuucugacacaacuguguucacuagcaaccucaaa
cagacaccgccaccauggacuggaccuggauccuguuccugguggccgccgccaca
agagugcacagcgcacacuucccaggcuucggccagagccugcuguucggcuaccc
cguguacguguucggcgacugcgugcaaggcgacuggugccccaucagcggcggcc
ugugcagcgccagacugcacagacacgcccugcuggccacaugccccgagcaccag
aucaccugggaccccaucgacggcagagugaucggaagcgcccugcaguuccugau
ccccagacugcccagcuuccccacacagagaaccagcaagacccugaaggugcuga
cccccccaucacacacaccacacccaacaucccccaagcuuccugcaggccaug
cggaaguacagccccuucagaaacggcuacauggaacccacacugggccagcaccu
gcccgccagcagcuuccccgaccccggacugaggccccagaaccuguacacacugu
ggggcggcagcgucgugugcauguaccuguaccagcugagcccaccaaucacaugg
ccccugcugccccacgugaucuucugccaccccggacagcugggcgccuuccugac
caacgugcccuacaagcggaucgaggaacugcuguacaagaucagccugaccacag
gcgcccugaucauccugcccgaggacugccugcccaccacacuguuccagcccgcc
agagcccccgugacacugaccgccuggcagaacggccugcugccauuccacagcac
ccugacaacacccggccugaucuggaccuucaccgacggcacacccaugaucagcg
gccccugccccaaggacggccagcccagccuggugcugcagagcagcagcuucauc
uuccacaaguuccagaccaaggccuaccaccccagcuuccugcugagccacggacu
gauccaguacagcagcuuccacagccugcaccugcuguucgaagaguacacaaaca
uccccaucagccggagcuucaacgagaaagaggccgacgacaacgaccacgagccc
cagaucagccccggcggacuggaacccccagcgagaagcacuuccgggaaaccga
agugugagcucgcuuucuugcuguccaauuucuauuaaagguuccuuuguucccua
aguccaacuacuaaacuggggauauuaugaagggccuugagcaucuggauucugc
cuaauaaaaacauuuauuuucauugcaaaaaaaaaaaaaaaaaaaaaaaaaaaaaa
aaaaaaaaaaaaaaaaaaaaaaaaaaaaaaaaaaaaaaaaaaaaaaaaaaaaaaaa
aaaaaaaaaa
(SEQ ID NO: 36)

# Fig. 45

**mRNA sequence for sec Tax mutant polyA80**

**aggagacccaagcuacauuugcuucugacacaacuguguucacuagcaaccucaaa
cagacaccgccaccauggacuggaccuggauccuguuccugguggccgccgccaca
agagugcacagcgcacacuucccaggcuucggccagagccugcuguucggcuaccc
cguguacguguucggcgacugcgugcaaggcgacuggugccccaucagcggcggcc
ugugcagcgccagacugcacagacacgcccugcuggccacaugccccgagcaccag
aucaccugggaccccaucgacggcagagugaucggaagcgcccugcaguuccugau
ccccagacugcccagcuuccccacacagagaaccagcaagacccugaaggugcuga
ccccccccaucacacacaccacacccaacaucccccccaagcuuccugcaggccaug
cggaaguacagccccuucagaaacggcuacauggaacccacacugggccagcaccu
gcccgccagcagcuuccccgaccccggacugaggccccagaaccuguacacacugu
ggggcggcagcgucgugugcauguaccuguaccagcugagcccaccaaucacaugg
ccccugcugccccacgugaucuucugccaccccggacagcugggcgccuuccugac
caacgugcccuacaagcggaucgaggaacugcuguacaagaucagccugaccacag
gcgcccugaucauccugcccgaggacugccugcccaccacacuguuccagcccgcc
agagcccccgugacacugaccgccuggcagaacggccugcugccauuccacagcac
ccugacaacacccggccugaucuggaccuucaccgacggcacacccaugaucagcg
gccccugccccaaggacggccagcccagccuggugcugcagagcagcagcuucauc
uuccacaaguuccagaccaaggccuaccaccccagcuuccugcugagccacggacu
gauccaguacagcagcuuccacagccugcaccugcuguucgaagaguacacaaaca
uccccaucagccggagcuucaacgagaaagaggccgacgacaacgaccacgagccc
cagaucagccccggcggacuggaacccccccagcgagaagcacuuccgggaaaccga
agugugagcucgcuuucuugcuguccaauuucuauuaaagguuccuuuguucccua
aguccaacuacuaaacugggggauauuaugaagggccuugagcaucuggauucugc
cuaauaaaaaacauuuauuuucauugcaaaaaaaaaaaaaaaaaaaaaaaaaaaaaaaaa
aaaaaaaaaaaaaaaaaaaaaaaaaaaaaaaaaaaaaaaaaaaaaaa
(SEQ ID NO: 37)**

# Fig. 46

**mRNA sequence for sec Tax mutant  polyA60**

**aggagacccaagcuacauuugcuucugacacaacuguguucacuagcaaccucaaa
cagacaccgccaccauggacuggaccuggauccuguuccugguggccgccgccaca
agagugcacagcgcacacuucccaggcuucggccagagccugcuguucggcuaccc
cguguacguguucggcgacugcgugcaaggcgacuggugccccaucagcggcggcc
ugugcagcgccagacugcacagacacgcccugcuggccacaugccccgagcaccag
aucaccugggaccccaucgacggcagagugaucggaagcgcccugcaguuccugau
ccccagacugcccagcuuccccacacagagaaccagcaagacccugaaggugcuga
ccccccccaucacacacaccacacccaacaucccccaagcuuccugcaggccaug
cggaaguacagccccuucagaaacggcuacauggaacccacacugggccagcaccu
gcccgccagcagcuuccccgaccccggacugaggccccagaaccuguacacacugu
ggggcggcagcgucgugugcauguaccuguaccagcugagcccaccaaucacaugg
ccccugcugccccacgugaucuucugccaccccggacagcugggcgccuuccugac
caacgugcccuacaagcggaucgaggaacugcuguacaagaucagccugaccacag
gcgcccugaucauccugcccgaggacugccugcccaccacacuguuccagcccgcc
agagcccccgugacacugaccgccuggcagaacggccugcugccauuccacagcac
ccugacaacacccggccugaucuggaccuucaccgacggcacacccaugaucagcg
gccccugccccaaggacggccagcccagccuggugcugcagagcagcagcuucauc
uuccacaaguuccagaccaaggccuaccaccccagcuuccugcugagccacggacu
gauccaguacagcagcuuccacagccugcaccugcuguucgaagaguacacaaaca
uccccaucagccggagcuucaacgagaaagaggccgacgacaacgaccacgagccc
cagaucagccccggcggacuggaacccccagcgagaagcacuuccgggaaaccga
agugugagcucgcuuucuugcuguccaauuucuauuaaagguuccuuuguucccua
aguccaacuacuaaacugggggauauuaugaagggccuugagcaucuggauucugc
cuaauaaaaaacauuuauuuucauugcaaaaaaaaaaaaaaaaaaaaaaaaaaaaaa
aaaaaaaaaaaaaaaaaaaaaaaaaaaaaa
(SEQ ID NO: 38)**

# Fig. 47

**mRNA sequence for sec Tax mutant  polyA40**

**aggagacccaagcuacauuugcuucugacacaacuguguucacuagcaaccucaaa
cagacaccgccaccauggacuggaccuggauccuguuccugguggccgccgccaca
agagugcacagcgcacacuucccaggcuucggccagagccugcuguucggcuaccc
cguguacguguucggcgacugcgugcaaggcgacuggugccccaucagcggcggcc
ugugcagcgccagacugcacagacacgcccugcuggccacaugccccgagcaccag
aucaccugggaccccaucgacggcagagugaucggaagcgcccugcaguuccugau
ccccagacugcccagcuuccccacacagagaaccagcaagacccugaaggugcuga
cccccccaucacacacaccacacccaacaucccccaagcuuccugcaggccaug
cggaaguacagccccuucagaaacggcuacauggaacccacacugggccagcaccu
gcccgccagcagcuuccccgaccccggacugaggccccagaaccuguacacacugu
ggggcggcagcgucgugugcauguaccuguaccagcugagcccaccaaucacaugg
ccccugcugccccacgugaucuucugccaccccggacagcugggcgccuuccugac
caacgugcccuacaagcggaucgaggaacugcuguacaagaucagccugaccacag
gcgcccugaucauccugcccgaggacugccugcccaccacacuguuccagcccgcc
agagcccccgugacacugaccgccuggcagaacggccugcugccauuccacagcac
ccugacaacacccggccugaucuggaccuucaccgacggcacacccaugaucagcg
gccccugccccaaggacggccagcccagccuggugcugcagagcagcagcuucauc
uuccacaaguuccagaccaaggccuaccaccccagcuuccugcugagccacggacu
gauccaguacagcagcuuccacagccugcaccugcuguucgaagaguacacaaaca
uccccaucagccggagcuucaacgagaaagaggccgacgacaacgaccacgagccc
cagaucagccccggcggacuggaacccccagcgagaagcacuuccgggaaaccga
agugugagcucgcuuucuugcuguccaauuucuauuaaagguuccuuuguucccua
aguccaacuacuaaacugggggauauuaugaagggccuugagcaucuggauucugc
cuaauaaaaaacauuuauuuucauugcaaaaaaaaaaaaaaaaaaaaaaaaaaaaa
aaaaaaaaaaa
(SEQ ID NO: 39)**

# Fig. 48

**mRNA sequence for sec Tax mutant  polyA20**

**aggagacccaagcuacauuugcuucugacacaacuguguucacuagcaaccucaaa**
**cagacaccgccaccauggacuggaccuggauccuguuccugguggccgccgccaca**
**agagugcacagcgcacacuucccaggcuucggccagagccugcuguucggcuaccc**
**cguguacguguucggcgacugcgugcaaggcgacuggugccccaucagcggcggcc**
**ugugcagcgccagacugcacagacacgcccugcuggccacaugccccgagcaccag**
**aucaccugggaccccaucgacggcagagugaucggaagcgcccugcaguuccugau**
**ccccagacugcccagcuuccccacacagagaaccagcaagacccugaaggugcuga**
**cccccccaucacacacaccacacccaacaucccccaagcuuccugcaggccaug**
**cggaaguacagccccuucagaaacggcuacauggaacccacacugggccagcaccu**
**gcccgccagcagcuuccccgaccccggacugaggccccagaaccuguacacacugu**
**ggggcggcagcgucgugugcauguaccuguaccagcugagcccaccaaucacaugg**
**ccccugcugccccacgugaucuucugccaccccggacagcugggcgccuuccugac**
**caacgugcccuacaagcggaucgaggaacugcuguacaagaucagccugaccacag**
**gcgcccugaucauccugcccgaggacugccugcccaccacacuguuccagcccgcc**
**agagcccccgugacacugaccgccuggcagaacggccugcugccauuccacagcac**
**ccugacaacacccggccugaucuggaccuucaccgacggcacacccaugaucagcg**
**gccccugccccaaggacggccagcccagccuggugcugcagagcagcagcuucauc**
**uuccacaaguuccagaccaaggccuaccaccccagcuuccugcugagccacggacu**
**gauccaguacagcagcuuccacagccugcaccugcuguucgaagaguacacaaaca**
**uccccaucagccggagcuucaacgagaaagaggccgacgacaacgaccacgagccc**
**cagaucagccccggcggacuggaacccccagcgagaagcacuuccgggaaaccga**
**agugugagcucgcuuucuugcuguccaauuucuauuaaagguuccuuuguucccua**
**aguccaacuacuaaacuggggggauauuaugaagggccuugagcaucuggauucugc**
**cuaauaaaaaacauuuauuuucauugcaaaaaaaaaaaaaaaaaaaa**
**(SEQ ID NO: 40)**

**INTERNATIONAL SEARCH REPORT**

International application No. **PCT/JP2022/020646**

**A. CLASSIFICATION OF SUBJECT MATTER**

***A61K 39/21***(2006.01)i; ***A61K 9/127***(2006.01)i; ***A61K 31/7115***(2006.01)i; ***A61K 38/16***(2006.01)i; ***A61K 47/10***(2006.01)i; ***A61K 47/18***(2006.01)i; ***A61K 47/24***(2006.01)i; ***A61K 47/28***(2006.01)i; ***A61P 31/14***(2006.01)i; ***C12N 15/88***(2006.01)i
FI: A61K39/21 ZNA; A61P31/14; A61K38/16; A61K31/7115; A61K9/127; A61K47/24; A61K47/28; A61K47/10; C12N15/88 Z; A61K47/18

According to International Patent Classification (IPC) or to both national classification and IPC

**B. FIELDS SEARCHED**

Minimum documentation searched (classification system followed by classification symbols)

A61K39/21; A61K9/127; A61K31/7115; A61K38/16; A61K47/10; A61K47/18; A61K47/24; A61K47/28; A61P31/14; C12N15/88

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2010-508014 A (CUREVAC GMBH) 18 March 2010 (2010-03-18)<br>claims, paragraphs [0019]-[0021], [0070], examples, etc. | 1-57 |
| Y | OHASHI, T. et al. Prevention of Adult T-Cell Leukemia-Like Lymphoproliferative Disease in Rats by Adoptively Transferred T Cells from a Donor Immunized with Human T-Cell Leukemia Virus Type 1 Tax-Coding DNA Vaccine. J Virol. 2000, vol. 74, no. 20, pp. 9610-9616, ISSN 0022-538X<br>abstract, etc. | 1-57 |
| Y | 大橋貴, 外2名, Tax発現DNAワクチン接種ラットより誘導したCTL細胞株のHTLV-I感染細胞に対する細胞傷害性の解析, 日本免疫学会総会・学術集会記録, vol. 30, 2000, p. 57<br>p. 57, column "1-C-131-P/O", (OHASHI, Takashi et al. Proceedings of the Japanese Society for Immunology.), non-official translation (Analysis of cytotoxicity for HTLV-I infected cells of CTL lines induced from rats vaccinated with Tax-expressing DNA) | 1-57 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 July 2022** | **09 August 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No. **PCT/JP2022/020646**

**C. DOCUMENTS CONSIDERED TO BE RELEVANT**

| Category* | Citation of document, with indication, where appropriate, of the relevant passages | Relevant to claim No. |
|---|---|---|
| Y | 大橋貴, ラットモデルを用いたヒト成人T細胞白血病に対する免疫療法の開発, 平成15年度がん研究に係わる特定領域（がん特定）研究報告集録, 2004, pp. 129-131<br>abstract, etc., (OHASHI, Taka. Development of immunotherapy for human adult T-cell leukemia using rat models.), non-official translation (2003 collection of research reports on a particular field (cancer-specific) related to cancer research) | 1-57 |
| Y | KABIRI, M. et al. The novel immunogenic chimeric peptide vaccine to elicit potent cellular and mucosal immune responses against HTLV-1. Int J Pharm. 2018, vol. 549, pp. 404-414, ISSN 0378-5173<br>abstract, introduction, etc. | 1-57 |
| Y | 長谷川秀樹, 外3名, ワクチン接種を行った母親マウスの母乳中における抗HTLV抗体応答の解析, 厚生労働省科学研究費補助金 新型インフルエンザ等新興・再興感染症研究事業 HTLV-1感染症予防ワクチンの開発に関する研究 平成24年度 総括・分担研究報告書, 2013, pp. 11-15<br>p. 11, research abstract, etc., (HASEGAWA, Hideki et al.), non-official translation (Analysis of anti-HTLV antibody response in breast milk of vaccinated mother mice. Scientific research grants of the Ministry of Health, Labor, and Welfare of Japan. Emerging and re-emerging infectious disease research project such as new strains of influenza. Research on the development of a prophylactic vaccine for HTLV-1 infectious disease. 2012 Research report on generalization and allotment.) | 1-57 |
| Y | WO 2015/005253 A1 (DAIICHI SANKYO COMPANY,LIMITED) 15 January 2015 (2015-01-15)<br>claims, examples, etc. | 1-57 |

Form PCT/ISA/210 (second sheet) (January 2015)

**INTERNATIONAL SEARCH REPORT**

International application No.
**PCT/JP2022/020646**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/020646**

| Patent document cited in search report | Publication date (day/month/year) | Patent family member(s) | Publication date (day/month/year) |
|---|---|---|---|
| JP 2010-508014 A | 18 March 2010 | US 2010/0047261 A1<br>claims, paragraphs [0019]-[0050], [0097], examples<br>WO 2008/052770 A2<br>EP 2083851 A2 | |
| WO 2015/005253 A1 | 15 January 2015 | US 2016/0257951 A1<br>claims, examples, etc.<br>EP 3020701 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015164674 A
- JP 2021084942 A
- WO 2015005253 A

### Non-patent literature cited in the description


- *Front Microbiol.,* 2012, vol. 3, 388
- *Blood,* 2012, vol. 119 (10), 2409-2416
- *Cancer Research,* 2010, vol. 70 (15), 6181-6192
- *Lancet Infect Dis,* 2007, vol. 7, 266-281
- *Front Microbiol.,* 2012, vol. 3, 406
- *Viruses,* 2011, vol. 3 (6), 714-749
- *Gene Dev,* 1990, vol. 4, 1875
- *J. Biochem.,* 2011, vol. 150 (6), 679-686
- *J. Biol Chem.,* 2006, vol. 281 (19), 13075-13082
- *Viruses,* 2016, vol. 8 (2), 41
- *Science,* 2021, vol. 371, 1152
- *J. Biol. Chem.,* 1992, vol. 267, 16396
- *J. Biol. Chem.,* 1991, vol. 266, 12127
- **SAMBROOK, J. et al.** Molecular Cloning a Laboratory Manual. 1989
- **RASHTCHIAN, A.** *Current Opinion in Biotechnology,* 1995, vol. 6 (1), 30-36
- **GIBSON D. G. et al.** *Science,* 2008, vol. 319 (5867), 1215-1220
- **KORMANN, M.** *Nature Biotechnology,* 2011, vol. 29, 154-157